# EUROPEAN PATENT APPLICATION

(11) **EP 2 354 247 A1**
(43) Date of publication of application: **10.08.2011**
(21) Application number: 10153379.2
(22) Date of filing: 11.02.2010
(51) Int. Cl.: C12Q 1/68

(54) **Means and methods for typing a sample for rheumatoid arthritis or spondyloarthritis**

(71) Applicant: Academisch Medisch Centrum bij de Universiteit van Amsterdam, 1105 AZ Amsterdam (NL); Arthrogen BV, 1105 BA Amsterdam (NL)
(72) Inventor: Baeten, Dominique Lievin Pierre, 3628 AJ Kockengen (NL); Tak, Paul Peter, 1241 AR Kortenhoef (NL); Yeremenko, Nataliya Gennadiyivna, 1096 HR Amsterdam (NL)
(74) Representative: Hatzmann, Martin

(57) **Abstract**

The invention provides a method for typing samples for rheumatoid arthritis and spondyloarthritis. Typing can be performed in order to discriminate between rheumatoid arthritis and spondyloarthritis in early disease. The invention further provides sets of genes to be used in a method of the invention and a nucleotide microarray and use thereof.

## Description

The invention relates to the fields of biology and medicine, more specifically the invention relates to arthritis.

Rheumatoid arthritis (RA) and spondyloarthritis (SpA) are the two most frequent forms of chronic arthritis in humans. RA has a prevalence of 1% and SpA of 0.5% in Europe and North America. Rheumatoid arthritis is a chronic inflammatory auto-immune disease. It can affect many tissues or organs, but mostly results in destruction of synovial joints. In rheumatoid arthritis immune complexes are formed in affected joints inducing inflammatory responses. In the serum of rheumatoid arthritis patients (auto)antibodies directed against Fc domains of antibodies called rheumatoid factor (RF) are found, however 40-50% of patients are seronegative for this factor. There are also a high number of false positive test results due to other disorders. Treatment of RA aims at relieving symptoms and preventing destruction of joints. Treatment regimes include disease modifying anti-rheumatic drugs (DMARDs), such as sulfasalazine and methotrexate, glucocorticoids and nonsteroidal anti-inflammatory drugs (NSAIDs) such as diclofenac and ibuprofen. In more recent years biologicals such as Tumor Necrosis Factor (TNF)α inhibitors or TNFα blocking antibodies, have become available for treatment of RA. Examples of these biologicals include etanercept (receptor fusion protein) and infliximab and adalimumab (monoclonal antibodies).

Spondyloarthritis is the name of a family of rheumatic inflammatory diseases that are characterized by arthritis of the spine but also peripheral joints. SpA comprises ankylosing spondylitis, psoriatic arthritis and psoriatic spondylitis, reactive arthritis and enteropathic arthritis. Patients suffering from ankylosing spondylitis usually experience pain and stiffness in the lower back, which becomes rigid and curved as the vertebrae fuse together. Approximately 40% of patients will also develop eye inflammation during the course of the disease. Reactive arthritis is redness and swelling of the large joints following a bacterial infection of the gastrointestinal tract, often caused by food poisoning, or genito-urinary tract, usually caused by micro-organisms responsible for chlamydia and gonorrhea. Psoriatic arthritis and psoriatic spondylitis occur in about 5% of people with psoriasis and often manifests itself with tendinitis and swelling of the fingers, and tends to develop 10 years after the onset of psoriasis. Enteropathic arthritis is associated with inflammatory bowel diseases, ulcerative colitis and Crohn's disease. Inflammation of the peripheral joints is accompanied by gastrointestinal symptoms such as abdominal pain and diarrhea. SpA is often referred to as seronegative to differentiate the diseases from RA. Symptoms often overlap between RA and SpA but SpA patients are negative for RF. SpA is often treated with NSAIDs or DMARDs. As with RA, anti-TNF treatment has been shown to be effective in treating spinal and peripheral joint symptoms of spondyloarthritis, psoriasis and intestinal inflammation.

The consequences of joint inflammation in RA and SpA are entirely different. Chronic inflammation in RA leads to cartilage and bone destruction almost without repair, whereas joint damage in SpA is dominated by excessive cartilage and bone formation leading to ankylosis. Moreover successful treatment of inflammation by TNF blockade inhibits cartilage and bone destruction in RA but does not affect structural remodeling and ankylosis in human SpA. Consequently, RA and SpA diseases require different treatments. Early diagnosis is crucial as very early diagnostic intervention is the best way to diminish or prevent long-term morbidity and irreversible structural damage and functional impairment of joint mobility. In full-blown disease, the two different disorders are relatively easy to recognize clinically. However, RA and SpA are often indistinguishable in the early phases of the disease. Therefore, a major challenge is to develop methods for early diagnosis of RA versus SpA.

It is an aim of the present invention to provide methods and means for typing a sample for discrimination between rheumatoid arthritis and spondyloarthritis.

The present invention provides the insight that levels of expression products of a here disclosed set of genes in individuals suffering from rheumatoid arthritis differ from expression product levels of said set of genes in individuals suffering from spondyloarthritis. Importantly, a difference in expression levels of said set of genes according to the invention is already measurable at an early stage of disease (less than six months of disease duration). Thus, by measuring expression levels of at least some genes of the herein provided set of genes, it has now become possible to distinguish between rheumatoid arthritis and spondyloarthritis.

Accordingly the invention provides a method for typing a sample of an individual suffering from or suspected of suffering from rheumatoid arthritis or spondyloarthritis, the method comprising determining levels of expression products of a set of genes, said expression products being obtained from, and/or present in, a sample of said individual, and typing said sample on the basis of the expression product levels determined for said set of genes, wherein said set of genes comprises at least 5 genes listed in table 3. Preferably, typing a sample comprises measuring the expression product levels of a set of genes according to the invention and comparing said expression levels with reference values. Said reference values are preferably representative of the expression product levels of the same genes in RA and/or SpA patients. Subsequently, said sample is typed as being indicative for RA or SpA depending on similarity with the RA or SpA reference values.

In principle, the more gene expression product levels according to the invention are determined, the more accurate the typing of a sample of an individual will be. Determining the levels of expression products of at least 10 genes according to the invention often results in a more accurate typing of a sample of an individual suspected of suffering from RA or SpA than determining the levels of expression products of 5 genes. Likewise, determining the levels of expression products of at least 20 genes according to the invention often results in a more accurate typing of a sample than determining the levels of expression products of 10 genes. Therefore, determining the levels of expression products of at least 10 genes according to the present invention is preferred over determining the levels of expression products of 5 genes. Determining the levels of expression products of at least 20 genes according to the invention is even more preferred. Determining the levels of expression products of at least 50 genes according to the invention is most preferred.

As used herein spondyloarthritis or SpA encompasses all subgroups of spondyloarthritis including psoriatic and nonpsoriatic spondyloarthritis. The original data shown in the Examples were obtained in exclusively non-psoriatic SpA but the subsequent confirmation study showed exactly the same findings in psoriatic SpA.

Table 3 shows the genes which in a synovial tissue sample of an individual suffering from spondyloarthritis have an at least 1.5 fold higher or lower expression level than in a synovial tissue sample of an individual suffering from rheumatoid arthritis. Table 4 shows the genes which in a synovial tissue sample of an individual suffering from spondyloarthritis have an at least 2 fold higher or lower expression level than in a synovial tissue sample of an individual suffering from rheumatoid arthritis. Thus, determining the levels of expression products of genes listed in table 4 is often preferred over determining the levels of expression products of genes listed in table 3, because the difference between said levels is larger. Hence, typing a sample with a method according to the invention is more sensitive when expression product levels of genes listed in table 4 are determined.

Further provided is therefore a method according to the invention wherein a set of genes according to the invention comprises at least 5 genes from table 4. In another preferred embodiment, a method according to the invention is provided wherein a set of genes according to the invention comprises at least 10 genes from table 3 or 4. More preferably a set of genes according to the invention comprises at least 10 genes from table 4. As explained above, in an even more preferred embodiment expression levels of at least 20 genes according to the invention are determined. Further provided is therefore a method according to the invention wherein a set of genes according to the invention comprises at least 20 genes from table 3 or 4. More preferably a set of genes according to the invention comprises at least 20 genes from table 4. Even more preferably a set of genes according to the invention comprises at least 50 genes from table 3 or 4, more preferably a set of genes according to the invention comprises at least 50 genes from table 4.

Hence, as defined herein, a set of genes according to the invention comprises at least 5 genes, more preferably at least 10 genes, more preferably at least 20 genes, even more preferably at least 50 genes listed in table 3 or 4. More preferably, a set of genes according to the invention comprises at least 5 genes, more preferably at least 10 genes, more preferably at least 20 genes, even more preferably at least 50 genes listed in table 4.

Every sample comprising expression products of genes is, in principle, suitable for a method according to the invention. Preferably, however, said sample comprises synovial tissue, blood or peripheral blood mononuclear cells (PBMC's).

Synovial tissue from an individual suffering from rheumatoid arthritis or spondyloarthritis is the primary target tissue of the disease. In view of this, the present inventors identified differentially expressed genes in the inflamed tissue, namely synovial tissue. For identifying differentially expressed genes in RA and SpA, a synovial tissue sample is highly preferred over samples normally used in prior art studies. These studies mostly use peripheral blood for the identification of differentially expressed genes in a wide variety of applications and may reveal some signatures of inflammation, but do not completely assess the complex interaction between inflammation and tissue reaction, such as destruction, remodeling and new tissue formation, which is the crucial distinction between rheumatoid arthritis and spondyloarthritis.

According to the invention, now that differentially expressed genes in early RA as compared to early SpA in synovial tissue samples of individuals suffering from RA or SpA have been identified, levels of expression product of the identified genes can also be measured in blood and PBMC's. It is generally not self-evident that genes that are differentially expressed in one kind of tissue, such as synovial tissue, are also differentially expressed in another type of sample, such as PBMC's. However, as is shown in the Examples, several genes identified as being differentially expressed in early RA as compared to early SpA in synovial tissue samples are surprisingly also differentially expressed in PBMC's of individuals suffering from RA or SpA.

As explained above, one of the advantages of a method according to the invention is that it is useful in early disease (less than six months disease duration) when the two disorders are indistinguishable clinically. As mentioned above, early diagnosis is crucial because the disorders require different treatment and very early intervention is the best way to diminish or prevent long-term morbidity, irreversible structural damage and functional impairment of joint mobility.

Therefore in a preferred embodiment the invention provides a method for typing a sample of an individual suffering from or suspected of suffering from rheumatoid arthritis or spondyloarthritis, the method comprising determining levels of expression products of a set of genes, said expression products being obtained from, and/or present in, a sample obtained or prepared from synovial tissue, blood or PBMC's of said individual, and typing said sample on the basis of the expression product levels determined for said set of genes, wherein said set of genes comprises at least 5 genes listed in table 3. As outlined above, said set of genes preferably comprises at least 5, more preferably at least 10, more preferably at least 20, most preferably at least 50 genes from table 3 or 4. More preferably said set of genes comprises at least 5, more preferably at least 10, more preferably at least 20, most preferably at least 50 genes from table 4.

Preferably a synovial tissue sample of an individual suffering from or suspected of suffering from rheumatoid arthritis or spondyloarthritis is obtained or prepared from a knee joint or an ankle-joint of said individual. Knee joints and ankle-joints are part of the group of large synovial joints in the body that are usually affected in rheumatoid arthritis and spondyloarthritis. Thus the invention provides a method for typing a sample of an individual suffering from or suspected of suffering from rheumatoid arthritis or spondyloarthritis, the method comprising determining levels of expression products of a set of genes, said expression products being obtained from, and/or present in, a sample of a knee joint or an ankle-joint of said individual, and typing said sample on the basis of the expression product levels determined for said set of genes, wherein said set of genes comprises at least 5 genes listed in table 3. As outlined above, said set of genes preferably comprises at least 5, more preferably at least 10, more preferably at least 20, most preferably at least 50 genes from table 3 or 4. More preferably said set of genes comprises at least 5, more preferably at least 10, more preferably at least 20, most preferably at least 50 genes from table 4.

A synovial tissue sample according to the invention is for instance obtained via a biopsy. Alternatively, a synovial sample can be obtained by blind needle biopsy or during surgery. Preferably, however, a blood sample or PBMC sample is used, because obtaining such sample is performed easily with little discomfort for the subject. As used herein a PBMC sample is defined as a sample comprising cells derived from the blood of a subject. Such a sample for instance includes lymphocyte, monocytes, macrophages and/or combinations thereof.

With the insight of the present invention, from each gene listed in tables 3 and 4 it has been determined whether said gene has a higher or lower expression level in synovial tissue in SpA and RA. When determining levels of expression products of a set of genes according to the invention using synovial tissue samples from individuals suffering from or suspected of suffering from SpA or RA, not all of said genes will always be regulated as indicated in tables 3 and 4. It is possible that certain individuals suffering from SpA or RA will have an expression level of one or several genes according to the invention that does not match with tables 3 and 4. However, the overall expression pattern in a synovial tissue sample of an individual suffering from RA will still resemble the RA expression pattern according to the invention more than the SpA expression pattern according to the invention, and vice versa. Typing of a synovial tissue sample from individuals suffering from or suspected of suffering from SpA or RA is therefore preferably based on the overall results of the expression levels of the genes determined. Thus in order to type a synovial tissue sample with a method according to the invention, it is preferably determined whether up- or downregulation of a set of genes better resembles either up- or downregulation of genes established in synovial tissue samples from individuals suffering from SpA or up- or downregulation of genes established in synovial tissue samples from individuals suffering from RA. If the expression levels of a set of genes according to the invention of a test sample more closely resemble the expression levels of said genes in RA individuals, the tested sample is typed to be an RA sample. If, on the other hand, the expression levels of said set of genes more closely resemble the expression levels of said genes in SpA individuals, the tested sample is established to be a SpA sample.

Likewise, in order to type a blood sample or a PBMC sample with a method according to the invention, it is preferably determined whether up- or downregulation of a set of genes according to the invention better resembles the expression levels of said set of genes in blood samples or PBMC samples from individuals suffering from SpA or the expression levels of said set of genes in blood samples or PBMC samples from individuals suffering from RA. If the expression levels of a set of genes according to the invention of a test sample more closely resemble the expression levels of said genes in RA individuals, the tested sample is typed to be an RA sample. If, on the other hand, the expression levels of said set of genes more closely resemble the expression levels of said genes in SpA individuals, the tested sample is established to be a SpA sample. It is possible that certain individuals suffering from SpA or RA will have an expression level of one or several genes according to the invention that does not match with the differential expression of these genes established in blood samples or PBMC samples from individuals suffering from SpA or RA. However, the overall expression pattern in a blood sample or a PBMC sample of an individual suffering from RA will still resemble the RA expression pattern according to the invention more than the SpA expression pattern according to the invention, and vice versa.

Whether the expression levels of a set of genes according to the invention of a test sample more closely resemble the expression levels of said genes in RA or SpA individuals is for instance determined by counting the number of genes from which the up- or downregulation corresponds with the up- or downregulation of said genes in RA or SpA. For example, when the differential regulation of more than 30 out of 50 genes according to the invention, more preferably 35 out of 50 genes according to the invention, even more preferably 40 out of 50 genes according to the invention corresponds to the differential regulation in RA, a test sample is typed as being indicative for RA. When the differential regulation of more than 30 out of 50 genes according to the invention, more preferably 35 out of 50 genes according to the invention, even more preferably 40 out of 50 genes according to the invention corresponds to the differential regulation in SpA, a test sample is typed as being indicative for SpA.

In one embodiment the invention provides a method wherein expression levels of at least one of 12 genes with an at least 3-fold positive regulation and/or at least one of 8 genes with an at least 3-fold negative regulation are determined. The 12 selected genes with with an at least 3-fold positive regulation in RA compared to SpA all function in the same pathophysiological mechanism, namely plasmablast and plasma cell differentiation, survival, and function. These genes are tumour necrosis factor receptor superfamily member 17 (TNFRSF17), , immunoglobulin J polypeptide, linker protein for immunoglobulin alpha and mu polypeptides (IGJ), POU class 2 associating factor 1 (POU2AF1), Der1-like domain family, member 3 (DERL3), Fc receptor-like 5 (FCRL5), plasma cell-induced ER protein 1 (PACAP), prepronociceptin (PNOC), secreted phosphoprotein 1 (SPP1), interferon regulatory factor 4 (IRF4), lymphocyte transmembrane adaptor 1 (LAX1), ELL associated factor 2 (EAF2), and pim-2 oncogene (PIM2). The 8 selected genes with an at least 3-fold regulation in RA compared to SpA all function in the same pathophysiological mechanism, namely myocyte or myofibroblast differentiation, survival, and function. These genes are actin, alpha 1, skeletal muscle (ACTA1), Myosin, heavy chain 3, skeletal muscle, embryonic (MYH2), myosin, heavy chain 1, skeletal muscle, adult (MYH1), cysteine and glycine-rich protein 3 (CSRP3), actinin, alpha 2 (ACTN2), troponin I type 2 (TNNI2), cytochrome P450, family 26, subfamily B, polypeptide 1 (CYP26B1), and titin-cap (TCAP). Determining the levels of expression products of these genes is preferred because typing a sample from an individual suspected of suffering from SpA or RA by determining said levels is particularly sensitive. Thus the invention provides a method for typing a sample of an individual suffering from or suspected of suffering from rheumatoid arthritis or spondyloarthritis, the method comprising determining levels of expression products of a set of genes, said expression products being obtained from, and/or present in, a sample of said individual and typing said sample on the basis of the expression product levels determined for said set of genes, wherein said set of genes comprises tumour necrosis factor receptor superfamily member 17 (TNFRSF17) and/or immunoglobulin J polypeptide, linker protein for immunoglobulin alpha and mu polypeptides (IGJ) and/or POU class 2 associating factor 1 (POU2AF1), and/or Der1-like domain family, member 3 (DERL3), and/or Fc receptor-like 5 (FCRL5), and/or plasma cell-induced ER protein 1 (PACAP), and/or prepronociceptin (PNOC), and/or secreted phosphoprotein 1 (SPP1), and/or interferon regulatory factor 4 (IRF4), and/or lymphocyte transmembrane adaptor 1 (LAX1), and/or ELL associated factor 2 (EAF2), and/or pim-2 oncogene (PIM2) and/or actin, alpha 1, skeletal muscle (ACTA1) and/or Myosin, heavy chain 3, skeletal muscle, embryonic (MYH2) and/or myosin, heavy chain 1, skeletal muscle, adult (MYH1), and/or cysteine and glycine-rich protein 3 (CSRP3), and/or actinin, alpha 2 (ACTN2), and/or troponin I type 2 (TNNI2), and/or cytochrome P450, family 26, subfamily B, polypeptide 1 (CYP26B1), and/or titin-cap (TCAP). In a preferred embodiment of the invention levels of expression products of at least 2, more preferably at least 3, more preferably at least 4, more preferably at least 5, more preferably at least 6, more preferably at least 7 genes selected from the group consisting of TNFRSF17 and IGJ and POU2AF1 and DERL3, and FCRL5, and PACAP, and PNOC, and SPP1, and IRF4, and LAX1, and EAF2, and PIM2 and ACTA1 and MYH2 and MYH1, and CSRP3, and ACTN2, and TNNI2, and CYP26B1, and TCAP are determined. In a most preferred embodiment, the invention provides a method for typing a sample of an individual suffering from or suspected of suffering from rheumatoid arthritis or spondyloarthritis, the method comprising determining levels of expression products of a set of genes, said expression products being obtained from, and/or present in, a sample of said individual, and typing said sample on the basis of the expression product levels determined for said set of genes, wherein said set of genes comprises TNFRSF17 and IGJ and POU2AF1 and DERL3, and FCRL5, and PACAP, and PNOC, and SPP1, and IRF4, and LAX1, and EAF2, and PIM2 and ACTA1 and MYH2 and MYH1, and CSRP3, and ACTN2, and TNNI2, and CYP26B1, and TCAP.

In another preferred embodiment, the invention provides a method for typing a sample of an individual suffering from or suspected of suffering from rheumatoid arthritis or spondyloarthritis, the method comprising determining levels of expression products of a set of genes, said expression products being obtained from, and/or present in, a sample of said individual, and typing said sample on the basis of the expression product levels determined for said set of genes, wherein said set of genes comprises TNFRSF17, and POU2AF1, and FCRL5, and PACAP, and PNOC, and SPP1, and IRF4, and EAF2, and PIM2, and ACTA1, and MYH2, and CSRP3, and CYP26B1, and TCAP.

In one embodiment the invention provides a method according to the invention wherein expression levels of key regulatory genes of significantly regulated molecular pathways are determined. Significantly regulated molecular pathways overrepresented in RA include cytotoxic T lymphocyte-mediated apoptosis, communication between innate and adaptive immune cells, hepatic fibrosis / hepatic stellate cell activation, death receptor signaling and type I diabetes mellitus signaling. Significantly regulated molecular pathways overrepresented in SpA include calcium signaling, actin cytoskeleton signaling, ILK signaling, cellular effects of sildenafil (Viagra) and hepatic fibrosis / hepatic stellate cell activation.

Expression products of a set of genes obtained from or present in a sample from an individual suffering from or suspected of suffering from rheumatoid arthritis or spondyloarthritis comprise for instance nucleic acid molecules and proteins. Analysis of expression products according to the invention can be performed with any method known in the art. Protein levels are for instance measured using antibody-based binding assays. Enzyme labeled, radioactively labeled or fluorescently labeled antibodies are for instance used for detection of protein. Assays that are for instance suitable include enzyme-linked immunosorbent assays (ELISA), radio-immuno assays (RIA), Western Blot assays and immunohistochemical staining assays. Alternatively, in order to determine the expression level of multiple proteins simultaneously protein arrays such as antibody-arrays are for instance used.

An expression product according to the invention preferably comprises RNA. The lifespan of RNA molecules is shorter than the lifespan of proteins. RNA levels are therefore more representative of the status of an individual at the time of sample preparation. Furthermore, determining RNA expression levels is less laborious than determining protein levels. For instance oligonucleotides arrays are used that are easier to develop and process than protein chips.

Therefore, in a preferred embodiment of the invention an expression product according to the invention is RNA. Accordingly, the invention provides a method for typing a sample of an individual suffering from or suspected of suffering from rheumatoid arthritis or spondyloarthritis, the method comprising determining levels of RNA of a set of genes, said RNA being obtained from, and/or present in, a sample of said individual and typing said sample on the basis of the RNA levels determined for said set of genes, wherein said set of genes comprises at least 5 genes listed in table 3. As explained above, said sample preferably comprises synovial tissue, more preferably said sample is obtained or prepared from a knee joint or an ankle-joint of said individual. Most preferably, however, a blood sample or a PBMC sample is used.

RNA samples can be processed in numerous ways, as is known to a skilled person. For example, they can be freshly prepared from cells or tissues at the moment of harvesting, or they can be prepared from samples that are stored at -70°C until processed for sample preparation. Tissues or cell samples can also be stored under alternative conditions that preserve the quality of the RNA. Examples of these preservative conditions are fixation using e.g. formaline, RNase inhibitors such as RNAsin (Pharmingen) or RNasecure (Ambion), aquous solutions such as RNAlater (Assuragen), Hepes-Glutamic acid buffer mediated Organic solvent Protection Effect (HOPE), and RCL2 (Alphelys), and non-aquous solutions such as Universal Molecular Fixative (Sakura Finetek USA Inc.). Usually, a chaotropic nucleic acid isolation lysis buffer (Boom method, Boom et al. J Clin Microbiol. 1990; 28:495-503) is used for RNA isolation.

Preferably RNA levels are quantified. The RNA level of a set of genes according to the invention is determined by any method known in the art. RNA levels can for instance be quantified in relation to a household gene and/or can be determined relatively in relation with RNA levels of other genes measured at the same time. Methods to determine RNA levels of genes are known to a skilled person and include, but are not limited to, Northern blotting, (quantitative) PCR, and microarray analysis.

Northern blotting comprises the quantification of RNA of a specific gene by hybridizing a labeled probe that specifically interacts with said RNA, after separation of RNA by gel electrophoresis. Probes are for instance labeled with radioactive isotopes or chemiluminescent substrates. Quantification of the labeled probe that has interacted with said nucleic acid expression product serves as a measure for determining the level of expression. The determined level of expression can be normalized for differences in the total amounts of nucleic acid expression products between two separate samples with for instance an internal or external calibrator by comparing the level of expression of a gene that is known not to differ in expression level between samples or by adding a known quantity of RNA before determining the expression levels.

Additionally or alternatively, RNA is reverse transcribed into cDNA. Reverse transcriptase polymerase chain reaction (RT-PCR) is for instance performed using specific primers that hybridize to an RNA sequence of interest and a reverse transcriptase enzyme. Furthermore, RT-PCR can be performed with random primers, such as for instance random hexamers or decamers which hybridize randomly along the RNA, or oligo d(T) which hybridizes to the poly(A) tail of mRNA, and reverse transcriptase enzyme.

Quantitative Polymerase Chain Reaction (qPCR) provides an alternative method to quantify the level of expression of nucleic acids. qPCR can be performed by real-time PCR (rtPCR), in which the amount of product is monitored during the amplification reaction, or by end-point measurements, in which the amount of a final product is determined. As is known to a skilled person, rtPCR is for instance performed by the use of a nucleic acid intercalator, such as for example ethidium bromide or SYBR® Green I dye, which interacts which all generated double stranded products resulting in an increase in fluorescence during amplification, or for instance by the use of labeled probes that react specifically with the generated double stranded product of the gene of interest. Alternative detection methods that can be used are provided by amongst other things dendrimer signal amplification, hybridization signal amplification, and molecular beacons.

Microarray analysis involves the use of selected nucleic acid molecules that are immobilized on a surface. These nucleic acid molecules, termed probes, are able to hybridize to nucleic acid expression products. In a preferred embodiment the probes are exposed to labeled sample nucleic acid, hybridized, washed and the (relative) amount of nucleic acid expression products in the sample that are complementary to a probe is determined. Microarray analysis allows simultaneous determination of nucleic acid expression levels of a large number of genes. In a method according to the invention it is preferred that at least 5 genes according to the invention are measured simultaneously.

Therefore, in a preferred embodiment of the invention RNA levels obtained from, and/or present in, a sample of an individual suffering from or suspected of suffering from rheumatoid arthritis or spondyloarthritis are determined by microarray analysis. Thus the invention provides a method for typing a sample of an individual suffering from or suspected of suffering from rheumatoid arthritis or spondyloarthritis, the method comprising determining RNA levels of a set of genes, said RNA being obtained from, and/or present in, a sample of said individual, and typing said sample on the basis of the RNA levels determined for said set of genes, wherein said set of genes comprises at least 5 genes listed in table 3 and wherein RNA levels are determined by microarray analysis. Background correction can be performed for instance according to the "offset" method that avoids negative intensity values after background subtraction. Furthermore, normalization can be performed in order to make the two channels on each single array comparable for instance using global loess normalization, and scale normalization which ensures that the log-ratios are scaled to have the same median-absolute-deviation (MAD) across arrays.

In a preferred embodiment a method according to the invention is provided wherein RNA levels of at least 10, more preferably at least 20, most preferably at least 50 genes from table 3 or 4 are determined. More preferably RNA levels of at least 5, more preferably at least 10, more preferably at least 20, most preferably at least 50 genes from table 4 are determined in order to enhance specificity.

Now that the invention has provided the insight that the genes listed in tables 3 and 4 are differentially expressed in SpA patients compared to RA patients, a nucleotide microarray is provided in order to measure these changes in expression. Also provided is therefore a nucleotide microarray comprising nucleic acid molecules being able to hybridize to at least 5 genes listed in table 3 or 4, and wherein at least 60%, preferably at least 70%, more preferably at least 80% of the nucleic acid molecules of said microarray are able to hybridize to genes listed in table 3 or 4. Probes immobilized on said nucleotide microarray comprise at least 5 nucleic acid molecules able to hybridize to at least 5 genes listed in table 3 or 4. Each of said at least 5 nucleic acid molecules is capable of hybridizing to a different gene of table 3 or 4, so that, overall, at least 5 different genes listed in table 3 or 4 can be bound.

More preferably a nucleotide microarray according to the invention comprises nucleic acid molecules being able to hybridize to at least 10 genes listed in table 3 or 4, more preferably a nucleotide microarray according to the invention comprises nucleic acid molecules being able to hybridize to at least 20 genes listed in table 3 or 4, more preferably a nucleotide microarray according to the invention comprises nucleic acid molecules being able to hybridize to at least 50 genes listed in table 3 or 4. Hybridization is known in the art and refers to the combining of complementary, single-stranded nucleic acids, preferably under stringent conditions. Complementary is also known in the art and refers to two nucleic acid strands from which the base pairs can be non-covalently connected. A nucleotide microarray according to the invention comprises nucleic acid molecules, preferably DNA oligonucleotides. The length of said DNA oligonucleotides can vary between 15 bases and several kilo bases, and is preferably between 20 bases and 1 kilobase, more preferably between 40 and 100 bases, and most preferably between 50 and 80 nucleotides. Most (i.e. at least 60% of) nucleic acid molecules of a nucleotide microarray according to the invention are able to hybridize to genes listed in table 3 or 4. Of note, tables 3 and 4 contain non-limiting examples of suitable nucleic acid probes which are used in the Examples. Other nucleic acid probes which are able to hybridize to a different region of the in table 3 or 4 listed genes are of course also suitable for use in a microarray or method according to the invention. Using a nucleotide microarray according to the invention an accurate gene expression profile is obtained in order to type a sample from an individual suspected of suffering from SpA or RA by determining whether the profile of a sample resembles a SpA expression profile or an RA expression profile according to the invention.

The remaining at most 40% of the nucleic acid molecules of a nucleotide microarray are for instance able to hybridize to a set of reference genes or a set of normalization genes. Normalization is preferably performed in order to reduce systemic bias. Systemic bias results in variation by inter-array differences in overall performance, which can be due to for example inconsistencies in array fabrication, staining and scanning, and variation between labeled RNA samples, which can be due for example to variations in purity. Systemic bias can be introduced during the handling of the sample in a microarray experiment. To reduce systemic bias, the determined RNA levels are preferably corrected for background non-specific hybridization and normalized. Said normalization genes detect for instance RNA expression levels of housekeeping genes such as glyceraldehyde-3-phosphate dehydrogenase (GAPDH), hypoxanthine phosphoribosyltransferase (HPRT) and 18S RNA levels, of which the RNA level is thought to be constant in a given cell or tissue and independent from the disease affecting said cell or tissue or inflammatory status of said cell or tissue.

The use of such reference probes is advantageous but not mandatory. In one embodiment a microarray according to the invention is provided wherein at least 90% of the nucleic acid sequences are able to hybridize to genes listed in table 3 or 4. In a further embodiment at least 95% or even 100% of the nucleic acid sequences are able to hybridize to genes listed in table 3 or 4.

As used herein, a nucleic acid molecule or nucleic acid sequence of the invention that serves as a probe in a microarray analysis preferably comprises a chain of nucleotides, more preferably DNA and/or RNA. In other embodiments a nucleic acid molecule or nucleic acid sequence of the invention comprises other kinds of nucleic acid structures such as for instance a DNA/RNA helix, peptide nucleic acid (PNA), locked nucleic acid (LNA) and/or a ribozyme. Hence, as used herein the term "nucleic acid molecule" also encompasses a chain comprising non-natural nucleotides, modified nucleotides and/or non-nucleotide building blocks which exhibit the same function as natural nucleotides.

Also provided is a use of a nucleotide microarray according to the invention for typing a sample of an individual suffering from or suspected of suffering from rheumatoid arthritis or spondyloarthritis. As said before, said microarray preferably comprises nucleic acid molecules being able to hybridize to at least 10 genes, preferably at least 20 genes, most preferably at least 50 genes listed in table 3 or 4 in order to improve specificity and/or sensitivity.

The invention is further explained in the following examples. These examples do not limit the scope of the invention, but merely serve to clarify the invention.

### Figure legends

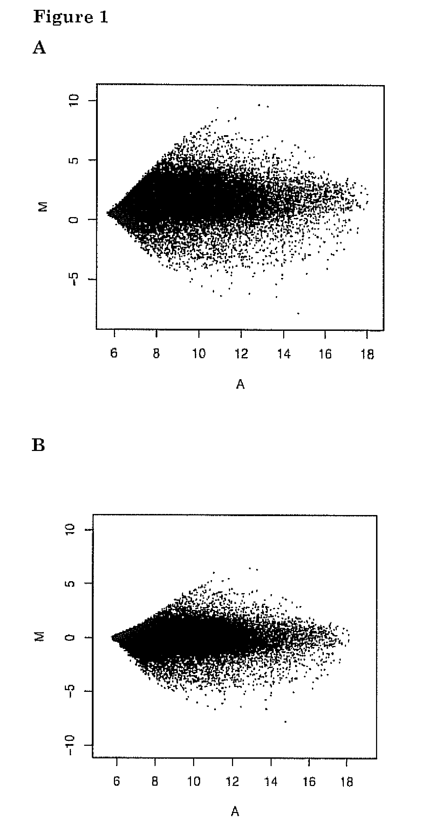

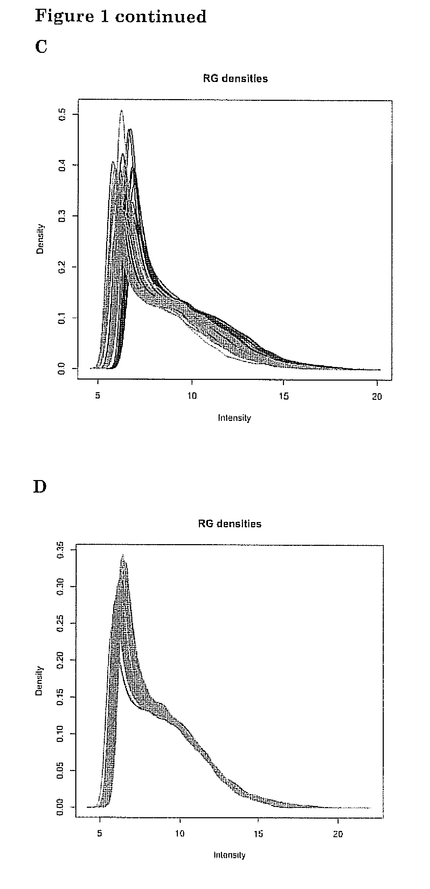

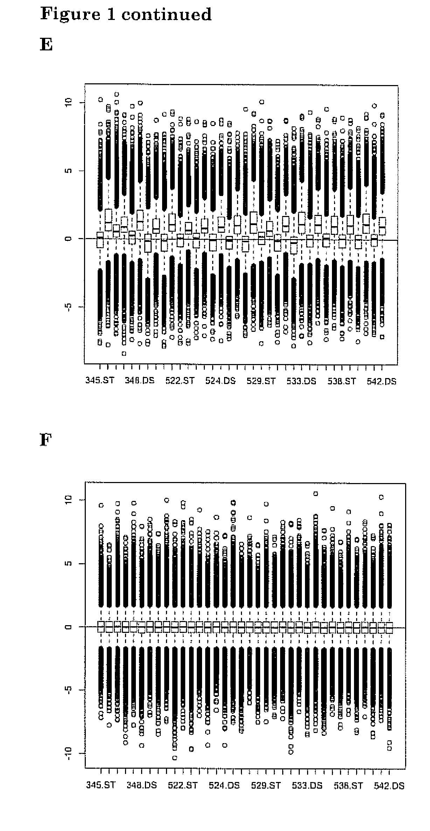

**Figure 1****. Normalization of the microarray data.** Raw data from one of the microarrays plotted in MA-plot, A is mean log intensity, M is log ratio. (A). Normalized data from the same microarray plotted in MA-plot (B). Density distribution of the intensities across the red and green channels for the raw data (C) and normalized data (D) of all microarrays in the experiment. Box-plots of the log ratios observed in the microarrays, using the raw data (E) and normalized data (F). The boxes span the range of log ratios that fall in the middle 50% of the distribution. The whiskers show extreme upper and lower observations on each microarray.

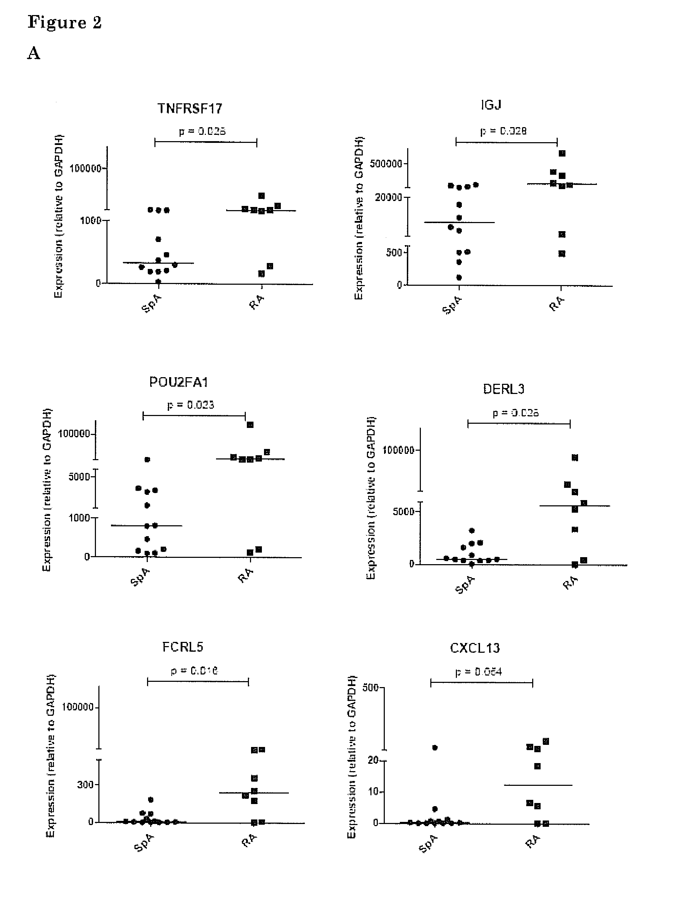

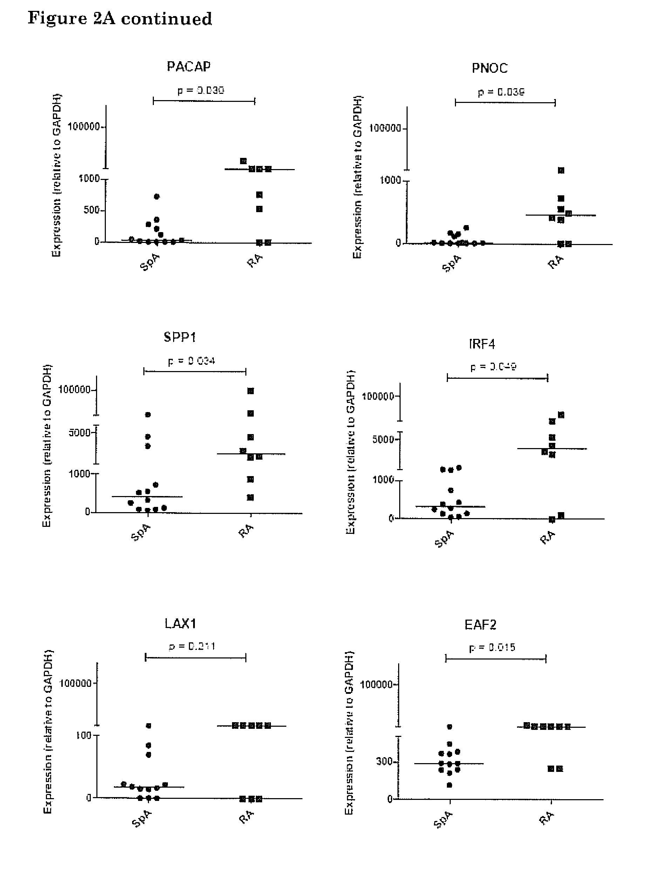

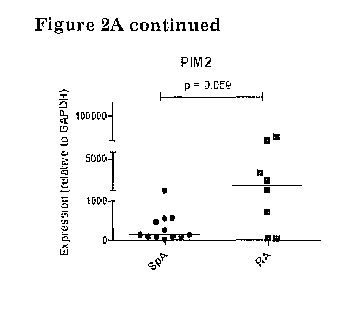

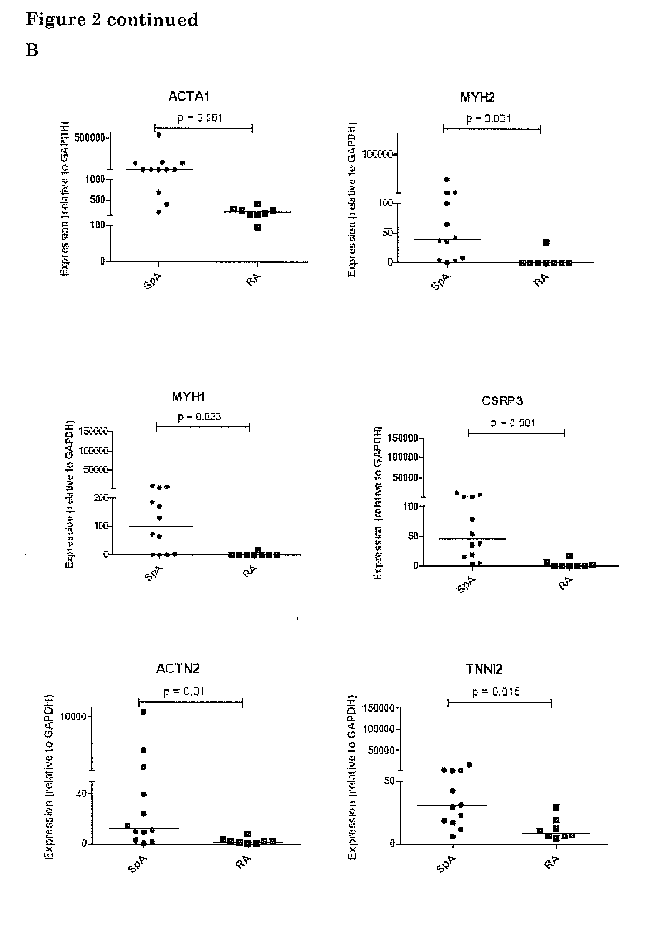

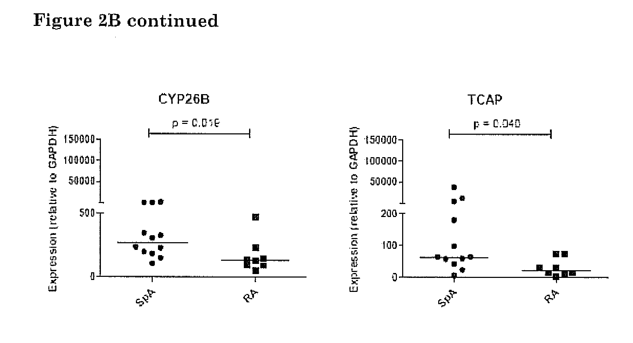

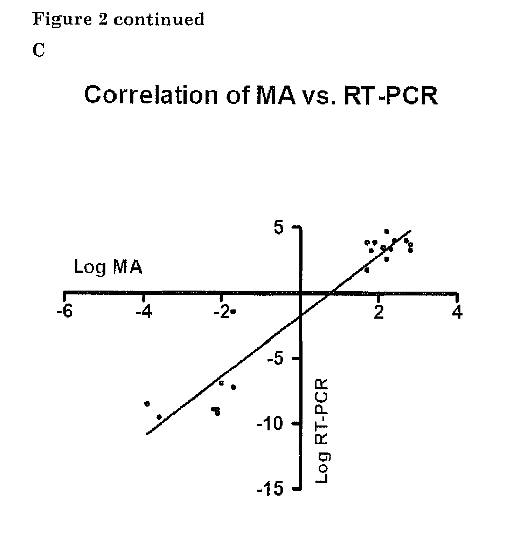

**Figure 2****. Quantitative RT-PCR analysis for technical validation of microarray data on the same cohort.** (A). RT-PCR of TNFRSF17, IGJ, POU2AF1, DERL3, FCRL5, CXCL13, PACAP, PNOC, SPP1, IRF4, LAX1, EAF2, PIM2 transcripts which have been determined by microarray analysis as upregulated in synovial tissue samples of RA patients. (B). RT-PCR of ACTA1, MYH2, MYH1, CSRP3, ACTN2, TNNI2, CYP26B and TCAP transcripts which have been determined by microarray analysis as upregulated in synovial tissue samples of SpA patients. Data is represented as an individual expression corrected for expression of the control gene (GAPDH) and bars show the median. Relative expression level was considered significant if p<0.05 (Mann-Whitney). (C). Correlation of microarray data and real time PCR data

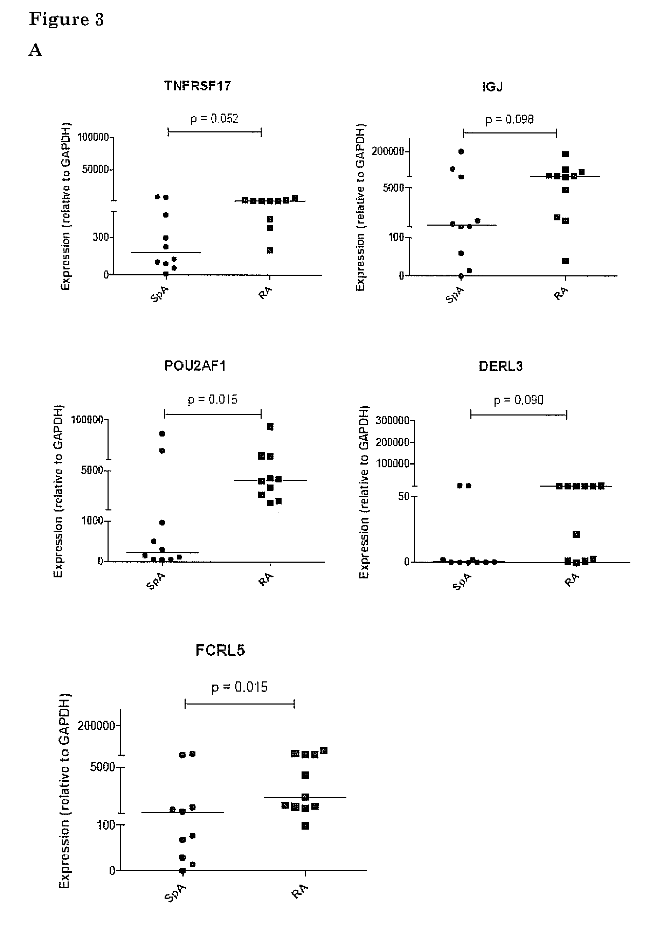

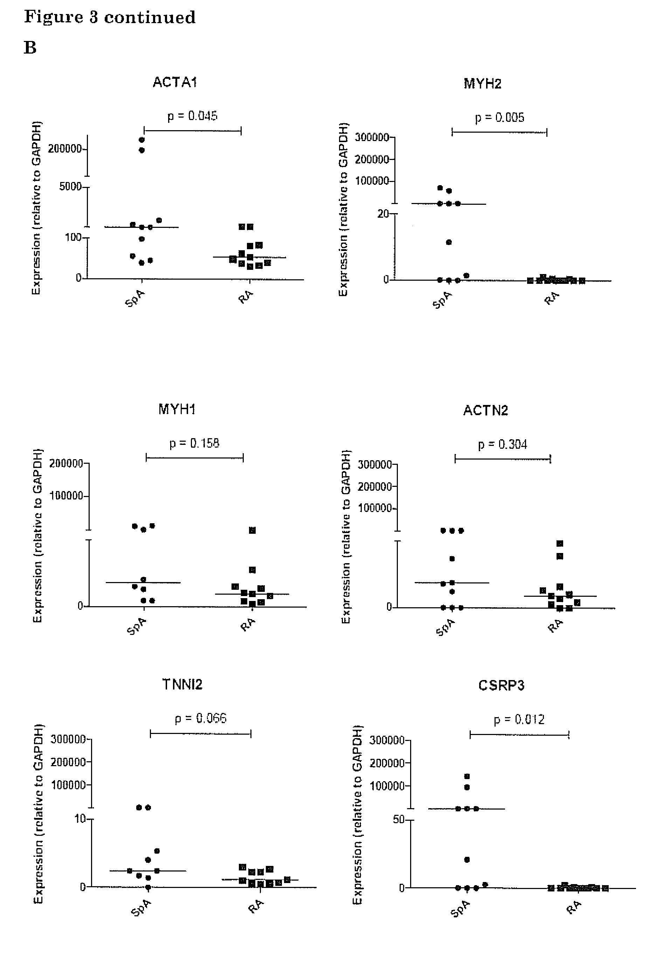

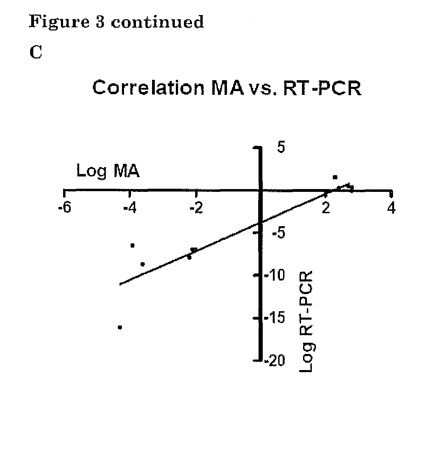

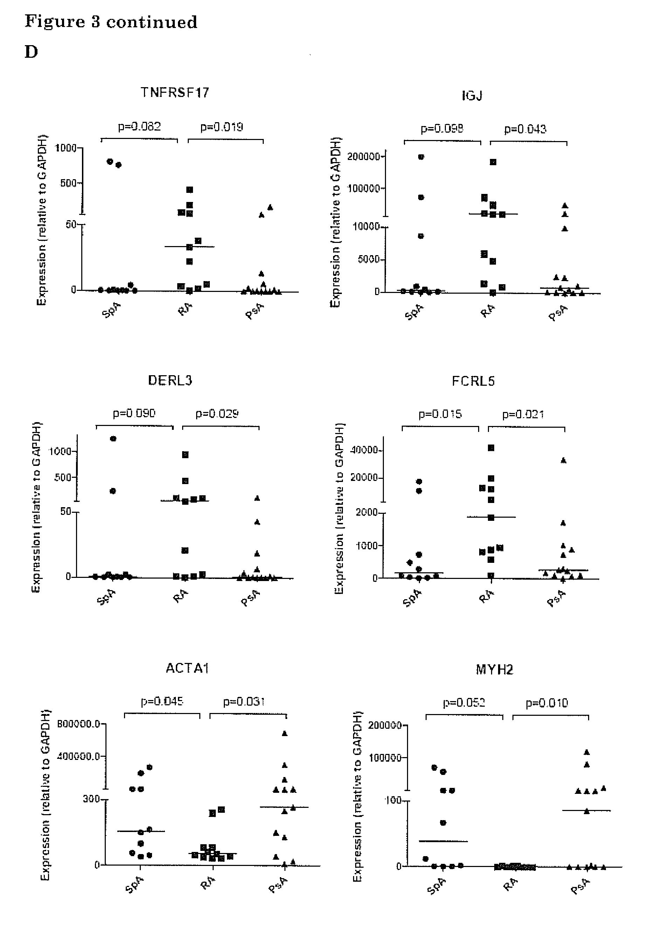

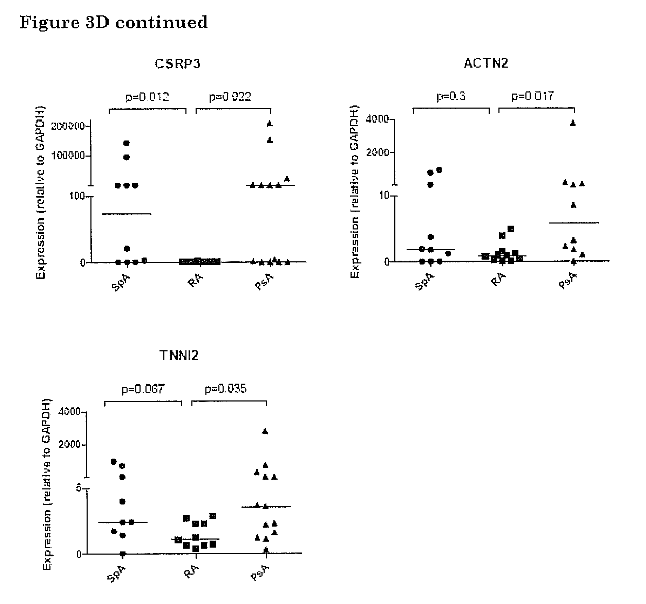

**Figure 3****. Quantitative RT-PCR analysis for biological validation of microarray data on the independent cohort.** (A). RT-PCR of TNFRSF17, IGJ, POU2AF1, DERL3 transcripts which have been determined by microarray analysis as upregulated in synovial tissue samples of RA patients. (B). RT-PCR of ACTA1, MYH2, MYH1, CSRP3, ACTN2, TNNI2 transcripts which have been determined by microarray analysis as upregulated in synovial tissue samples of SpA patients. Data is represented as an individual expression corrected for expression of the control gene (GAPDH) and bars show the median. Relative expression level was considered significant if p<0.05 (Mann-Whitney Test). (C). Correlation of microarray data and real time PCR data. D. RT-PCR of TNFRSF17, IGJ, DERL3, FCRL5, ACTA1, MYH2, MYH1, CSRP3, ACTN2, TNNI2 transcripts on RA, psoriatic and non-psoriatic SpA synovium.

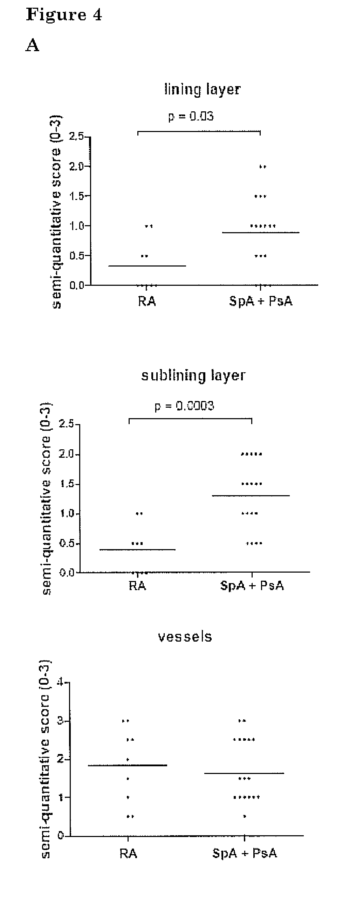

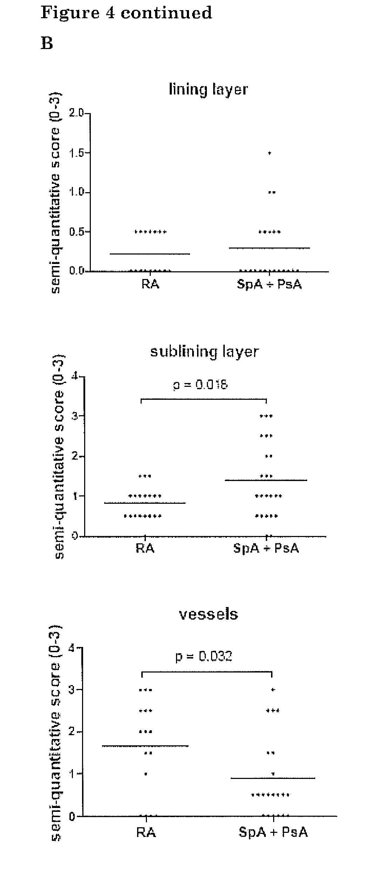

**Figure 4****. Semiquantitative scores for α-SM actin (A) and smooth muscle myosin heavy chain (B) expression in synovial membrane biopsy specimens obtained from RA and SpA joints.** Scatterplots show the individual scores, and bars show the mean. p values were calculated using unpaired T test.

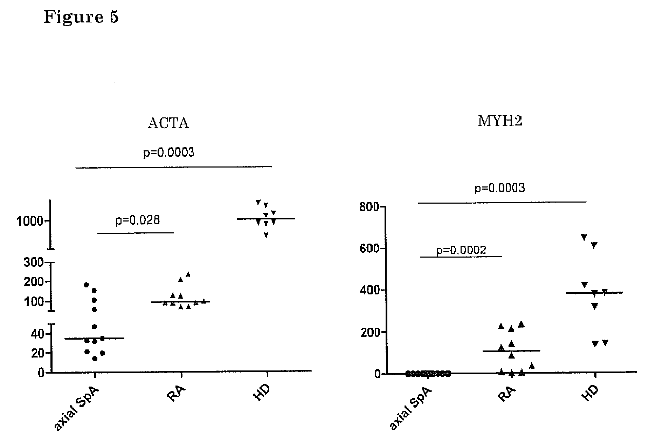

**Figure 5****. Quantitative RT-PCR analysis for biological validation of microarray data on PBMC's of patients with axial SpA (no peripheral disease), RA, and healthy donors.** RT-PCR of ACTA1 and MYH2 transcripts which have been determined by microarray analysis as upregulated in PBMC samples of SpA patients. Data is represented as an individual expression corrected for expression of the control gene (GAPDH) and bars show the median. Relative expression level was considered significant if p<0.05 (Mann-Whitney Test).

### Examples

### Material and methods

### Patients and samples

synovial tissue samples were obtained from three independent patient cohorts: the first cohort was used for microarray analysis and technical confirmation of differentially expressed genes by quantitative RT-PCR, the second cohort was used for an independent confirmation of the biological validity of the gene expression signature, and the third cohort was used for confirmation of candidate pathways at the protein level by immunohistochemistry. All patients fulfilled the classification criteria for either SpA according to the ESSG criteria (1) or RA according to the ACR criteria (2). All patients had active disease with clinical involvement of at least one knee or ankle joint and were not treated with biological therapies. A total of 20-25 synovial tissue biopsies were obtained and processed as described earlier from these inflamed joints (3): 6-8 biopsies were pooled and used for RNA isolation and 6-8 additional biopsies were used for immunohistochemistry. In order to generate robust microarray data, it was aimed to assess homogeneous and representative samples of RA and SpA in cohort 1. At the histological level, samples were selected for a median histological disease activity as assessed by level of synovial infiltration with CD3+ T and CD20+ B lymphocytes in order to match the cohorts for local disease activity. At the clinical level, only patients who were not treated with disease-modifying anti-rheumatic drugs or biologics were selected in order to exclude treatment-induced biases. Additionally, psoriatic SpA (PsA) was excluded in order to obtain a clinically homogeneous SpA cohort. In the independent validation cohort (cohort 2), additionally patients with early disease of less than 1 year and added PsA were selected as an additional SpA group. The demographic and clinical data of the patient cohorts are given in Table 1.

Peripheral blood mononuclear cells (PBMC's) were obtained from patients with axial SpA who did not show symptoms of peripheral disease, patients with RA and healthy donors.

### RNA isolation and microarray hybridisation

RNA was obtained from 6-8 snap frozen synovial tissue biopsies per patient. The RNA isolation, cDNA synthesis, and labelling was carried out using Agilent's Two-Colour Microarray-Based Gene Expression Protocol according to the manufacturer's instructions (G4140-90050, Version 5.7, Agilent Technologies, Palo Alto, CA, USA. RNA quantity was checked with the Bioanalyzer system using the RNA 6000 Nano LabChip kit (Agilent Technologies). As to RNA quality, only the samples with RNA integrity numbers (RIN) values of greater than 6.0 were considered to be of adequate quality to be selected for hybridization. The RNA integrity number (RIN) measures RNA quality on a quantitative scale of 1 (poor) to 10 (high) as measured by a software algorithm that works with the 2100 bioanalyzer and Agilent RNA 6000 Nano LabChip^{®}kit. To correct for gene-specific dye biases, we used a reverse labelling (dye swap) design with common reference. Labeled samples were hybridized to Agilent 4x44K Human Whole Genome microarrays (G4112F). Slides were washed and scanned on an Agilent G2565 Microarray scanner, and data was extracted using Agilent Feature Extraction software (Version.10.1, Agilent Technologies).

### Microarray data analysis

The microarray data were analyzed using the Limma package (4) of the Bioconductor project (software for analysis of genomic data; www.bioconductor.org).Raw intensity data of each spot on the array from all biological and technical replicates were acquired as array-object, including annotation for each spot. Background correction was performed according to the "offset" method that avoids negative intensity values after background subtraction. Background correction was followed by "within array" normalization for each microarray separately using global loess normalization (5) in order to make the two channels on each single array comparable. After normalization, the mean log intensity A and the log ratio M of the intensity data were calculated for each spot on the microarray. These quantities are defined as A=(1/2)(²logR+²logG) and M=²1og(R/G), where R and G are the intensities measured in the red and green channels, respectively (6,7). Next, "between array" normalization was performed in order to obtain similar distributions of the mean log intensities or log ratios across a series of arrays. In particular, scale normalization which ensures that the log-ratios are scaled to have the same median-absolute-deviation (MAD) across arrays was applied (6) (Figure 1). The normalized data were then fitted with a linear model for each probe on the array for each biological replicate (8). Limma uses an empirical Bayes method to estimate the standard error of the log ratios (9). Subsequently, it was tested whether the log ratio of each spot on the microarray differs significantly from zero using a t test. The p-values were adjusted for multiple hypotheses testing by Bonferroni correction.

### Quantitative RT-PCR

Total RNA was isolated from synovial tissue biopsies or PBMC's using RNA Stat-60 (Tel-Test Inc, Friendswood, Tx, USA), treated with DNase I (Invitrogen, Carlsbad, CA, USA), and reverse transcribed using RevertAid™H Minus First Strand cDNA Synthesis Kit (Fermentas, St. Leon-Rot, Germany). RNA concentration was determined with the Nanodrop (Nanodrop Technologies, Wilmington, DE USA). Quantitative real-time PCR was performed using StepOnePlus™ Real-Time PCR System (Applied Biosystems, Foster City, CA, USA). Each 20µl reaction was performed in 96-well format with 5 ng of cDNA, 10 µl of SYBR green PCR Master Mix (Applied Biosystems), and a concentration of 50 nmol of each primer. All reactions were performed in duplicate. The mRNA expression levels were normalized to those of the human housekeeping gene glyceraldehyde 3-phosphate dehydrogenase (GAPDH). Oligonucleotide primers were designed using online tool for Real-time PCR (TaqMan) Primer Design (Genscript) and obtained from Invitrogen. The primers are shown in Table 2.

### Pathway analysis

The microarray data were analyzed using Ingenuity Pathways Analysis tools (Ingenuity Systems, Mountain View, CA, USA, http://www.ingenuity.com), to perform functional pathway analysis and to examine the gene ontology, including biological processes, cellular components, molecular functions and genetic networks. Significantly regulated gene lists containing Agilent probe ID and expression ratio were uploaded into the Ingenuity Pathways Analysis environment. Each gene identifier was mapped to its corresponding gene object in the Ingenuity Pathways Knowledge Base and used as a starting point for generating biological networks and canonical pathway analysis. The Functional Analysis of a network identified the biological functions and/or diseases that were most significant to the genes in the network. Fischer's exact test was used to calculate a p value determining the probability that each biological function and/or disease assigned to that network is due to chance alone. A p value ≤ 0.05 was considered significant. Canonical pathways analysis of the Ingenuity Pathways Analysis Library identified those pathways that were most significant to the data set.

### Immunohistochemistry

Immunohistochemical staining for alpha smooth muscle actin (α -SM actin) and smooth muscle myosin heavy chain (SMMH) was performed on frozen tissue sections of synovial tissue biopsies obtained from patients with RA (n=17) and SpA (n=32). All patients had active disease with at least one clinically affected knee or ankle joint, which was used to obtain synovial biopsies by needle arthroscopy. None of the patients was treated with biological agents. The SpA group consisted of both non-psoriatic (n=21) and psoriatic (n=11) SpA. Synovial biopsy samples (8 per patient) were snap-frozen and mounted in Jung tissue freezing medium (Leica Instruments, Nussloch, Germany). The following primary antibodies were used: monoclonal mouse anti-human Actin (Smooth Muscle), clone 1A4 (Dako, Glostrup, Denamrk) and monoclonal mouse anti-human Smooth Muscle Myosin Heavy Chain, clone SMMS-1 (Dako). Parallel sections were incubated with irrelevant isotype- and concentration-matched monoclonal antibody as negative control. After incubation with the primary antibodies, sections were sequentially incubated with a biotinylated second antibody, a streptavidin-horseradish peroxidase link, and finally with aminoethylcarbazole substrate as chromogen (all from Dako, Glostrup, Denmark). Biopsy sections were stained in a single run and the investigator was blinded to the diagnosis and clinical data in order to minimize technical bias. Expression in the different synovial compartments (vessels, intimal lining layer, and synovial sublining) was quantified on a semiquantative four-point scale by two independent observers blinded for diagnosis. The staining and scoring procedures have been extensively described and validated previously (10-16). Data were represented as median and range.

### Statistics

Results from qPCR and immunohistochemistry were analyzed using non-parametrical statistics, using the Mann-Whitney U test for comparison between groups and the Spearman's rank correlation for correlation between data. P<0.05 was considered statistically significant.

### Results

### Synovial gene expression analysis by microarray

In order to identify novel and unsuspected cellular and molecular pathways involved in the pathogenesis of RA or SpA, pan-genomic microarrays on synovial tissue biopsies were performed and the gene expression profile between both diseases were compared. For this analysis, exclusively non-treated patients were selected and psoriatic SpA was excluded in order to obtain clinically homogenous patient cohorts. All samples were obtained from clinically inflamed knee joints and were selected for a median level of local tissue inflammation as assessed by synovial infiltration with T and B lymphocytes on histology. Furthermore, only samples yielding high RNA quantity and quality were hybridized and only those microarrays passing the technical quality thresholds were retained for further analysis. Using these strict clinical, histological, and technical selection criteria in order to avoid biases due to sample variability and to obtain robust gene signatures, gene expression data from 11 SpA and 7 RA synovial samples were obtained. Using very stringent data analysis criteria, a set of 6270 genes that were highly significantly differentially expressed (p<0.001) between patients SpA and RA was identified. Of these genes, 296 transcripts displayed a more then 2 fold differential expression between SpA and RA synovium (Table 4). 84 transcripts showed a more than 3 fold upregulation in RA versus SpA, with TNFRSF17 showing a 7 fold upregulation. On the other hand, 21 transcripts showed a more than 3 fold upregulation in SpA versus RA, with ACTA1 showing a 15 fold upregulation.

### Technical validation of the gene expression signature by quantitative RT-PCR

In order to confirm the obtained results by an independent technique, 13 transcripts with a known function with an at least 3-fold upregulation in RA synovitis and 8 transcripts with a known function with an at least 3-fold upregulation in SpA synovitis were selected. The expression of these genes was assessed by quantitative real-time RT-PCR (qPCR) on the samples of cohort 1 (RA n=8, SpA n=12), which was also used for the original microarray experiments. Of the 13 genes upregulated in RA, 10 were also significantly upregulated in RA versus SpA synovitis (p<0.05) by qPCR (Figure 2a). An additional 2 genes, CXCL13 (p=0.054) and PIM2 (p=0.059), tended to be significantly higher in RA versus SpA. LAX1 (p =0.211) was not differentially expressed. 8 genes upregulated in SpA were also confirmed to be significantly differentially expressed by qPCR (Figure 2b). Globally, there was a very good correlation between the microarray and qPCR results (r=0.80, p<0.0001)(Figure 2c). Interestingly, the fold-change between RA and SpA for those 21 genes was on average 22 times higher by qPCR than by microarray reaching a 25.4 fold upregulation in RA for PACAP and a 574 fold upregulation in SpA for ACTN2.

### Biological validation of the gene expression signature by quantitative RT-PCR in an independent sample set

In order to verify the biological validity of the gene signatures, the top 11 genes (5 upregulated in RA and 6 upregulated in SpA) were subsequently reanalysed by qPCR in an independent set of synovial tissue samples obtained from 10 non-psoriatic SpA and 11 RA patients (Table 1). These patients were specifically selected for short disease duration in order to ascertain that the gene signature is specific for primary pathways involved in the disease process rather than for secondary down-stream effector mechanisms. Also in this independent sample set, the 5 RA genes were confirmed to be differentially expressed, reaching significance for POU2AF1 (p=0.015) and FCRL5 (p=0.015) and tending to significance for TNFRSF17 (p=0.052), IGJ (p=0.098), and DERL3 (p=0.090) (Figure 3a). As to the 6 SpA genes, ACTA-1 (p=0.045), MYH2 (p=0.005) and CSRP3 (p=0.012) were significantly upregulated in SpA versus RA with a similar trend for TNNI2 (p=0.066). MYH1 and ACTN2 did not reach significance (Figure 3b). As for cohort 1, the qPCR gene expression levels in cohort 2 correlated well with the original microarray results (r=0.76 p= 0.009) (Figure 3c).

As psoriatic SpA were excluded from our original analyses, additionally qPCR for 9 of previously mentioned genes (4 upregulated in RA and 5 upregulated in SpA) was performed in biopsy samples of 13 psoriatic SpA patients (Table 2). As shown in Figure 3d, the psoriatic SpA completely resembled the non-psoriatic SpA, with none of the genes being differentially expressed between both groups whereas 9 genes were significantly different between psoriatic SpA and RA. When the non-psoriatic and psoriatic SpA were pooled in one single SpA set, 9 of the 9 investigated transcripts were differentially expressed between RA and SpA. Taken together, these qPCR data obtained in an independent sample set demonstrated that the identified gene signatures are not only technically but also biologically reproducible without major influence of disease duration and clinical SpA subtype.

### Pathway analysis

Analysis of the highly expressed genes (3 fold up- or down-regulation) indicated that 14 of the 37 known genes upregulated in RA were related to B lymphocyte/plasma cell biology despite the fact that the samples of cohort 1 were histologically matched for infiltration with T and B cells. This was also the case for cohort 2, indicating that the gene expression signature does not merely reflect a different cellular composition of inflamed synovial tissue in RA versus SpA. As to genes upregulated in SpA, 20 out of the 21 known genes were related to myocyte/myofibroblast biology. Taken together, these data indicate the biological consistence of the gene signature. Analysis of the pathways overrepresented in the microarray dataset by Ingenuity revealed cytotoxic T lymphocyte-mediated apoptosis of target cells, communication between innate and adaptive immune cells, hepatic fibrosis/hepatic stellate cell activation, and Type I Diabetes Mellitus signaling as canonical pathways in RA (Table 5). Calcium signaling, actin cytoskeleton signaling, ILK signaling, cellular effects of Sildenafil, and hepatic fibrosis/hepatic stellate cell activation were the canonical pathways overrepresented in SpA (Table 5). More detailed analysis of these and other pathway with different software tools is currently ongoing in order to define pivotal pathways in RA and SpA synovial inflammation.

### Validation at the protein level by immunohistochemistry

As indicated, almost all of the top-ranking differentially expressed genes identified in SpA versus RA were related to muscle genes. Some of these genes showed a more than 100-fold upregulation in SpA versus RA synovitis in the qPCR experiments (Figure 2b and 3b). This signature is unexpected as myocytes are not present in the synovial tissue with the exception of a few smooth muscle cells around small arteries. As vascularity is more pronounced in SpA than in RA synovitis (10,14,15), we performed immunohistochemistry on a large set of SpA and RA synovial biopsies in order to confirm the microarray data on the protein level and to exclude biases due to differences in vascularization. Immunostainings revealed the expression of α-smooth muscle actin and smooth muscle myosin heavy chain not only around blood vessels, as in healthy synovium, but also in the intimal lining layer and the synovial sublining. α-smooth muscle actin positive cells were significantly increased in SpA versus RA in the synovial sublining (p=0.035 ) as well as in the intimal lining layer (p=0.003) despite similar staining around vessels (Figure 4a). Similarly, smooth muscle myosin heavy chain positive cells were significantly increased in SpA versus RA in the synovial sublining (p=0.018) whereas the expression around vessels was lower in SpA than in RA synovitis (p=0.031) (Figure 4b). These data confirm the gene signature at the protein level and strongly suggest the specific presence of myofibroblasts in the intimal lining layer and the sublining of SpA synovium.

### Biological validation of the gene expression signature by quantitative RT-PCR in peripheral blood mononuclear cells (PBMC)

The data summarized above demonstrate the presence of a robust and reproducible gene expression signature in RA versus SpA synovitis, this is in the target tissue of the disease. We next explored whether genes that were differentially expressed in synovial tissue of SpA versus RA were also differentially expressed in the peripheral compartment, this is in PBMC. Two 'myo'-genes (ACTA and MYH2) that were strongly upregulated in SpA synovitis by microarray as well as qPCR were analyzed by qPCR in PBMC of an independent cohort of 11 SpA patients, 10 RA patients and 8 healthy donors. Importantly, we selected specifically SpA patients with exclusive axial disease as in this subset of patients analysis of inflamed peripheral joint tissue is not possible. Both ACTA and MYH2 appeared to be differentially downregulated in axial SpA patient PBMC's compared to both RA patient and healthy donor PBMC's (Figure 5), demonstrating an inverse gene signature in peripheral blood compared to synovial tissue. These data show that analysis of the expression of our target genes in PBMC is able to discriminate between the different disease states. Moreover, these data show that the differential expression is not restricted to SpA with peripheral joint disease but can also be found in pure axial SpA.

**Table 1. Demographic and clinical data of the patient cohorts. None of the patients was treated with biological drugs. Data are represented as mean and standard deviation for age (years), disease duration (years), number of swollen joints, and C-reactive protein serum levels (mg/L). Gender is given as male/female. Presence of autoantibodies and use of anti-rheumatic drugs are indicated if positive. RA = rheumatoid arthritis. SpA = spondyloarthritis. PsA = psoriatic spondyloarthritis. SJC = swollen joint count. CRP = C-reactive protein. RF = rheumatoid factor. ACPA = anti-citrullinated protein antibodies. DMARD = disease modifying anti-rheumatic drugs.**

| | Cohort 1 | | Cohort 2 | | |
|---|---|---|---|---|---|
| | RA | SpA | RA | SpA | PsA |
| | (n=8) | (n=12) | (n=11) | (n=10) | (n=13) |
| Age | 56 ± 15 | 44 ± 17 | 56 ± 13 | 35 ± 14 | 47 ± 12 |
| Gender | 4/4 | 8/4 | 4/7 | 7/3 | 9/4 |
| Disease | 3.7 ± | 6.8 ± | 0.5 ± | 0.6 ± 0.4 | 0.9 ± |
| duration | 2.9 | 5.8 | 0.3 | | 0.8 |
| SJC | 10 ± 5 | 3 ± 3 | 12 ± 10 | 2 ± 1 | 2 ± 3 |
| CRP | 45 ± 22 | 43 ± 47 | 34 ± 48 | 21 ± 39 | 12 ± 13 |
| RF or ACPA | 4 | 0 | 9 | 0 | 0 |
| DMARD | 0 | 0 | 0 | 3 | 2 |

**Table 2. Primers used for quantitative RT-PCR. The genes were selected from the microarray data as being upregulated more then 3 fold in RA (13 genes selected from 37 known transcripts) or in SpA (8 genes selected from 21 known transcripts).**

| **Gene/primer (GenBank accession number)** | **Forward** | **Reverse** |
|---|---|---|
| TNFRSF17 | | |
| (NM_001192) | CTCTGGACCTGTTTGGGACT | TTCCAGGTCAATGTTAGCCA |
| IGJ(NM_144646) | CTTCCAGGATCATCCGTTCT | GGTGAGGTGGGATCAGAGAT |
| POU2AF1 (NM_006235) | AAGGAACTGCTGAGGAGGAA | CACAGAACCTTCCATGTCCA |
| DERL3 (NM_198440) | CAACATGCTCTTCGTGTTCC | AGTCCCAGCAGGGTCATAAG |
| FCRL5 (NM_031281) | GGTGCATATCGCTGTACTGGA | AATGGCTCTTGGACTTGGATTTTGAC |
| CXCL13 (NM_006419) | AATGAGCCTGGACTCAGAGC | ACCTCCAGAACACCTTGGAC |
| PACAP (NM_016459) | GATGTACTCAGCCCACATGC | CCACATCTGGTAAGCCACAG |
| PNOC (NM_006228) | GCAGCTGCAGAAGAGATTTG | TGCTCAGGACCAAGTATTGC |
| SPP1 (NM_000582) | TCACCTGTGCCATACCAGTT | CCACAGCATCTGGGTATTTG |
| IRF4 (NM_002460) | CAGTGGGCTGTTTCTGCTTA | CAAGTGGAGGTCTTTGGGAT |
| LAX1 (NM_017773) | AACGGAAGAAGCGACAAGTT | TTGGCTCTTTGTCTGGTTTG |
| EAF2 (NM_018456) | GCTGAGGTGGCAGATAGTGA | CGACGGTCTAGGTGTGAGAA |
| PIM2 (NM_006875) | AATGGTCAGAAGAGCCATCC | CCCTGATCCTCAATCCCTTA |
| ACTA1 (NM_001100) | TGCCCATTTATGAGGGCTAC | CTCAGCTGTGGTCACGAAG |
| MYH2 (NM_017534) | GGCTGGAGAAGAACAAGGAC | TCCCTCTCCTTCAGCAGTTT |
| MYH1 (NM_005963) | ACAAACTGCAAGCAAAGGTG | GGAGAGGTTGACGTTGGATT |
| CSRP3 (NM_003476) | AGCAACCCTTCCAAATTCAC | CCATAACCTTCTCAGCAGCA |
| ACTN2 (NM_001103) | ACAAGATGGAGGAGATTGCC | TTCAGCTGGTTCATCTGGTC |
| TNNI2 (NM_003282) | CAACCTGAAGCAGGTCAAGA | ATGCCAGACTTCTCCTCGAT |
| CYP26B1 (NM_019885) | CCATCAACGTGTACCAGGAG | ACAGGCAGGGAGAAGACATT |
| TCAP (NM_003673) | AAGGTACTGGTGAGGGCAAG | GCTTCTTCTGCCTAGAGCGT |

**Table 3. Transcripts displaying a more then 1.5 fold differential expression between SpA and RA in synovial tissue (FC: fold change).**

| **FC** | **p.value** | **Systematic Name** | **Gene** | **Probe Sequence** |
|---|---|---|---|---|
| 7,14 | 1,0E-21 | NM_001192 | TNFRSF17 | GATCTCTTTAGGATGACTGTATTTTTCAGTTGCCGATACAGCTTTTTGTCCTCTAACTGT |
| 6,97 | 4,7E-18 | AY172962 | AY172962 | AAGATAGCAGCCCCGTCAAGCGGGAGTGGAGACCACCACACCCTCCAAACAAAGCAACAA |
| 6,96 | 1,0E-21 | NM_144646 | IGJ | TTGGGTGATGTAAAACCAACTCCCTGCCACCAAAATAATTAAAATAGTCACATTGTTATC |
| 6,28 | 1,9E-19 | NM_006235 | POU2AF1 | TTTTCTGGGAAATGACTTTTCTGGGAAATGACAGTTTCTTTGACATATTTTCTTTGCCCA |
| 5,80 | 1,1E-19 | Bl521983 | Bl521983 | GAAGTCACTTATGAGACACACCAGTGTGGCCTTGTTGGCTTGAAGCTCCTCAGAGGAGGG |
| 5,72 | 5,5E-19 | AW136683 | AW136683 | TCTGTCATAAGTGTAGCAGGTCTCTGTAGCACTGTCTTCATCACAGATATTGCTCTGGGT |
| 5,66 | 5,6E-18 | X57802 | X57802 | ATTCTCTGGCTCCAACTTTGGGAACACAGCCACTCTGACCATCAGCGGGACCCAGGCTAT |
| 5,47 | 1,1E-17 | NM_020070 | IGLL1 | TCCAAGCCAACAAGGCTACACTGGTGTGTCTCATGAATGACTTTTATCCGGGAATCTTGA |
| 5,33 | 1,4E-17 | NM_001013618 | CTA-246H3.1 | AACAAGGCCACACTGGTGTGTCTCATGAATGACTTCTATCTGGGAATCTTGACGGTGACC |
| 5,15 | 9,4E-18 | NM_014792 | KIAA0125 | CCATTTTAAAGATGGCTACTTAGGACCATATGGATGTTGTACTGATGTCATTTGACCACG |
| 5,15 | 1,0E-21 | NM_198440 | DERL3 | AGGCCTAAGAGGCTTCTGGCAGCTTCCATCCTACCCATGACCCCTACTTGGGGCAGAAAA |
| 4,92 | 1,3E-18 | NM_031281 | FCRL5 | TGGCATATGGACTGAAAGAAACTATGCTATTGGATCTCCTGGATCTCCAGCTTGCTGACT |
| 4,80 | 4,2E-16 | ENST00000216649 | ENST00000216649 | CTCAAGAGTCGAGTCACCATATCAGTGGACACGTCCAAGAACCAGTTCTCCCTGAGGCTG |
| 4,70 | 1,0E-19 | NM_006419 | CXCL13 | CAAGAGGCAAAGGAATCCATGTAGTAGATATCCTCTGCTTAAAAACTCACTACGGAGGAG |
| 4,67 | 1,3E-18 | ENST00000377233 | ENST00000377233 | TTGTATTGGTACCTGCAGAAGCCAGGCCAGCCTCCACAGCTCCTGATCTATGAAGTTTCC |
| 4,53 | 2,8E-14 | NM_016459 | PACAP | GGACATGTTTGCACTACTTGGGGGAGTTTGGAGAAGACCAGATCTATGAAGCCCACCAAC |
| 4,48 | 9,4E-18 | AF076205 | AF076205 | CCTCCAGTCTGAGGATGAGGCTGACTATTACTGTCAAACCTGGGGCACTGGCATTGGGGT |
| 4,39 | 3,1E-17 | ENST00000327926 | ENST00000327926 | TATTTGAATTGGTACCTGCAGAAGCCAGGCCAGTCTCCACAGCTCCTGATCTATGAGGTT |
| 4,38 | 0,0E+00 | NM_006228 | PNOC | GCCACTGCCATAACTTGTTTGTAAAAGAGCTGTTCTTTTTGACTGATTGTTTTAAACAAC |
| 4,35 | 5,4E-14 | BC073764 | IGKC | TCAGGGTATTAGCAGCTGGTTAGCCTGGTATCAGCAGAAACCAGAGAAAGCCCCTAAGTC |
| 4,29 | 4,3E-19 | ENST00000379877 | ENST00000379877 | GTGCAGCTGCAGGAGTCGGGCCCAGGACTGGTGAAGCCTCCGGGGACCCTGTCCCTCACC |
| 4,27 | 3,1E-16 | BX110985 | BX110985 | CTCTGTGGGGTAGAGATGAAAAGGCTCACTCAGTTCCAGCCCCTGCAGATCCCCCAGGAC |
| 4,24 | 7,2E-17 | BC070333 | IGHV1-69 | CAGGCCCCTGGACAAGGGCTTGAGTGGATGGGAAGGATCATCCCTATCCTTGGTATAGCA |
| 4,19 | 0,0E+00 | THC2347909 | THC2347909 | GGAAATGATGTTTGTCTAAAATGGCCAGAGACAACTTTTATAAGCTTCCAGGAAGTGGAG |
| 4,16 | 1,4E-11 | ENST00000377226 | ENST00000377226 | GAAAGCTCCTAAGCTCCTGATCTATGATGCCTCCAGTTTGGAAAGTGGGGTCCCATCAAG |
| 4,15 | 7,0E-20 | NM_000582 | SPP1 | TTCCACAGCCATGAATTTCACAGCCATGAAGATATGCTGGTTGTAGACCCCAAAAGTAAG |
| 4,10 | 5,9E-11 | ENST00000259219 | ENST00000259219 | TGATCTATGCTGCATCCAGTTTGCAGTCGGGGGTCCCATCTCGGTTCAGTGGCAGTGGAT |
| 4,08 | 4,5E-17 | ENST00000312946 | ENST00000312946 | GGGCAGTCTCCACAGCTCCTGATTTATAGGGTTTCCAATCATCTTTCTGGGGTCCCAGAC |
| 4,06 | 7,3E-15 | BC012876 | BC012876 | TCTGACACCTCAGCCTCCCTGGCCATCACTGGACTCCAGGCTGAAGATGAGGCTGATTAT |
| 3,86 | 4,3E-18 | NM_014479 | ADAMDEC1 | TGACTTAGTTCTGCCCTTTGGAGAACAAAAGAAAGCAGTCTTCCATCAAATCACCTTAAA |
| 3,80 | 0,0E+00 | NM_002460 | IRF4 | TTTTGTATTGATTTTCACAGCTTTGAGGAACATGCATAAGAAATGTAGCTGAAGTAGAGG |
| 3,75 | 2,3E-17 | NM_012390 | SMR3A | CACCCTATGGTCCAGGGAGAATTCAATCACACTCTCTTCCTCCTCCTTATGGCCCAGGTT |
| 3,66 | 1,3E-14 | AF063695 | AF063695 | CTGTCAGGTGTGGGATAGTACTAGTGATCATTATGTCTTCGGAACTGGGACCAAGGTCGC |
| 3,63 | 1,2E-20 | BC009479 | TTLL3 | GCTATATCCGCTTTTCCACGCAGCCCTTCTCCCTGAAGAACCTGGACAAGTGAGCCCCTC |
| 3,63 | 9.1E-18 | X57818 | X57818 | ACCCTCAGTGTCCGTGTCCCCAGGACAGACAGCCAGCATCACGTGCTCTGGACATAAATT |
| 3,63 | 4,0E-21 | AJ252276 | AJ252276 | AGATGGTGAAACAATATACGCAGAGAAGTTCCAGGGCAGAGTCACCATAACCGCGGACAC |
| 3,62 | 7,1E-14 | AY998685 | AY998685 | AATAAGAACTACTTAGTTTGGTACCAGCAAAGACCAGGACAGCCTCCTAAAATGCTCATT |
| 3,55 | 4,8E-20 | NM_017773 | LAX1 | TCGCACATGACTGTTTATAGCAGCTTCATTCATAATTACCAAAAACTAGAAATAACCCAG |
| 3,54 | 8,5E-13 | A_24_P384604 | A_24_P384604 | GGTTCAATGGCAGTGGGTCAGGCACTGATTTCACACTGAAAATCAGCCGGGTGGCTGAAG |
| 3,53 | 1,8E-10 | ENST00000328419 | ENST00000328419 | AGCCTGATGCCTGAACAGTGGAGATCCCGCAGCAGCTACAACTGCTGGGCCATGCATAAA |
| 3,51 | 2,6E-13 | ENST00000379884 | ENST00000379884 | ACACAGTCTACATGGAGCTGAGAAGCCTGAGATCTGAGGACACGGCCGTGTATTACTGTG |
| 3,51 | 5,1E-14 | ENST00000331195 | ENST00000331195 | ATACTGGTACCAGCAGCTCCCAGGAACGGCCCCCAAACTCCTCATCGAAAGGAATAATCA |
| 3,50 | 5,9E-13 | ENST00000360623 | ENST00000360623 | TCTCAGTTATTTATAGCTGTGGTAGCACATACTACGCAGACTCCGTGAAGGGCCGATTCA |
| 3,47 | 5,3E-15 | THC2428956 | THC2428956 | CATAGTTATAACCACCAACGTCACTGCTGGTTCCAGTGCAGGAGATGGTGATCGACTGTC |
| 3,46 | 6,5E-11 | ENST00000295410 | ENST00000295410 | GACAGAGTCACCATCACTTGTCGGGCGAGTCAGGGAATTAGCAATTATTTAGCCTGGTTT |
| 3,45 | 6.1E-14 | AJ399872 | AJ399872 | CAGTTTATTACTGTCAGCAGCGTCGCAATTGGCCTTCTTTCGGCGGAGGGACCCAGCTCA |
| 3,42 | 1,2E-15 | D83692 | D83692 | ACAACAGAATACGCCGCGTCTGTGAAAGGCAGATTCACCATCTCAAGAGATGATTCCAAA |
| 3,41 | 2,9E-17 | ENST00000361266 | ENST00000361266 | TGCAATCTGGGTCTGAGTTGAAGAAGCCTGGGGCCTCAGTGAAGGTCTCCTGCAAGGCTT |
| 3,35 | 3.1E-16 | ENST00000360329 | ENST00000360329 | ACATTGGGAAGGGATGGAGGATGGACGGAGGAAGGACGTGCAGTTGCAGCTCTTTCTGCA |
| 3,35 | 1,0E-21 | NM_018456 | EAF2 | CAGGATTCCTGATATAGATGCCAGTCATAATAGATTTCGAGACAACAGTGGCCTTCTGAT |
| 3,30 | 2,0E-11 | ENST00000379895 | ENST00000379895 | ATGGTATGATGGAAGTAATAAATACTATGCAGACTCCGTGAAGGGCCGATTCACCATCTC |
| 3,27 | 2,0E-14 | BC023973 | IGLV6-57 | AACTCTGCCTCCCTCACCATCTCTGGACTGAGGACTGAGGACGAGGCTGACTACTACTGT |
| 3,27 | 8,0E-15 | XM_372630 | LOC390712 | CTGTGAAGGGCAGATTCACCATCTCCACAGACAACTCAAAGAACACGCTCTACCTGCAAA |
| 3,26 | 2,5E-16 | AF103312 | AF103312 | AATGAACAGCCTGAAAACCGAGGACACGGCCATGTATTACTGTACTAGCGCGCCAAATAT |
| 3,25 | 4,9E-18 | NM_006875 | PlM2 | GGTGAGGGGACCCTACTTTGTTATCCCAAGTGCTCTTATTCTGGTGAGAAGAACCTTAAT |
| 3,25 | 6,4E-16 | AB063923 | AB063923 | CCTCAGTGAAGGTCTCCTGCAAGGCTTCTGGTTACACCTTTACCAGTCATGGTATCACCT |
| 3,24 | 7,8E-12 | NR_001564 | XIST | AATATTTCAATGCCTATTCTCTGCAAGGTACTATGTTTCGTAAATTAAATAGGTCTGGCC |
| 3,22 | 1,8E-13 | ENST00000328018 | ENST00000328018 | CAATAGCCTGGAAGCTGAAGATGCTGCAACGTATTACTGTCATCAGAGTAGTAGTTTACC |
| 3,22 | 2,8E-11 | ENST00000359488 | ENST00000359488 | TAGGAGACAGAGTCACCATCACTTGCCGGGCAAGTCAGACCATTAGCAGCTATTTAAATT |
| 3,21 | 1,2E-11 | BX640624 | l.GHA1 | TCGGTGATCAGCACTGAACACAGAACTCACCATGGAGTTTGGACTGAGCTGGGTTTTCCT |
| 3,21 | 1,5E-11 | ENST00000283657 | ENST00000283657 | GGCCACCATCAACTGCAAGTCCAGCCAGAGTGTTTTATACAGCTCCAACAATAAGAACTA |
| 3,20 | 1,5E-12 | AF471475 | AF471475 | ACATTAGTGATAGTGGTAGTACCATGTATTACGCAGACTCTGTGAAGGGCCGATTCACCA |
| 3,19 | 9,1E-15 | BC095489 | IGKC | ACATCCAGATGACCCAGTCTCCATCCTCCCTGTCTGCATCTGTAGGAGGCAGAGTCACCA |
| 3,18 | 3,2E-11 | XM_942451 | LOC652791 | GCACAAGCTACGCAGACTCCATGAAGGGCCAATTCACCATCTCCAGAGACAATGCTAAGA |
| 3,18 | 9,5E-16 | L04336 | L04336 | GTTTCCTGCAAGGCTTCTGGATACACCTTCACTAGCTATGCTATGCATTGGGTGCGCCAG |
| 3,17 | 1,1E-17 | M_017709 | FAM46C | CCTATCCACATCTTTCCAAGATAGACACTAACATGTCATGTCCCAAACATTAGCACGTGG |
| 3,17 | 1,0E-21 | NM_000096 | CP | TCACGGCCATAGCTTCCAATACAAGCACAGGGGAGTTTATAGTTCTGATGTCTTTGACAT |
| 3,17 | 1,6E-11 | BC034142 | IGKV1-5 | GATTTTACACTGAAAATCAGCAGAGTGGAGGCTGAGGATGTTGGGGTTTATTACTGCATG |
| 3,15 | 2,8E-19 | NM_033014 | OGN | AACTAATGATCACAGCTATTATACTACTTTCTCGTTATTTTGTGTGCATGCCTCATTTCC |
| 3,12 | 5,9E-19 | NM_012261 | C20orf103 | AGAAAACGACTAATGTAACTATGCAGAGTTGTTTGGACTTCTTCCTGTGCCAGGTCCAAG |
| 3,11 | 6,7E-15 | AF035035 | LOC647460 | GTCTGAAGATTTTGCAACTTATTACTGTCAACAGTATTATAGTTTCCCTCCGACGTTCGG |
| 3,11 | 1,3E-13 | A_24_P204574 | A_24_P204574 | GAGGATGAGGCTGATTATTACTGTGCAGCATGGGATGACAGCCTGAGTGGTCCCACAGTG |
| 3,11 | 1,4E-19 | NM_033280 | SEC11L3 | TTCAAGTATGCTCTTTTGGCTGTAATGGGTGCATATGTGTTACTAAAACGTGAATCCTAA |
| 3,09 | 2,0E-21 | NM_012081 | ELL2 | TGTCTTTTCAAAGTGCTGCCAGTTGAAAAGGGAAGCATTATGTTTACAAATCTGTTTTGA |
| 3,09 | 6,5E-11 | ENST00000379879 | ENST00000379879 | CTTCAAATGAACAGCCTGAGAGCCGAGGACACGGCCGTGTATTACTGTGCGAGAGACACA |
| 3,08 | 2,7E-18 | NR_001442 | FER1L4 | GAAGTCTTTCTTACCCATGTGAGCTACCCCAGAGTCTAGTGCTTCCTCTGAATAAACCTA |
| 3,06 | 2,3E-08 | M_001040077 | IGHG1 | TGAATGGCAAGGAGTACAAGTGCAAGGTCTCCAACAAAGCCCTCCCAGCCCCCATCGAGA |
| 3,06 | 5,6E-15 | AF267875 | AF267875 | TGAGGACGAGGCTGACTACTACTGTCAGTCTTATGATAGCAGCATCGGGAATGTGATATT |
| 3,03 | 5,1E-14 | U21252 | U21252 | CCAGAGATGATTCCAAGAACACGGCGTATCTGCAAATGAACAGCCTGAAAACCGAGGACA |
| 3,03 | 1,0E-08 | NM_005570 | LMAN1 | TTGACTACCATTTTCCTGTGTACTTCATCTATTTGTGTACAAAATGATGTCGTTTTGAGG |
| 3,03 | 2,7E-17 | NM_001006946 | SDC1 | ACCCTGGGCCCTGGGCTGGAATCAGGAATATTTTCCAAAGAGTGATAGTCTTTTGCTTTT |
| 3,02 | 7,4E-10 | Y11328 | Y11328 | GTGGCACATACTATGCAGACTCCGTGAAGGGCCGATTCACCATCTCCAGAGACAATGCCA |
| 3,02 | 8,6E-11 | ENST00000360102 | ENST00000360102 | TGGGTCCGCCAGGCTCCAGGGAAGGGACTGGAGTGGGTTTCATACAGTAGTGGTAATAGT |
| 3,01 | 1,4E-15 | A_24_P203886 | A_24_P203886 | ATCTCCTGCAGGTCTAGTCGCAGCCTCCTGCATAGTAATGGAAACACCTATTTACATTGT |
| 3,00 | 3,9E-18 | NM_006398 | UBD | ATTTGGGTGGGATGGGTAGGATGAAGTATATTGCCCAACTCTATGTTTCTTTGATTCTAA |
| 3,00 | 9,3E-10 | ENST00000383048 | ENST00000383048 | AGTTTGGGCTGAGCTGCCTTTTTCTTGTGGCTATTTTAAAAGGTGTCCAGTGTGAGGTGC |
| 2,99 | 5,7E-18 | NM_005079 | TPD52 | AACTGCTTACTCAACACTACCACCTTTTCCTTATACTGTATATGATTATGGCCTACAATG |
| 2,99 | 2,0E-16 | X01147 | X01147 | TGGGCATCACCGGACTCCAGACTGGGGACGAGGCCGATTATTACTGCGGAACATGGAATA |
| 2,98 | 8,9E-14 | NM_000450 | SELE | GATGTGATAAGGATCAGAACAGCAGAGGTTCTTTTAAAGGGGCAGAAAAACTCTGGGAAA |
| 2,97 | 4,2E-19 | AF343666 | AF343666 | GGAGGAACCTCTTGGAGAAGCCAGCTATGCTTGCCAGAACTCAGCCCTTTCAGACGTCAC |
| 2,97 | 4,4E-15 | AK026408 | AK026408 | TACTGTGGTACATGGCACAGCAACTCTAAGACTCACACAGTGCTCCAGACCCATGAGGAA |
| 2,96 | 1,9E-15 | THC2321023 | THC2321023 | GGTGCCTTCCAGCCTGACCCCTCCTCTGAGCACCACCTGCCAGTGTGACAGCTCCATCTG |
| 2,94 | 2,6E-10 | ENST00000307840 | ENST00000307840 | GCAGCCTGCAGCCTGAAGATATTGCAACATATTACTGTCAACAGTATGATAATCTCCCTC |
| 2,94 | 3,8E-14 | AF067420 | IGHA1 | TCTATGATAGTAGTGGTCCCTTTGACTACTGGGGCCAGGGAACCCTGGTCACCGTCTCCT |
| 2,94 | 1,4E-12 | AY003763 | AY003763 | GCTACAAGTTTGACTACTGGGGCCAGGGAACCCTGGTCACCGTCTCCTCAGCATCCCCGA |
| 2,93 | 6,3E-14 | BC008026 | MGC16025 | GCCTCTGACCCTGATGAAATAGCTTCGGTGGCATTTGCATCAAGATCATGTTAGTGTCAT |
| 2,92 | 2,4E-13 | BC032451 | BC032451 | AGCAGCTCCTTAGCCTGGTACCAGCAGAAACCTGGCCTGGCGCCCAGGCTCCTCATCTAT |
| 2,92 | 7,7E-15 | ENST00000354689 | ENST00000354689 | CTGTGAAAGGCAGATTCACCATCTCAAGAGTTGATTCAAAAAACACGCTGTATCTGCAAA |
| 2,92 | 3,2E-15 | ENST00000358917 | ENST00000358917 | AGGGGAAGGACTGTGTCCCTGTGTGATGCTTTTGATATCTGGGGCCAAGGGACAATGGTC |
| 2,91 | 4,1E-11 | AB063751 | AB063751 | CTGGATTCATCTTCAGTAATTACACCATGAACTGGGTCCGCCAGGCTCCAGGGAAGGGGC |
| 2,89 | 7,0E-21 | NM_000439 | PCSK1 | GAGTTTAACATGTGTGGTCTTGGTATTCTTAAGGGAACTTCCACATTATACATTTGATGT |
| 2,88 | 5,2E-16 | BC022362 | BC022362 | CAGCAGAGGCCAGGCCAATCTCCAAGGCGCCTAATTTATAAGGTTTCTAACTGGGACTCT |
| 2,87 | 6,8E-16 | M_001040075 | LOC651928 | TGCTAATGCTCTGGGTCCCTGGATCCAGTGGTGATATTGTGATCGCCCAGACTCCACTCT |
| 2,87 | 8,4E-20 | NM_002281 | KRT81 | TTGAAAGACCCCTCCCACTCCTGGCCTCACATTTCTCTGTGTGATCCCCCACTTCTGGGC |
| 2,86 | 5,4E-14 | NP102468 | NP102468 | TTACCATCAGTAGCCTGGAAGCTGAAGATGCTGCAACATATTACTGTCAGCAGGGCAATA |
| 2,86 | 5,0E-21 | NM_001775 | CD38 | TGAAAAATCCTGAGGATTCATCTTGCACATCTGAGATCTGAGCCAGTCGCTGTGGTTGTT |
| 2,85 | 1,9E-11 | AF035034 | AF035034 | GCAGGGTGGAGGCTGAGGATGTTGGGGTTTATTACTGCATGCAAGGTACACACTGGCCTC |
| 2,85 | 7,9E-11 | BC024289 | BC024289 | GCCAAGAACACGCTGTATCTGCAAATGAACAGTCTGAGAGCCGAGGACACGGCTGTGTAT |
| 2,84 | 5,5E-10 | AF471454 | AF471454 | ATGCAGACTCCGTGAAGGGCCGATTCACCATCTCCAGAGACAATTCCAAGAACACGGTGT |
| 2,83 | 3,9E-13 | A_24_P110487 | A_24_P110487 | CAGGATTTTATGGTGATAGTGGTTACACAAACTACGCAGACTCTGTGAAGGGCCGATTCA |
| 2,82 | 7,4E-14 | Z18824 | Z18824 | CTCCAGGGAAGGGGCTGGAGTGGGTTTCATACATTAGTAGTAGTAGTAGTACCATATACT |
| 2,82 | 1,0E-21 | M_002610 | PDK1 | TGGACACGAAGTGATTTTTGTAACCTGAGCAGTTAATGAATGTGCCAACATTTTCTAGGA |
| 2,82 | 5,1E-14 | NM_002922 | RGS1 | GAAGTCCCATTAACTTAAAGTATATGTTTTCAAATTGCCATTGCTACTATTGCTTGTCGG |
| 2,81 | 1,0E-13 | AJ245002 | AJ245002 | TTTGACTGGTTATTATAGAAGGGGGTTTGACTACTGGGGCCAGGGAACCCTGGTCACCGT |
| 2,80 | 1,7E-12 | XM-496145 | LOC440361 | ATTAAAAGGTGTCCAGTGTGAGGTTCAGCTGGTGCAGTCTGGGGGAGGCTTGGTACATCC |
| 2,79 | 1,4E-12 | NM_006274 | CCL19 | AGTGTGAGTGTGAGCGAGAGGGTGAGTGTGGTCAGAGTAAAGCTGCTCCACCCCCAGATT |
| 2,78 | 1,8E-08 | NM_002416 | CXCL9 | TGTGACCCACTTACCTTGCATCTCACAGGTAGACAGTATATAACTAACAACCAAAGACTA |
| 2,78 | 2,2E-09 | AK128476 | IGHA1 | TGCTGAGTTGGGTTTTCCTTGCTGCTATTTTAAAAGGTGTCCAGTGTGAGGTGCAGCTGG |
| 2,76 | 1,8E-14 | NM_021181 | SLAMF7 | GGAGACCTCCCTACCAAGTGATGAAAGTGTTGAAAAACTTAATAACAAATGCTTGTTGGG |
| 2,75 | 1,7E-10 | AJ519285 | AJ519285 | CAATTCCAAGAACACGCTGTATCTGCAAGTGAACAGCCTGAGAGTCGAGGACACGGCCCT |
| 2,75 | 2,3E-16 | BX648200 | BHLHB8 | CCCGTGCCCCCACTGTAATTGTGCTGAAAATGTTTCTCAAATGACCCAAATTGGCTCTTA |
| 2,75 | 3,4E-16 | AK000347 | LAX1 | ATTTGTAATTTTGTAAACCCTGTCTCTCATATTTTGTAAAAATACCAAAATTAGCGGTGG |
| 2,73 | 9,7E-17 | DB362335 | DB362335 | CATCTTCCATTCCTTCCCAAATTATGGAAGTAAGGTTCTTCTCACCAGAATAAGAGCACT |
| 2,72 | 5,3E-17 | NM_001770 | CD19 | TACATGCCAGTGACACTTCCAGTCCCCTTTGTATTCCTTAAATAAACTCAATGAGCTCTT |
| 2,72 | 4,6E-15 | NM_003116 | SPAG4 | CGATTTCGCGGTCTTTGGCCTCCAGGTTTATGATGAAACTGAAGTTTCCTTGGGGAAATT |
| 2,71 | 5,5E-16 | CR621698 | CR621698 | ATGTTGCTCCCCTCTAGTCACTAAGATGCCACATATTTCTACTTGAGTGAACAGATTGCA |
| 2,70 | 5,6E-07 | BC067092 | IGKC | TCACTATCAGCAGCCTGCAGCCTGAAGATTTTGCAACTTACTATTGTCAACAGGCTAACA |
| 2,69 | 2,3E-07 | ENST00000331696 | ENST00000331696 | CAGCAGCCTGCAGCCTGAAGATTTTGCAGCTTATTACTGTCAACAGAGTGACAGTACCCC |
| 2,68 | 2,6E-05 | BC030813 | BC030813 | GTGGGTCTGGGACAGAGTTCACTCTCACCATCAGCAGCCTGCAGTCTGAAGATTTTGCAG |
| 2,66 | 3,8E-15 | NM_001783 | CD79A | TGATTGTAGCAGCCTCGTTAGTGTCACCCCCTCCTCCCTGATCTGTCAGGGCCACTTAGT |
| 2,63 | 3,3E-18 | NM_012328 | DNAJB9 | ATTTCTTTCTTAGTTGTTGGCACTCTTAGGTCTTAGTATGGATTTATGTGTTTGTGTGTG |
| 2,61 | 3,4E-15 | AF035040 | AF035040 | CACCCTTAACTGGGGATACGAAAAAGATGATGCTTTTGATATCTGGGGCCAAGGGACAAT |
| 2,59 | 1,5E-18 | NM_005080 | XBP1 | CCTTTTTGGCATCCTGGCTTGCCTCCAGTTTTAGGTCCTTTAGTTTGCTTCTGTAAGCAA |
| 2,58 | 7,6E-16 | NM_001077 | UGT2B17 | TTTCCTGCGACAAAACGTCTTTTCACAACTTACCCTGTTAAGTCAAAATTTATTTTCCAG |
| 2,58 | 4,5E-16 | NM_007029 | STMN2 | TATAATGGATCATGCGATATCAGGATGGGGAATGTATGACATGGTTTAAAAAGAACTCAT |
| 2,54 | 3,1E-14 | ENST00000327436 | ENST00000327436 | CTGGAGTGGATGGGGAGCATCTATCCTGGGAACTCTGATACCAGATACAGCCCATCCTTC |
| 2,52 | 8,9E-17 | NM_152866 | MS4A1 | GAGCTCACACACCATATATTAACATATACAACTGTGAACCAGCTAATCCCTCTGAGAAAA |
| 2,48 | 4,1E-07 | ENST00000295339 | ENST00000295339 | AGCAGCCTGCAGCCTGAAGATTTTGCAGTTTATTACTGTCAGCAGGATTATAACTTACCT |
| 2,48 | 5,7E-15 | BC031698 | FLJ40330 | AAACCTTCAAGCTGAGTGCAGAAAGCGCCGTCTTTTACTAGAAGACAGTAAAAGGTTGGT |
| 2,48 | 4,8E-16 | NM_005409 | CXCL11 | TAAGAAAGGCTGGTTACCATCGGAGTTTACAAAGTGCTTTCACGTTCTTACTTGTTGTAT |
| 2,45 | 1,6E-09 | NM_003299 | HSP90B1 | AAGAGATTTTCCTGAGAGAACTGATTTCAAATGCTTCTGATGCTTTAGATAAGATAAGGC |
| 2,44 | 1.1E-13 | M_001870 | CPA3 | GCCAAGTATATCCTCAAGCATACTTCCTAAAGAACTGCCCTCTGTTTGGAATAAGCCAAT |
| 2,40 | 1,5E-14 | ENST00000283694 | ENST00000283694 | GAGCTTGGGTGGATGGGACGGATCAACCCTAACAGTGGTGGCACAAACTATGCACAGAAG |
| 2,39 | 9,7E-18 | NM_015187 | KIAA0746 | CTGCTAAGGTAGTGAATAAATCAGTAATGCAATATTGTGGGTCCAAACTACTCTTTGCAC |
| 2,39 | 5,5E-18 | NM_021928 | SPCS3 | GAATGTCACTTTGACCCTGTCTTGGAACGTCGTACCAAATGCTGGAATTCTACCTCTTGT |
| 2,38 | 4,8E-15 | AW977527 | AW977527 | GATAAGACATCATTTCCTGCACTACTGGACACACTCAATGAGGTAACTGTTGTTGAACAA |
| 2,38 | 1,4E-06 | ENST00000310579 | ENST00000310579 | GCAGATTACACTCTCACCATCCGCAGCCTGCAGCCTGAAGATTTTGCAAATTATTACTGT |
| 2,36 | 1,8E-08 | THC2275252 | THC2275252 | GTCACCGACCCGCCCTTACTCACATGCCTTCCAGGTGCAATAAAGTGGCCCCAAGGAAAA |
| 2,36 | 1,7E-14 | NM_005065 | SEL1 L | CATTATTTCAGTGTGCATAAGTTCTTAATGTCAACCATCTTTAAGGTATTGTGCATCGAC |
| 2,35 | 6,4E-11 | BC018749 | IGLV2-14 | CATCACTGGTCTCCAGGCTGAGGACGAGGCTGATTATTACTGCAGCTCATATACAAGCAG |
| 2,34 | 3,6E-08 | AY062331 | AY062331 | AGCCTGAAGATTTTGCAGTTTATTACTGTCAGCAGCGTAGCGCCTGGCCTCTCACTTTCG |
| 2,34 | 1,6E-16 | NM_016594 | FKBP11 | TTCTCACTTGGCCTATGGAAAACGGGGATTTCCACCATCTGTCCCAGCGGATGCAGTGGT |
| 2,33 | 1,7E-12 | NM_021777 | ADAM28 | CTTAGAAGCTTCGAACTCAAAATCATGGAAAGGTTTTAAGATTTGAGGTTGGTTTTAGGG |
| 2,32 | 6,2E-18 | NM_002201 | ISG20 | CAGGGACTAGAGGCTTTCGGCTTTTTGGGACAGCAACTACCTTGCTTTTGGAAAATACAT |
| 2,32 | 1,9E-17 | DQ100868 | DQ100868 | GAATGATGATAAGCGCTACAGCCCATCTCTGAAGAGCAGGCTCACCATCACCAAGGACAC |
| 2,32 | 2,4E-10 | NM_002078 | GOLGA4 | GTGAAATGGAGCAAAAAGTAAAATCTTTAACCCAAGTCTATGAGTCCAAACTTGAAGATG |
| 2,29 | 6,1E-13 | NM_144616 | JSRP1 | GAGAGGCCTCGGAGAGAGGGGAAGCCGCGGAAGGAGAAGCCGCGGAAGGAGGAGAGACCT |
| 2,28 | 2.1E-09 | NM_001884 | HAPLN1 | GCTTCGTGGGTTTCCCAGATAAAAAGCATAAGCTGTATGGTGTCTACTGCTTCAGAGCAT |
| 2,28 | 2,6E-12 | NM_003123 | SPN | TCCTCACCCACCTCTTCACTCTGAATCCTCATGAGGCTTCTCAGCCCTGGATTTCCTGCT |
| 2,28 | 1,7E-15 | BC107852 | BC107852 | GAGGAAAACAACGCCCCAGCTGGGAAGCCTGAGAACACTTAGCCTTCATGAGTGTCCCCA |
| 2,27 | 1,4E-15 | AK074473 | C20orf82 | TATCATTCTGCTAAACGGCCCCAAAACAGTAGAATTTCTGCTCATGTCCTAGCAGGTTCA |
| 2,27 | 6,9E-18 | NM_014333 | IGSF4 | GAAAGAGTACTTCATCTAGATCAGCCTTTTTGTTTCAATGAGGTGTCCAACTGGCCCTAT |
| 2,26 | 8,9E-17 | NM_022567 | NYX | GAAGTCCTTTGTTTTCTACCACAATCCTCCTCCTCCTCTCCAGGGGCCTGGAAACACTAG |
| 2,26 | 1,1E-14 | NM_015208 | ANKRD12 | TCATCCCTGAAACATCAAATTCTGATATGCAAACCAAAAAGGAATATGTAGTTTCAGGTG |
| 2,25 | 1,4E-17 | ENST00000379969 | ENST00000379969 | TTTTCAGTTGTTACAGAGCTCAGCAGCTGTGGTTGCCCCTGTTCTACACCAATTTCAGTT |
| 2,25 | 2,6E-17 | NM_198491 | FAM92B | AGGGATCTACTGGATTTTAGAGCCAAGATGCAAGGAGTTTATGGGCATTATGACACTCGG |
| 2,24 | 2,2E-15 | THC2404289 | THC2404289 | AAGCTAATGCGAAATCATTTCTCTCTGATTTACCTGAGGAGGTTAACAAGGACCCATCAA |
| 2,24 | 2,2E-09 | NM_002345 | LUM | TTTTTATTTCTACTGTCAAATGATGTGCAAAACCTTTTACTGGTTGCATGGAAATCAGCC |
| 2,24 | 5,0E-17 | NM_030926 | ITM2C | ACTCTTAAATGCTTTGTATATTTTCTCAATTAGATCTCTTTTCAGAAGTGTCTATAGAAC |
| 2,23 | 8,8E-18 | CR620293 | CR620293 | CAACAAAGGAGCGTCACTTGGATTTTTGTTTTCATCCATGAATGTAGCTGCTTCTGTGTA |
| 2,23 | 1,3E-15 | L06610 | L06610 | TGAGAGGAAAGTTCTTCACCATGGACTGGACCTGGAGGGTCTTCTGCTTGCTGGCTGTAG |
| 2,23 | 1,5E-15 | ENST00000321715 | ENST00000321715 | TCCGGCTTCTTCGTCTTCAGCCGCCTGGAGGTGACCAGGGCCGAATGGGAGCAGAAAGAT |
| 2,22 | 5,4E-15 | NM_030776 | ZBP1 | ATGTTTGAGTCCCAACAAAATTCATATCAAAACATAATCCCAACTGGGTGCAGTGGCTCA |
| 2,21 | 3,2E-15 | NM_033285 | TP531NP1 | GGGAGGTTAGATGTGTGTTTCAGGCTTGGAGTGTATGAGTGGTTTTGCTTGTATTTTCCT |
| 2,21 | 3,9E-15 | NM_006280 | SSR4 | GCCTCCTCAGGAAGGCTCAGAGGAATAACGAGGACATTTCCATCATCCCGCCTCTGTTTA |
| 2,21 | 2,2E-14 | NM_182568 | FLJ36492 | CTCATAGGCATCAGAAAGAAAAGGATCTCTTTCATGAAAATTGCATGTTGCAGGAAGAAA |
| 2,20 | 7,2E-14 | NM_013244 | MGAT4C | TAGATGTTGGGGAAAACGTTATGCCTAGCAAACAAAGGAGACAATGTTCTAGTTACTTAA |
| 2,20 | 4,6E-17 | NM_024641 | MANEA | AGATTTGTGGTTACCTATACCACGCTAGGTGTTTTGACATGTTTAGTGTTTCTGCTTTAC |
| 2,18 | 2,8E-15 | NM_001012978 | NGFRAP1L1 | TCTTGAGGTTAATAATCATAAAATCCCTGCTTTCTAAATTCGCATTTTTCCTGGTGTACC |
| 2,18 | 6,9E-09 | ENST00000322032 | ENST00000322032 | GTAAACCCACCCACATCAATGTGTCTGTTGTCATGGCGGAGGCGGATGGCACCTGCTACT |
| 2,18 | 3,7E-15 | NM_014383 | ZBTB32 | AGGAGCGAAGGTTAACAGTAGGGGAGATTGTCGATCTCATCACCATAATAAAGAGTTTCC |
| 2,18 | 5,1E-14 | NM_013250 | ZNF215 | AAGTTGCAGTTCCTAAGAACCTATTGATGATGTTCAGTGCAGACTTACTGTACCTTTGGG |
| 2,17 | 9,4E-13 | NM_013377 | PDZRN4 | TCGGTAGAGTATGATTGCCTCGTTCAATGTGGCGTTTTTATATATATTTTGTGACTCTTT |
| 2,17 | 1,1E-15 | NM_005084 | PLA2G7 | AAAGCATTTAGGACTTCATAAAGATTTTGATCAGTGGGACTGCTTGATTGAAGGAGATGA |
| 2,17 | 6,6E-12 | THC2339566 | THC2339566 | CTGGATCATAGGTTTTGAGTTCATAATCCAGTAATAACAGCTTTCAGCTTTCTATGAGTA |
| 2,17 | 9,0E-14 | NM_032855 | HSH2D | GAATCCGAGCCCTTTTCCCATATCATCTGTTTGTTCTGTTGTCTAAAAGCACACTGCAAG |
| 2,16 | 1,3E-13 | NM_000867 | HTR2B | TCTACTAGATACGCTTCTCCTCACTGAAAATGAAGGTGACAAAACTGAAGAGCAAGTTAG |
| 2,16 | 5,3E-15 | NM_000626 | CD79B | CGCTGAAGGATGGTATCATCATGATCCAGACGCTGCTGATCATCCTCTTCATCATCGTGC |
| 2,16 | 1,1E-11 | NM_000092 | COL4A4 | TCCTAAAAGTTCAACCTGTTCATCTTGAACTTGGCCTGAGAACATTTTCTGGGAAGAGGT |
| 2,16 | 1,6E-15 | NM_024563 | C5orf23 | TGTTTGGATATAAATGTGTATGTGTCCTTGTAAATGTTTCTATCAAGCAAGAATGCCACG |
| 2,16 | 3,2E-12 | AK025047 | AK025047 | CCTCTCACACCCCTCCAACCTCATTTTGAGAGAACAATTCACATTTTAATTTTTGATGGC |
| 2,15 | 1,7E-17 | AK098753 | C22orf35 | TGGCTGAGATGATACCCGACCCTCTAGGGAAATTCTTAGAGTAACTTCTAGGAAATGTCA |
| 2,13 | 3,6E-16 | NM_022136 | SAMSN1 | CTCTGGTTGCTATATCTCATCAGGAAATTCAGATAATGGCAAAGAGGATCTGGAGTCTGA |
| 2,12 | 1,5E-14 | NM_152446 | Cl4orf145 | GGCCAAATATCCTACTGTGATCACTTCAGTCATATCCTGGACCGTCTTTGGTTTACTGTG |
| 2,12 | 1,8E-15 | THC2287925 | THC2287925 | GTCCCTTCTCCCTAACACTCCAGTTCAAGATAGCAACCTAAATTCTGAGTTGATTATTCT |
| 2,12 | 1,5E-14 | NM_003292 | TPR | ATGATGTTTCTCTTGCATCAACTCCAAAACGTCCAAGTACATCACAGACTGTTTCCACTC |
| 2,12 | 1,6E-09 | NM_004887 | CXCL14 | GTGGTACAACGCCTGGAACGAGAAGCGCAGGGTCTACGAAGAATAGGGTGAAAAACCTCA |
| 2,12 | 7,9E-13 | NM_012108 | BRDG1 | CCATACAATTTCATTATCTTATCAGAATGAAACCAATTCACAGGGAAACTAGAGACTACG |
| 2,11 | 2,0E-14 | NM_001362 | DlO3 | AAGTTGGGGTGTCTGCTTTGGGACCAGAGGAAGATAGCTTGAGAGGCATTGGCGAGGTTC |
| 2,11 | 1,7E-06 | ENST00000379913 | ENST00000379913 | CACAAGCCCAGCAACACCAAGGTGGACAAGAGAGTTGAGTCCAAATATGGTCCCCCATGC |
| 2,11 | 2,9E-16 | NM_022154 | SLC39A8 | GGAAAATGATTGACAAAGCCCAACAATGATCTCAGGAATTACATTTTCCAACAGACCAAA |
| 2,11 | 3,7E-10 | THC2380963 | THC2380963 | TATTGAGAGGGGCCTCGACCCCTCCTCATCCCCTCAGTGAAAACAATAAAGACAAATTTT |
| 2,11 | 3,1E-11 | NM_002927 | RGS13 | ATTAACAAACTTCTTTATCACATGTATCTTCTACATGTAAAACATTTCTGATGATTTTTT |
| 2,10 | 2,2E-12 | NM_001890 | CSN1S1 | CCTATGCTGTTTGGTACTATCCACAAATCATGCAGTATGTTCCTTTCCCACCGTTTTCCG |
| 2,10 | 1,9E-17 | M_018284 | GBP3 | GAGATGCCAAGGTGAAAGTACCCAACTTCAAAATGAGATACAAAAGCTACAGAAGACCCT |
| 2,10 | 1,7E-16 | NM_014398 | LAMP3 | ACCATGTTGACTTTCCTCATGTGTTTCCTTATGACTCAGTAAGTTGGCAAGGTCCTGACT |
| 2,09 | 2,8E-16 | NM_002009 | FGF7 | GGAGGACTTAGTTTTTCATATGTGTTTCCTTAGTGCCTAGCAGACTATCTGTTCATAATC |
| 2,09 | 1,4E-12 | CR594705 | CR594705 | ATCCACTCTCTAGAGTCCAGTGTACTTTAGACTTCATCTGAGTCCAATACATGTACCACA |
| 2,09 | 5,6E-14 | NM_003810 | TNFSF10 | GCAACAATCCATCTCTCAAGTAGTGTATCACAGTAGTAGCCTCCAGGTTTCCTTAAGGGA |
| 2,08 | 4,2E-10 | NM_152687 | FLJ33641 | CCCAAATGCCTAGAACATCACATAAGGCACTAAATGCCTCATGTTTTACTGACGGGAATT |
| 2,07 | 2,2E-15 | XM_926222 | LOC441368 | AGAGTTCTACATTTATTCCAGCATTACCAGGTGAAACTCTCACTTACCTATGGAAAATCC |
| 2,07 | 5,3E-13 | NM_003558 | P!P5K1B | TTCCTATGGACTTTTGCATTATTTCATTGTGCATGCATCCAGTGATTATACATAAGCAAC |
| 2,06 | 7,3E-09 | AW979273 | AW979273 | GGATATTTGGTATGCTGGCAATGGTAATTCAAATTGGCGTTTAAGAATAATCTTGGCCGG |
| 2,06 | 7,9E-11 | NM_005711 | EDIL3 | ACATGACTGCCTATCAGTAGATTGATCTGTATTTAATATTCGTTAATTAAATCTGCAGTT |
| 2,06 | 1,2E-12 | NM_000139 | MS4A2 | TCAATGTCATCTTCTCCATGAAGACCACTGAATGAACACCTTTTCATCCAGCCTTAATTT |
| 2,06 | 4,4E-14 | NM_003037 | SLAMF1 | AGGCGCAGAACAGAGCGTTACTTGATAACAGCGTTCCATCTTTGTGTTGTAGCAGATGAA |
| 2,06 | 2,1E-16 | NM_014674 | EDEM1 | TCCTTGCACACTTCAGTGTTTCTCTCCTGTTCAATAAAATGCCCTGTTAAGGTATAATTT |
| 2,06 | 2,9E-13 | NM_000587 | C7 | CAGATACAAACTATTTCTATCCTGAGTAGTAATCTCACACTTCATCCTATAGAGTCAACC |
| 2,04 | 2,4E-10 | THC2314833 | THC2314833 | AGGCATTGAAGGCCACGAGTTACATTTGTTGGCAAAGATGAGTATGCAAGTCAACAAGTC |
| 2,04 | 6,1E-07 | NM_022137 | SMOC1 | TATTCTCTCTCTTATTGTAAGTTTTTGGATCTGCTACTGACAACTTTTAGAGGGTTTTGG |
| 2,04 | 6,8E-16 | AF124170 | AF124170 | AAGCAGAACCAACATCGCAAGTAATACTGTGAACTGGTACCAGCACCTCCCAGGAATCGC |
| 2,03 | 1,2E-09 | M_018084 | KIAA1212 | TACTGGAACAGGAAAATGAACATCTGAATCAAACAGTGTCTTCCTTAAGGCAGCGGTCCC |
| 2,03 | 2,6E-14 | BC042469 | LOC644846 | AAATGTGAAGTCTGGCTTTGAAGAGGGTGTATAACACACATAATTTACTGTGCATCAGTC |
| 2,03 | 2,7E-09 | NM_006475 | POSTN | AGGAAGTTGCAAGCCAACAAAAAAGTTCAAGGATCTAGAAGACGATTAAGGGAAGGTCGT |
| 2,02 | 6,9E-14 | NM_006533 | MIA | CTGGCAAAGTCGATGTGAAGACAGACAAATGGGATTTCTACTGCCAGTGAGCTCAGCCTA |
| 2,02 | 5,1E-1 | NM_201269 | ZNF644 | TTGGAAAGACGAAATGGGATGCTCACAAATCTCCAATCTGGGTCTGAATGAGATGATGCA |
| 2,01 | 9,2E-17 | NM_001951 | E2F5 | GCACTTTAAGTTTATCACATTTTGTTGACTTCTGACATTCCACTTTCCTAGGTTATAGGA |
| 2,01 | 4,3E-11 | NM_020939 | CPNE5 | TGGTTCTGTGCCCGTCTCTGAGACAGTCTCTGTGTGGAATTTGCCTTAAACTGAAGTAAA |
| 2,01 | 1,0E-15 | NM_016570 | ERGIC2 | AGAAAGGGAACGTATCATTAACCATGCTGCAGGCAGCCATGGAGTCTCTGGGATATTTAT |
| 2,00 | 2,9E-13 | NM_181453 | GCC2 | AAGAACCTTCAAGAAAAGAATGGAGTATACTTACTTAGTCTCAGTCAAAGAGATACCATG |
| 2,00 | 3,1E-12 | NM_144590 | ANKRD22 | AATCCTTGTGACCACACCGATGGAGATACAGAAAAAGTTAACGACTGGATTCTATCTTCA |
| 2,00 | 2,7E-13 | NM_006495 | EVI2B | CACAAAACGTACATCAATCATTTCACTTACACCCTGGAAACCAAGCAAAAGCACACTTTT |
| 2,00 | 1,7E-15 | NM_005739 | RASGRP1 | ATTAACAGCAGGGTCACTTCTCATTTTCTTTGCTGACTTACCTTTTTACTGACCGTTGTG |
| 1,99 | 1,7E-15 | NM_130446 | KLHL6 | TTCTGGTCTCAATGGCTTCGGGAAACACACATATACACATACACCATGCCCTTGAACTCA |
| 1,99 | 9,4E-12 | NM_002838 | PTPRC | AGATGTCCTCCTTGTTCTACTCATATATATCTATCTTATATAGTTTACTATTTTACTTCT |
| 1,98 | 2,8E-12 | NM_017935 | BANK1 | GCACATTTTTGCTTTTTAAGTAGCTGGTTCATTTTCTGAATTTCTCACATTCAGAGTTCC |
| 1,98 | 3,1E-14 | NM_174950 | KGFLP1 | TGTATCAGACCTACAGGCTGCTGGCAGGATTTGTCAGATAATCAAGCCACACTAACTATA |
| 1,98 | 2,4E-15 | NM_000647 | CCR2 | ATGAAGTCATGCGTTTAATCACATTCGAGTGTTTCAGTGCTTCGCAGATGTCCTTGATGC |
| 1,98 | 1,8E-13 | BC042649 | BC042649 | CAGACCCCCTCCTTACAGAGCAAATGTCCAGGCTTCGGAGGTCACTGTCACATCCCAAGA |
| 1,97 | 2,2E-11 | ENST00000379900 | ENST00000379900 | GGGGCTGGAATGGGTGGCAGTTATATCACATGATGGAAGTAATAAATACTACGCAGACTC |
| 1,97 | 5,0E-17 | NM_002372 | MAN2A1 | ATGAATCATCCAGTCATTCCAATGGCAAATAAGTTCTCCTCACCTACCCTTGAGCTGCAA |
| 1,96 | 6,3E-11 | AX775927 | AX775927 | TATGTTTGTGGATGAAATATGAACCGAGTTTGTGGATGAAATATGAGCAGCATGAATATG |
| 1,96 | 4,0E-12 | NM_032932 | RAB11FIP4 | AGATGGCCTCTGACTCTGTGGGATTCAACTTGACTTTTTGCCCCAGGAGGCTCTGTCTG |
| 1,96 | 1,1E-07 | NM_00541 | SEPP1 | CGTAAACTATGACCTAGGGGTTTTCTGTTGGATAATTAGCAGTTTAGAATGGAGGAAGAA |
| 1,96 | 2,8E-13 | NM_005292 | GPR18 | TCAAAACAATTTCAGGCTCGAGTCATTAGTGTCATGCTATACCGTAATTACCTTCGAAGC |
| 1,96 | 3,4E-06 | BX161420 | IGHM | TGACCACCTATGACAGCGTGACCATCTCCTGGACCCGCCAGAATGGCGAAGCTGTGAAAA |
| 1,96 | 4,0E-09 | NM_006948 | STCH | TGAGTAACTTATTTTGTATCAGGAATGTTTTGGTACTGTGTTTTCACTCAAACCACTGAC |
| 1,96 | 1,4E-09 | AL831839 | FLJ20054 | ATAGAATCCCAACTTTTAAGAATGCTTTCCTTATATGCATGTACTTCCTACCGTTAATGC |
| 1,95 | 3,1E-11 | NM_152513 | RP5-821D11.2 | AGAATGAGACCTGGAGACAAAGGGCATAATTGTTGGGGAAATGGATGACAGCTGAAGCTA |
| 1,95 | 8,3E-12 | NM_006260 | DNAJC3 | AAGGACGAGAAGCCTGTTGAAGCTATTAGGGTTTGTTCTGAAGTTTTACAGATGGAACCT |
| 1,95 | 1,2E-14 | NM_002661 | PLCG2 | GAGTCAGCAACAGCAAGTTTTACTCATAGAAGCTGGGGTATGTGTGTAAGGGTATTGTGT |
| 1,95 | 2,4E-10 | NM_004000 | CH13L2 | ATGATGTGAAGAGTATGGAGACCAAGGTTCAGTTCTTAAAGAATTTAAACCTGGGAGGAG |
| 1,95 | 2,1E-08 | NM_003608 | GPR65 | AACAAGTTTAAATTGTGTTGCTGATCCAATTCTGTACTGTTTTGTAACCGAAACAGGAAG |
| 1,94 | 3,2E-14 | NM_002164 | INDO | ATCTGCAAATCGTGACTAAGTACATCCTGATTCCTGCAAGCCAGCAGCCAAAGGAGAATA |
| 1,94 | 5,5E-11 | NM_138738 | FCRL2 | GAGGAATCAGAAGGGAAGATCAACAGCAAGGATGGGGCATCATTAAGACTTGCTATAAAA |
| 1,94 | 3,0E-14 | M_018440 | PAG1 | TCTCTCACATGATGGGGTTCTTTAGTACATGGTAACAGCCATGTCATCTTACACACCTAG |
| 1,93 | 1,8E-16 | M_018169 | Cl2orf35 | CCCATCTTGAGGGAGGACCGTTCCTCAGTTAAGGACTTGTTTATTTAAATGGGACTGTAA |
| 1,93 | 5,9E-13 | A_23_P136961 | A_23_P136961 | ATATATATGTATGTATATATGTATATATTATGATGTTGGCTGACCCCCTTCCTCCCACTC |
| 1,93 | 6,4E-14 | ENST00000379872 | ENST00000379872 | TCACACTGACCTGCACCTTCTCTGGGTTCTCACTCAGCACTAGTGGAATGTGTGTGAGCT |
| 1,93 | 2,2E-16 | NM_022085 | TXNDC5 | ATACGCAAGGGGATGTGGATACTTGGCCCAAAGTAACTGGTGGTAGGAATCTTAGAAACA |
| 1,93 | 4,2E-14 | NM_006713 | SUB1 | AGGAAGAAAAGGTATTTCTTTAAATCCAGAACAATGGAGCCAGCTGACAGAACAGATTTC |
| 1,92 | 5,6E-10 | NM_003430 | ZNF91 | TGGAGAATGTGGCAAAGCCTTTAAAGAGTCCTCAGCTCTTACTAAACATAAGATAATTCA |
| 1,92 | 6,7E-13 | NM_138426 | GLCC11 | CATTGCGCTGAACAGTATTTGAGTTACCATATAATATGGCTTTACACAAGGAAATGTGTG |
| 1,91 | 2,4E-13 | NM_001759 | CCND2 | CAGCAGCGGGAGAAGCTGTCTCTGATCCGCAAGCATGCTCAGACCTTCATTGCTCTGTGT |
| 1,91 | 6,9E-12 | A1915259 | A1915259 | TAGGTGATTATATCTTTGGTACCGTATTGAGAACCCACTCTCCCTCCTTGGACCAACTCT |
| 1,90 | 3,1 E-11 | NM_001704 | BA13 | ACAGTGTGACTATCTTATGTCAGGACCTTCATGTGCCAAACGTCAGTGGTGTTTTCATAT |
| 1,90 | 4,7E-11 | THC2376729 | THC2376729 | CCCTCGGCAGGTACTTTAGAGTCACGTTCATGCTGTAATATGTGGGGAAGAAAGACTATT |
| 1,90 | 1,6E-10 | NM_130396 | WISP3 | TGCTCCAGAACATGTGGGATGGGAATATCTAACAGGGTGACCAATGAAAACAGCAACTGT |
| 1,90 | 5,5E-17 | NM_001146 | ANGPT1 | GTTTCAAACTTGGTGACTTGATCCACTATGCCTTAATGGTTTCCTCCATTTGAGAAAATA |
| 1,90 | 1,4E-10 | NM_002427 | MMP13 | TTTTTCAACGGACCCATACAGTTTGAATACAGCATCTGGAGTAACCGTATTGTTCGCGT |
| 1,89 | 7,6E-14 | NM_001076 | UGT2B15 | ATCAAAAGCCTGAAGTGGAATGACTGAAAGATGGGACTCCTCCTTTATTTCAGCATGGAG |
| 1,89 | 2,6E-14 | NM_005013 | NUCB2 | GATGAGCTTCAGAAACAAAAAGAAGAGCTACAACGTCAGCATGATCAACTGGAGGCTCAG |
| 1,89 | 4,7E-13 | L10374 | L10374 | CTGTCTTTATGTCCTGTCAGCTTCTATTAGAGGAATGATTGCTATGACCTCATGGTATAG |
| 1,89 | 1,0E-14 | AF086301 | MGC24039 | TGTTCACCCTCTCAAGGACAAGATAAAGATTGACTTCAGAGATATATCACATCAAGCTAG |
| 1,89 | 6,3E-14 | NM_024730 | FLJ22655 | GAAATGATTGAGTATGAACCACAGCTGTGTTTTCAAATATGTAGTTTGCCTTTTTGGTTG |
| 1,89 | 8,2E-09 | NM_001460 | FM02 | CCCTGAAGGATTCATCTAATTTCTCAGTTTCTTTTCTGTTGAAAATCCTGGGCCTTCTTG |
| 1,89 | 1,4E-12 | NM_002825 | PTN | AAAGGACATCAGCAAACAGGATCAGTTAACTATTGCATTTATATGTACCGTAGGCTTTGT |
| 1,88 | 5,1E-10 | NM_005300 | GPR34 | AGTAGGAGTGAAAGCACTTCAGAATTTAAACCAGGATACTCCCTGCATGATACATCTGTG |
| 1,88 | 3,5E-12 | NM_018460 | ARHGAP15 | AATGCATTGAAGCTGTTGAGAAAAGAGGTCTAGATGTTGATGGAATATATCGAGTTAGTG |
| 1,88 | 2,7E-09 | NM_000618 | IGF1 | AATGTAACTAATTGAATCATTATCTTACATTTACTGTTTAATAAGCATATTTTGAAAATG |
| 1,88 | 4,9E-12 | NM_002184 | IL6ST | ACAAAAATGAACTGAAGTTTCACATGAGCTATTTCCATTCCAGAATATCTGGGATTCTAC |
| 1,88 | 7,9E-12 | THC2377971 | THC2377971 | AAGTCACGATGTCCATCATGCTGTAGCTCAATGAATGAGTCTCACATTGTTTCATGTTGT |
| 1,87 | 2,8E-11 | NM_173216 | ST6GAL1 | ATGCAAATTATGATATGGACGTTATCATTGGTCTGGTGAGATGTTTCATATTTGTGACAG |
| 1,87 | 3,3E-14 | NM_004748 | CCPG1 | TACTTTTTGTCGCTGGAACGAACTTGATCAGTTCATCAATAAGTTTTTCCTAAACGGTGT |
| 1,87 | 1,5E-10 | NM_017948 | NOL8 | TAAGGACATCATACATTATGATCCAACGAAGCAAGACCATGCCACTTACGAAAGAAAAAG |
| 1,87 | 2,8E-14 | NM_014685 | HERPUD1 | TGCAGTGCTTTACTTTAAACTAAGGGGAACTTTGCGGAGGTGAAAACCTTTGCTGGGTTT |
| 1,87 | 6,8E-13 | NM_001034852 | SMOC1 | CTGCCCCAACGTGGAAAGGTGGGAAGGAAGCCTTCTCCCATTAGCCCCAATGAGAGAACT |
| 1,87 | 2,6E-11 | AK094167 | AK094167 | CGAGACCCACCTTCCTCTTCCTTTAGCAGCTGGGAAATTGGGGGCGTTTATGGCGCCCCG |
| 1,86 | 2,8E-09 | NM_024781 | CCDC102B | CATTGCAAAGTAGCATCTAGGTAGAAATCAGGCCAAAATTAAGCTGTGGTTTCCCTCTGA |
| 1,86 | 1,8E-10 | NM_148894 | FAM44A | TTTCTTCTTCAAAGGAGCTGAAGCATGTTCATGCAAAAAGTGAACCAAGTAAACCTGCCC |
| 1,86 | 1,1E-10 | THC2341371 | THC2341371 | TCTTCTACCTAAGACCTGAAATGTTGGCGAAGTAAATATGTTGTTCCAATTTTGGTCAAA |
| 1,86 | 3,2E-16 | THC2448843 | THC2448843 | GGGCAGGTAGGATTTCAATGGCATGTTACATAACCATCCTCTAAAGGGACAGAATGTACA |
| 1,86 | 8,7E-11 | NM_181780 | BTLA | CCTGACCTTTGTTTCAGGATGCAGGAAGGGTCTGAAGTTTATTCTAATCCATGCCTGGAA |
| 1,86 | 2,5E-14 | NM_015230 | CENTD1 | ACAGGCCCATACTTCAGTCAGTCCAATCATAGTACAGTGATGTGGTGGTAGATGCTATGA |
| 1,86 | 2,3E-13 | AW389914 | AW389914 | TGATCTCCAAGGTTCTGACCTCTGAGAGTGACGTTAAGTCCAGAGAGCCACACCACTACA |
| 1,86 | 2,9E-12 | NM_000959 | PTGFR | CCCATTCAAATTGTCCTAGGTCTATCAGAAATTAGGGAAGGTAGTCCTGCTTTATAATAG |
| 1,85 | 4,8E-13 | NM_006763 | BTG2 | TTTTAGAAGCAGCCTCATGGTCTCATGCTTAATCTTGTCTCTCTTCTCTTCTTTATGATG |
| 1,85 | 3,8E-12 | NM_032483 | PPAPDC1B | AAATTCTAATTTAGTACACTCTGAACAAAGCTTATTATACTTACTTAAGATGTGTTTTGA |
| 1,85 | 9,4E-08 | THC2331323 | THC2331323 | AGATTTTTCATTAGACCTCTTAGGTACAGGAGCCGATGCAGCAATTCCACTAACATGGAA |
| 1,85 | 1,3E-07 | NM_203282 | ZNF539 | AAACCCTACAAATGTGAAGAATGTGGCAAAGCTTTTAGCCAGTCCTCAACCCTTACTACA |
| 1,84 | 2,0E-14 | NM_002351 | SH2D1A | AAGGCTAAGCTTCACTGTAAAATAATAACTGGGAATTCTGGATTGTGTATGGGTGTTGGT |
| 1,84 | 7,1 E-09 | NM_032870 | C6orf111 | GAATTACGGCATCGAATCCGGCAAAAACAGGAAGCTTTTTGGAGAAAAGAAAAAGAACAG |
| 1,84 | 2,3E-11 | NM_005014 | OMD | TTATGGTGAACAACGAAGCACTAATGGTCAAACAATACAACTAAAGACACAAGTTTTCAG |
| 1,84 | 5,8E-12 | NM_014739 | BCLAF1 | CAAAAGAGGAAGAGGTCGTGGTACTTTTCAACGTGGCAGAGGGCGCTTTAACTTCAAAAA |
| 1,84 | 6,1E-12 | BU685299 | BU685299 | GGATAAACCCCTCATGATCATAATGAATAATGATGTGTGGAGAGTGGGGAGGGTTTACAT |
| 1,84 | 6,5E-07 | AK024584 | AK024584 | GTCATCCTCTTAATGAAGCATTATGCAGCTGTTAAAGATGATAGATGTAAAGGTTATGAG |
| 1,84 | 7,3E-13 | S82024 | STMN2 | GTAAATCCCCCTGCCTATATTTATAATGGATCATGCGATATCATGCGATATCAGGATGGG |
| 1,83 | 3,1E-08 | NM_003412 | ZIC1 | AAGTGCAAACATTTCGTCCCAAAGTCTAAGTACTTTAGTGCAGTAAAATGTTGTTTCATG |
| 1,83 | 1,7E-13 | NM_003571 | BFSP2 | CTGAAAAGCTTCCTGGAAGTGGAGAGATCCTTCTGCTTTAATCTGAGTAGTCTGTAGCTT |
| 1,83 | 6,7E-11 | AK123333 | AK123333 | AGAGGAATCCACCAACAGGCACCAGCACTCTGGCAGGCCACCGACCAATGGATTGACATA |
| 1,83 | 1,7E-11 | NM_006639 | CYSLTR1 | GGCCAAACCACAAAAAGATGAGAAAAATAATACCAAGTGCTTTGAGCCCCCACAAGACAA |
| 1,82 | 2,7E-12 | BC020847 | LOC644246 | TGATAGCATTAAGCCACTGTATCAACCAATCCTGACAATTTGTATGACTTCTGGATTTCC |
| 1,82 | 9,8E-14 | NM_018963 | BRWD1 | TTCAAAGTTTAGACCTGATACTAGTTCCAAATCATCAGATTTGGGATCTGTAACTGAATC |
| 1,82 | 1,4E-14 | ENST00000265341 | ENST00000265341 | GATTTATTTCTTCTAATCAAAGATGCATAACAGCTATTATCTAGGGGACCACCAAATGTG |
| 1,82 | 6,4E-09 | NM_018037 | RALGPS2 | ATGATATTCCCATGTTGCCTCATGTCCAAAAATATCTCAACTCTGTTCAGTATATAGAAG |
| 1,82 | 1,2E-15 | NM_153837 | GPR114 | GTGGACAAACCACTCAGCCTCTGTGTGCCTCAGTTTTCCTATTTGTAAAATAGAGGCCAT |
| 1,82 | 1,3E-10 | NM_006933 | SLC5A3 | AAAGAGAGAAAGAAAGAAACGGATGATGGAGGTCGGTACTGGAAGTTCATAGACTGGTTT |
| 1,82 | 3,2E-07 | BE082118 | BE082118 | ACACTGGAGAGAAACCCTACAAATGTGAAGAATGTGGCAAAAGCTTTACCTGCTCCTCAA |
| 1,82 | 5,5E-15 | NM_138554 | TLR4 | TTACTGAGTGTTTCAGAGTGTGTTTGGTTTGAGCAGGTCTAGGGTGATTGAACATCCCTG |
| 1,82 | 1,5E-13 | NM_001198 | PRDM1 | ATGACATCAGTGTGATCTCTGTAGTGGAGAAGGAAATTCTGGCCGTGGTCAGAAAAGAGA |
| 1,82 | 1,9E-13 | NM_016010 | C8orf70 | GCATTGAAGGACATTCACCTGGAAACTTACCAAAATTCTGCCATGAGTGTGGGACTAAAT |
| 1,82 | 2,0E-12 | NM_004675 | DIRAS3 | GTAACATCCATGTTGTCAATACGTATACCTTGTAAGTGGATAACTTTTCTTTTTCCCAGG |
| 1,82 | 1,9E-10 | NM_022091 | ASCC3 | GCTGGATGGAAAAGAGGAGGATGAGAAGATGAGCAGAGCTTCTGACAGATTCAGAGGACT |
| 1,82 | 1,4E-07 | NM_175910 | ZNF493 | ATGATTCACACTGGAGAAAAACCCTACAAATGTGAAGAATGTGGCAAAGCTTTTAATCGG |
| 1,82 | 8,0E-10 | NM_153607 | LOC153222 | AGTTCTCTAAAGGTTTTCTGTGTTCATACATGGTATACAGATAGCTCATAATGAAGTCCA |
| 1,81 | 1,0E-12 | CR593388 | CR593388 | ATGGAATTAAGGCCATTGCAATGTATCATCTTTGTAGCATTGTCATCACTCCTAAGCTGC |
| 1,81 | 7,2E-14 | NM_033181 | CNR1 | TGTACTAGGCCTACTGGGGATCAGAGTTCCCAAGAAAGGAAACCTTTTCTTGTATCTGGA |
| 1,81 | 2,7E-12 | BC025721 | ELTD1 | ATAGGACCAGCATGCCTAATCATTCTTGTTAATCTCTTGGCTTTTGGAGTCATCATATAC |
| 1,81 | 6,8E-15 | M_001003927 | EVI2A | GCTGAATCAGACACTTGGAAAAGAACAAAACAGCTCACAGGACCCAACCTAGTGATGCAA |
| 1,81 | 1,4E-09 | NM_000829 | GRIA4 | CATAGTCTTTGCCTACATTGGTGTCAGCGTGGTCTTATTCCTAGTTAGTAGATTTAGTCC |
| 1,80 | 8,7E-08 | NM_178558 | ZNF680 | GAGAATTCATGCTAGAGAGAAACCCTACAAATGTGAAGAATGTGGCAAAGCTTTTAACCG |
| 1,80 | 3,8E-14 | NM_004172 | SLC1A3 | GTGGCTGTCCTCTTAAGACACTAGTAGAGCAAAGACTTAATCATATCAACTTAATTCTGT |
| 1,80 | 1,5E-13 | AK093811 | FLJ36492 | CAGCAGATTCCTAGGAAGGAAAATGAAGAGCATGACAGGTAAGCCTATAGCAGCGTTTAA |
| 1,80 | 1,2E-14 | NM_013269 | CLEC2D | AGCTACCTTAATTTGGCGCTTATTTTTCTTAATCATGTTTCTGACAATCATAGTGTGTGG |
| 1,80 | 2,4E-16 | NM_052864 | | TIFA TACAGAGTTATAGTGTGTTTACTCCTAAGATGACAGTTCTCTTTGTCTATATTCAGCATC |
| 1,79 | 1,6E-08 | NM_006633 | IQGAP2 | GATTGCCAAGGATATTCGAAATCAAAGAATCTATCGTAAGCTTCGAAAAGCTGAATTGGC |
| 1,79 | 3,8E-12 | AK097118 | AK097118 | TAAACGGCTTTATCAGCTAAAGTCCTTGTTCATTCACTGAGTCTTTTGTATCTTTTAGGA |
| 1,79 | 1,4E-13 | NM_018177 | N4BP2 | AAGTCTTTTGTGAGAGGTATTTAAAGTGCTTTGAGACCTGATTCATGCCCCCCAAAGGGT |
| 1,79 | 1,8E-09 | NM_001005473 | PLCXD3 | GGATCTGGTTATTGATACTTTAATAAATGTGGTGTAAAGAAAATCCATGGCTACAGTCTG |
| 1,79 | 2,4E-07 | NM_019604 | CRTAM | AAGCAGAATAGATGTTTGTTTTTCTAGTGGTTATACCAAGCTATACTTCCTGTTTTCACG |
| 1,79 | 5,8E-10 | NM_004310 | RHOH | AAAGCTTGGTGTTTTCTCTGGGTACACCCCAAGCAGCGTCTCCTTTTGGATACAGTTATT |
| 1,79 | 5,5E-12 | NM_032441 | ZMAT1 | CCAGTACCCTTGAGCCTTAATCAGCAAGAAAATAACTCTGGCTCATACAGTGTAGAATCT |
| 1,78 | 2,4E-14 | NM_002120 | HLA-DOB | CCAAATCATTTACTTTTCTGTGGTCCAGCCCTACTCCTATAAGTCATGATCTCCAAAGCT |
| 1,78 | 3,2E-14 | NM_178154 | FUT8 | CGGAACAGCTCCTTACTCTGAGGAAGTTGATTCTTATTTGATGGTGGTATTGTGACCACT |
| 1,78 | 3,5E-12 | NM_000186 | CFH | AGGTCTTCACAAGAAAGTTATGCACATGGGACTAAATTGAGTTATACTTGTGAGGGTGGT |
| 1,78 | 4,2E-12 | BX538342 | GVIN1 | TAATACTCAGTGACCACAAAAGAGCCCCAGTGTCTGAAGAAAAGAGGCAGTGTACCTGAA |
| 1,77 | 6,5E-11 | ENST00000285383 | ENST00000285383 | CGGTGCCCCTCATGGCTGGAGCAGGGGTGCACCTGCTCCTCACCTTCATCCTCTTTTTCT |
| 1,77 | 1,5E-08 | ENST00000371207 | ENST00000371207 | AAGCCACATCTTATTTTTCCAAGGTTTAATTTAGTGAGAGGGCAGCATTAGTGTGGAGTG |
| 1,77 | 1,9E-06 | NM_021269 | ZNF708 | AGATAATTCATACTGGAGAGAAACTCTACAAATGTGAAGAATGTGGCAAAGCTTTTAACC |
| 1,77 | 2,3E-15 | NM_024508 | ZBED2 | AGACCACCAGTATGAATAAAAGCTTGTTCTGTGTGACCCAGCAAGTGGAAGGACAAAGAA |
| 1,77 | 1,1 E-14 | AF035790 | AF035790 | ACCCTCTCCAGAGACAACTCCGAGAACACGCTGTTCCTTCAAATGGACAACCTGAGAGCT |
| 1,77 | 2,4E-16 | NM_001024457 | RGPD2 | TGAAGAAAAGGGAAAACTTGCTGCGGTTGCTCAAGGTCTTCAAGAAACCTCCATACCCAA |
| 1,77 | 7,9E-08 | NM_003064 | SLPI | AGGTCCTTTCCACCCTGAGACTTGGCTCCACCACTGATATCCTCCTTTGGGGAAAGGCTT |
| 1,77 | 2,8E-06 | NM_052941 | GBP4 | GAGGAAAGGGAAAACCTTCTCAGAGAGCATGAAAGGCTGCTAAAACACAAGCTGAAGGTA |
| 1,77 | 2,2E-13 | NM_018353 | C14orf106 | ACAGAATTAAACATGTGCCACAGTAATTGCCAAAATAAACCAACATTAAGGTTCCCAGAT |
| 1,77 | 8,1E-13 | AK094201 | ZBTB38 | ACCTTGAATGAGACCCTCAAAATCCATGAAAGAATCCATACTGGAGAAAAGCGTTACCAC |
| 1,77 | 1,3E-08 | AK000675 | MARCH1 | GATTATTGTGTTCTTTGCATATTCATGTAAAACTGATGTGTGAATGACATTGCAGTGAGC |
| 1,77 | 1,3E-14 | NM_032966 | BLR1 | TTTTCTTTTTAATAAAAAGGCACCTATAAAACAGGTCAATACAGTACAGGCAGCACAGAG |
| 1,77 | 3,0E-15 | NM_012234 | RYBP | AGGTTATCAACCACACATCCAGTCCTGACATGGAGCTTTTCAGTGTTTGGAGACATTTCT |
| 1,76 | 7,3E-09 | NM_020659 | TTYH1 | GGCTCTGACCCCCTGATCTCAACTCGTGGCACTAACTTGGAAAAGGGTTGATTTAAAATA |
| 1,76 | 9,1E-15 | NM_020739 | CCPG1 | TATGGTCGCACTAATGGAAGACAAATGGCAAATCTTGAAATAGAATTGGGGCAATTACCT |
| 1,76 | 2,8E-11 | NM_001463 | FRZB | AACATTTTGCCTGATTGAGAAGCACAACTGAAACCAGTAGCCGCTGGGGTGTTAATGGTA |
| 1,76 | 2,0E-09 | NM_025159 | CXorf21 | AGCAACACTTTATCCATTTATTCTGAGAATGTGCAGGAGGGGTTAGTGAAGGGGAATTAA |
| 1,76 | 2,2E-09 | NM_004951 | EB12 | CTGAAACGGCAAGTCAGTGTATCGATTTCTAGTGCTGTGAAGTCAGCCCCTGAAGAAAAT |
| 1,76 | 1,8E-13 | NM_198930 | PYHIN1 | TGCTTTCGACTGAGAAAGAGGGAAAATATGTCAAAACTGATGTCAGAAATGCATAGTTTC |
| 1,76 | 1,8E-11 | M_018387 | STRBP | CCAACATATCCTGTTCCCCACTACTCATTCTTTTAGCAAATGACAGAAGCTAATTCCTAT |
| 1,76 | 2,3E-11 | AL036373 | AL036373 | CAGTCCTTTATATCCAAAAATCTACTCATGTATTTTATCTCTAGTTTGTTTTTTCCTGGT |
| 1,76 | 6,8E-11 | NM_005192 | CDKN3 | CTTACAACCTGCCTTAAAAATTACCGAAAAACCTTAATACACTGCTATGGAGGACTTGGG |
| 1,76 | 1,1E-12 | NM_024660 | TMEM149 | ATGCATTGCGGGTGCTGTCCAAGCTTGGCTCATCTGGGGTTTGCTGGGCTTAACACCCAA |
| 1,75 | 1,3E-12 | NM_014923 | FNDC3A | ATACTTGCCATTTGAGCCTCACTGCAAAATTAGTGCAGAGGAGAAAACAATTTTTAATGT |
| 1,75 | 2,6E-09 | NM_006603 | STAG2 | AGGGAGGATTGAGGATCTTAATGAGGGAATGGATTTTGACACCATGGATATAGATTTGCC |
| 1,75 | 4,9E-16 | NM_025195 | TRIB1 | ACTAACGCTCTCGTGGTTGCTCGACTGTTGTATCTGTGATACATTATCCGACTAAGGACT |
| 1,75 | 4,2E-07 | THC2447689 | THC2447689 | TTTATGCGTTTTCACTACAATAACAAGTAGAACAGTAGAACAGATGATTAGTCACAGCAG |
| 1,75 | 1,3E-13 | M_017577 | GRAMD1C | GAATTCCAACGAAGCATACCTAGGGGTAACAGTGAACCTACCTGGGTTTGTTTTGTTTTG |
| 1,75 | 8,8E-12 | NM_013308 | GPR171 | GAACCTGTGCTTTGATCCTATCCTGTACTATCACCTCTCAAAAGCATTCCGCTCAAAGGT |
| 1,75 | 7,3E-14 | THC2375651 | THC2375651 | GGCATGCTGGGATTAAATCCTGGCTCAATTTCTCTGTAGAAGAGAAAAGAGAGGGCACAA |
| 1,75 | 7,4E-15 | NM_003136 | SRP54 | ACACAATATACCAAGTTTGCACAGATGGTAAAAAAGATGGGAGGTATCAAAGGACTTTTC |
| 1,75 | 2,0E-12 | NM_003151 | STAT4 | CCATGTCTCCAAGTGTGTATGCGGTGTTGAGAGAAAACCTGAGTCCCACAACAATTGAAA |
| 1,74 | 1,8E-13 | NM_004226 | STK17B | TACCCAATCCCCATGAACTTGTTTCAGATTTGCTCTGTTAGCACTTTTTTCTTTGACTCA |
| 1,74 | 8,9E-14 | AB002384 | C6orf32 | GGTACATTGTACAGTCTGTAGTTAGGAGGTATAGCCTATAGCTTATGTTAAATGGTTGAA |
| 1,74 | 5,0E-11 | NM_032888 | COL27A1 | ATCATCAGTGTGGACAACCTCCCTCCTGCCTCATCAGGGAAGCAGTACCGCCTGGAAGTT |
| 1,74 | 1,3E-06 | NM_012252 | TFEC | AGTTGCTTATGGCATACAAGGCTAAAATTAATTCAGCTATTTAATCTTAATAATTATTAT |
| 1,74 | 2,1E-09 | NM_178565 | RSP02 | ACACAATACCGTGTCCAACCATTGCTGAATCCAGGAGATGCAAGATGACAATGAGGCATT |
| 1,74 | 1,1 E-09 | NM_005630 | SLCO2A1 | TGTTACAGACAAGCTTATTTCCAAAGCCACCTCATTTCCAAACATCTCACTCAGGAAGGG |
| 1,74 | 1,2E-12 | ENST00000376752 | ENST00000376752 | TACAAAGTTTGGAGACTCAACAAATGCTCTAACCTTCTAGAGACAAAAGTCCTATTTCTG |
| 1,74 | 1,1E-11 | NM_004131 | GZMB | AACCATGAAACGCTACTAACTACAGGAAGCAAACTAAGCCCCCGCTGTAATGAAACACCT |
| 1,74 | 5,0E-12 | NM_024943 | TMEM156 | TGAAGAATACTCAGAAGTTTATTTTGTGAATGAGTAGACTGGAAAATGTTTGTGTCCAGC |
| 1,74 | 5,8E-11 | NM_014265 | ADAM28 | TCTACACCTAAGGACTCAAATCCAGAAGCATGAAGCAACAGCTAAGCAAGAACTAATGGT |
| 1,73 | 2,6E-14 | NM_004419 | DUSP5 | TGGGAAAAGAGTTCTTCAGATCATAGACCAAAAAAGTCATACCTTCGAGGTGGTAGCAGT |
| 1,73 | 1,2E-07 | NM_052942 | GBP5 | AGTGAGCTCCAGCACGCCCAGAGGACTGTTAATAACGATGATCCATGTGTTTTACTCTAA |
| 1,73 | 2,4E-12 | NM_005241 | EVI1 | ATGAGGAAAATGGAAGATAATCAATATTCTGAAGCTGAGCTGTCTTCTTTTAGTACTTCC |
| 1,73 | 2,6E-11 | AF035020 | AF035020 | GTTGGACCACAAACTATGCACAGAAGTTTCAGGGGAAGGTCACCATGACCAAGGACACGT |
| 1,73 | 3,9E-09 | NM_005442 | EOMES | ATTTGTAAATTCTTAAGCAAATAGAAGCCGAGTGTTAAGGTGTTTTGCTTCTGAAAGAGG |
| 1,73 | 1,5E-11 | NM_006760 | UPK2 | ATTATTCCTCTCACCCCACTCCTGTCAGAGTTGACTTTCCTCCCATTTTACCACTTTAAA |
| 1,73 | 4,3E-10 | NM_024306 | FA2H | TCGCCAAGTGTAAAGGCTAAAGAGAAGCAGTTTGACGGACCTTGTGATTTGTACTGTTTG |
| 1,73 | 3,5E-12 | NM_178812 | MTDH | CTTAATTTAAGAATATTATCCTGGTTTAACAACAGTGCCCTGTTTACAACAGATTGTGCC |
| 1,73 | 1,2E-06 | NM_138330 | ZNF675 | AGAAACCCTACAAATGTGAAGAATGCGGCAAAGCCTCTAACCAGTTCTCAAATCTTACTA |
| 1,73 | 2,7E-14 | THC2437906 | THC2437906 | CCTCTGCATTGTTTAAAGGACAACTACAAATCATGCTGGATAAGAGTACTCATTCAATCT |
| 1,73 | 3,7E-11 | A_32_P109645 | A_32_P109645 | AGACAAGATGCAAGAACACAGCCTGCAGTGATTCACAGCTTTGTTTCAACCCACAAACCA |
| 1,73 | 1,7E-11 | NM_002655 | PLAG1 | TCCATTAGGAAACGGATTGCATCATACCTGAACATAAGCTGGACTGCTGAAATTGTATTT |
| 1,73 | 5,5E-11 | THC2407956 | THC2407956 | CCCATGTAAACTAAAGCTTCTTGGTGTTCTCAACAATTTTATGAATAAAAAGGGATCCTG |
| 1,72 | 1,2E-14 | M_016021 | UBE2J1 | CAATTCATCTGGAAAGACTATCTCTGAGTCAGACTTAAACCACTCTTTTTCACTAACTGA |
| 1,72 | 2,0E-11 | ENST00000369577 | ENST00000369577 | AAACCTACCGTCAGTCTGAAAAAACTTGAAGTACATTCAAATGATCCAGATATGTCTGTT |
| 1,72 | 3,8E-12 | NM_173799 | VSIG9 | AGTTACAGAAGCCTGGGTAACTGCAGCTTCTTCACAGAGACTGGTTAGCAACCAGAGGCA |
| 1,72 | 7,7E-16 | NM_031915 | SETDB2 | GAAGCTGGGACTGTGCCTGAGAAGGAAATCTTCTGCCAATGTGGGGTTAATAAATGTAGA |
| 1,72 | 3,6E-13 | NM_153218 | FLJ38725 | TGGAAAAACAGGTTGGAAACAGATAAGTAAGGGTCTCAAATGCAATGTCAAAGAGCTTGC |
| 1,72 | 9,9E-11 | NM_152322 | BTBD11 | TGGGGACAGCCAAGTTTTGTTGATAAACCTATTTCCTAGCATGCCTTCAGGAAGTTGTGC |
| 1,72 | 2,9E-08 | NM_001995 | ACSL1 | ACTCGGTTCTCCAGGCCTGATTCCCCGACTCCATCCTTTTTCAGGGTTATTTAAAAATCT |
| 1,72 | 2,7E-13 | NM_002249 | KCNN3 | AGTGACCAAGCCAACACTCTGGTGGACCTTTCCAAGATGCAGAATGTCATGTATGACTTA |
| 1,72 | 4,2E-07 | ENST00000331425 | ENST00000331425 | AGAAACCCTACACATGTGAAGAATGTGGCAAAGCCTTTAGCCGCTCTTCAACACTTACTA |
| 1,72 | 2,9E-10 | NM_002731 | PRKACB | AAACCTGTCTGAGATGTTCTTTCTACCAATCTATATGTCTTTCGGTTATCAAGTGTTTCT |
| 1,71 | 3,0E-09 | NM_007214 | SEC63 | GTTAAGTTGACAAGGCAAACAATGGCTGAAGTATTTGAAAAGGAGCAGTCCATCTGTGCT |
| 1,71 | 5,3E-10 | NM_001007544 | Clorf186 | TGAGAACTATGGAGTCCATCAGCAGAGATAGTAGTGAAGTCTCTCCCCAGGGAAAATTTT |
| 1,71 | 1,6E-13 | NM_178129 | P2RY8 | AACACAGGTCTATTGACTCACACACATGTTTTAAGATGGAAAACTTTACTTCTGTTCTTG |
| 1,71 | 1,2E-11 | NM_032738 | FCRLM1 | GACATACCAGTCTTTAGCTGGTGCTATGGTCTGTTCTTTAGTTCTAGTTTGTATCCCCTC |
| 1,71 | 1,4E-11 | BC007354 | MGC16075 | TTTGAGCAATTGTCATTTGTTGGACTGATCGTTCAATTTCCTCTTCGGTCCTTGGCCTTT |
| 1,71 | 1,1 E-12 | AL832717 | AL832717 | CAAATGCGTTCCTATGTGTCAAAAGGCCTAAATAGCCCTGTGTACCTAAAAGATGGTAAT |
| 1,71 | 2,1E-07 | NM_000639 | FASLG | AAGCAAGAGAGATGTTTTGGGGACTCATTTCATTCCTAACACAGCATGTGTATTTCCAGT |
| 1,71 | 3,6E-07 | NM_025189 | ZNF430 | AAACCCTACAGATGTGAAGAATGTGGCAGAGCTTTTAACCGGTCCTCACACCTTACTACA |
| 1,71 | 2,0E-09 | NM_005143 | HP | GATAAGATGTGGTTTGAAGCTGATGGGTGCCAGCCCTGCATTGCTGAGTCAATCAATAAA |
| 1,71 | 6,1E-11 | THC2397697 | THC2397697 | TGGTAAGTTTCTCTCTCTTTAGAGACTCCACAATAAAGTTTTCAACATGGTAAGGTTTTC |
| 1,71 | 1,2E-12 | AK090431 | NOD3 | TTTCCTTCCAGACACGTGACACTGATATTAAAGTGCTAATGAGAGGGATCTATTTCTTCT |
| 1,71 | 2,1 E-08 | AK096536 | LOC134466 | AAGCCTTAATAGATAGTGCAACAACCAATGGATTTGATGATTTTGGGGACTACATCTTTG |
| 1,71 | 4,0E-14 | NM_005127 | CLEC2B | ATTGGAATTCAAGTAAATACAACTGTTCCACTCAACATGCCGACCTAACTATAATTGACA |
| 1,71 | 5,3E-12 | AB063115 | NAV2 | AGAGAGGATTTTATTTCTGGGAAATGGAATCGGTTTCTGAGTCCAGCCAACAGCAGAAGA |
| 1,70 | 3,2E-11 | CR597260 | CR597260 | ACACAGAAGTTTTCAGATTCTATGAAAAGGTATTGAATGAATTGAAGGCTCTGTTAAGGG |
| 1,70 | 6,0E-09 | NM_138286 | ZNF681 | AACCCTACCAATGTGAAAAATGTGGCAAAGCTTTTAACCAGTCCTCAAACCTTACTGGAC |
| 1,70 | 2,9E-07 | THC2310027 | THC2310027 | ACCAACACCCACAGGATAGTGGACGGCAAAGTGGTGTTTGAGACCAACATCACAGACGTC |
| 1,70 | 2,9E-12 | NM_139176 | NALP7 | ATCGCTCTACGAATTACACAGGAAGCGGGATTCGGGTCTCTAAGATGTCTTATGAATGCA |
| 1,70 | 4,4E-11 | M_001002294 | FM03 | TCGGGAGACTTCAGAAGCCTTGCTTCTTTTTCCATTGGCTGAAGCTCTTTGCAATTCCTA |
| 1,70 | 2,3E-13 | NM_170743 | IL28RA | GGTGAAAGCAACATGTATCCCTTTAGACTACTAACGGTATATGTTGTTCTTATGTATTTG |
| 1,70 | 2,5E-10 | NM_053277 | CLIC6 | TGCATTAACAGAAACTATCCTGGGATTAGGTGAAAAATGTCTAGCAAAAGAAACAAAGTC |
| 1,70 | 3,0E-09 | NM_013314 | BLNK | TAAGCGAGTATATAATATTCCTGTGCGATTTATTGAAGCAACAAAACAATATGCCTTGGG |
| 1,70 | 5,2E-14 | THC2440554 | THC2440554 | AGTTGTGAAATACTGAGAGCCTTTGATATTCACCATCAGGCTGAAAGAAGTTGATGTTTT |
| 1,70 | 2,9E-10 | AL133118 | AL133118 | TTTGCCTGATTATTCTCTGTTGAGCTCTACTTGTGGTCATTCAAGATTTTATGATGTTGA |
| 1,70 | 1,1 E-14 | THC2442210 | THC2442210 | GGCTCAGACCTTAAGAACTGATGGTCTTTTCTTTTACTTCTACACAAAAGTCTAAGCAGT |
| 1,69 | 5,1 E-11 | NM_024920 | DNAJB14 | TGATACTGAAAACTAAACTGAATAGTTGGTTCCTGAAATCTTGGACTGTTTATGACCTAC |
| 1,69 | 1,3E-12 | NM_015130 | TBC1D9 | TGTCACAGAGAATCTGAAAGTAGCAGCAAAGACAGAGGGCTCATGACAGGTTTTTGCTTT |
| 1,69 | 8,5E-11 | AK127374 | AK127374 | CCCTGGTTTTTAATTTTCAAAATATTTTCTTTTTGAAGAGCCAGATTCCAGTGATCCTGC |
| 1,69 | 7,2E-12 | NM_014399 | TSPAN13 | GAGAGGTTTTGAGATTTGTTGGTGGCATTGGCCTGTTCTTCAGTTTTACAGAGATCCTGG |
| 1,69 | 6,7E-06 | NM_017923 | MARCH1 | GTCACGCAAAAGATTTTCAGAAAATGTTCGGATATAATTAGCTCTGTTAAATACCCACAG |
| 1,69 | 4,7E-13 | NM_001042413 | GLIS3 | GTCCTGTGGGGTATCTCCCATTACCCATGTCGCCCTCAAATTACATAAGCGATCATAAAA |
| 1,69 | 7,0E-10 | NM_003878 | GGH | GGAGGTCAATTGCACAGCAGAATGTTCCAGAATTTTCCTACTGAGTTGTTGCTGTCATTA |
| 1,69 | 1,5E-09 | M_001017373 | SAMD3 | TTACAGCCTTGGCTGCGCTAGTAGCTGCCTTTCATGTATTTAGGATTGAGTGTCCAAGAA |
| 1,69 | 2,5E-11 | NM_002727 | PRG1 | AGGACTTGGGTCAACATGGATTAGAAGAGGATTTTATGTTATAAAAGAGGATTTTCCCAC |
| 1,69 | 9,8E-12 | NM_138383 | LOC92154 | GCCCATCGTCCCTGTGAAGACGCCCACGGTGCCTGACTCCCCCGGCTACATGGGGCCCAC |
| 1,69 | 4,7E-13 | NM_015914 | TXNDC11 | CGATGCCTCAGAAAACCTCCTTACCGAGAACACGTGGCTCAAGATCCTGGTGGCGACCAT |
| 1,69 | 2,3E-13 | NM_144643 | FLJ30655 | GAAGAAGCTAACCTTCAAAAAAGTCAGGCTCTACTTGAGGAGAAGCAAAAAGAAGAAGAC |
| 1,69 | 1,4E-10 | NM_030950 | RFP | TACCCCAGCCTGATCCTCTCTGATAATCTGCGGCAAGTGCGGTACAGTTACCTCCAACAG |
| 1,68 | 8,7E-13 | BC071593 | KIT | AAAGTGGTTGTTAGTTATAGATGTCTAGGTACTTCAGGGGCACTTCATTGAGAGTTTTGT |
| 1,68 | 2,0E-12 | NM_145165 | CHURC1 | TTATTTGTAAGTTATCATGAAAAGGTTTATATTCACTATTCTTTATTTCAGTGTAGCACT |
| 1,68 | 3,5E-04 | NM_003251 | THRSP | GATGAGTGATTCACCCTCTTACTTGGCGACCACTGATGAGATCAACAACAGGTGAACTAT |
| 1,68 | 1,7E-14 | NM_007107 | SSR3 | AATAAATGCATTTGCTTATTTGACAAACCATCAGTGTCCACTATTTGTTACCAGAGTTGG |
| 1,68 | 1,3E-08 | NM_004385 | CSPG2 | ACTTCCTGTGCCTTTCCTATCACCTCGAGAAGTAATTATCAGTTGGTTTGGATTTTTGGA |
| 1,68 | 5,5E-09 | BC022313 | PRG1 | GCATCATATTCGGAAATATCTTCCTAGGTTTATCTACCATTTAGTGTTGTTTAGTCAGAC |
| 1,68 | 1,6E-06 | THC2442822 | THC2442822 | ATGCTATTCCTGCTACACACAAAAACAAAAAGTTTGATATTTACGGAATTCCACACACTG |
| 1,68 | 6,6E-09 | NM_025134 | CHD9 | TATGTTTACAGCGACAGCCTCCATCTTCCAAGAAGAGCGATGGTTCTGGGACATATACTA |
| 1,68 | 6,7E-13 | NM_032852 | ATG4C | TTAGAATTTGTGAAGGGTATTTTAAGCCTGGAATATTGTGTGGGTATTATTGGTGGCAAA |
| 1,68 | 1,6E-10 | THC228791 | THC2287911 | GAGCTGCCATGTCTTCCGAGTCTGCAGAGGGGTCAGAAATCTGGCTGTTTGGAGAAATCT |
| 1,68 | 6,3E-10 | ENST00000371030 | ENST00000371030 | CACTTGGAGCACTAGAGGATGACGGAATGTCTTGTATATGCGGTCAACTGTTCAGGATTT |
| 1,68 | 1,6E-12 | NM_080546 | SLC44A1 | CTCCCTGTTCCATATGCTCGCAATCTCAGCTATTTGGAAGCTACCAGGAATGCTTTCTAA |
| 1,68 | 3,7E-14 | NM_006152 | LRMP | AGGTTCTCAGAATGACCGTAAGATAGCTTACATTTCCTCTTTTTGCCTTTATCTCCCCAA |
| 1,68 | 1,2E-11 | NM_005668 | ST8SIA4 | CTTTTGGTTTGGATATTGGGATGCTTAGAAATCCTTTCTGAGATGCATATGAGTGAGGAA |
| 1,68 | 2,7E-11 | NM_017904 | TTC22 | AGAACCAACCTCCCATCCTGAATCGCCTGGCAAAAATCTTCTACTTCCTGGGAAAGCAGG |
| 1,68 | 5,9E-09 | NM_017847 | Clorf27 | GAAAAACAGATGTTATCCTCAGCACAAATTCAGTAAAGAGACTACAAAAGGATGATCTTC |
| 1,68 | 7,5E-10 | NM_014905 | GLS | TTCAGTGTTACTGGAGTTTTCTTCATTGTGCACACAGGACAAATCTGATCTCTTTGGGAA |
| 1,68 | 2,2E-08 | AL564305 | AL564305 | ATGGCCTGCTGAGCCTCGTGTATCATCTCAAGGAAGGGCTGGAACATGGCGTGGAAGTTC |
| 1,67 | 1,0E-11 | NM_030674 | SLC38A1 | TTGGGGATGATTCTTACCTTGGTAATTAAATGAAGCTACACATTTGGGTAATCTAGCAAA |
| 1,67 | 1,8E-13 | NM_005767 | P2RY5 | TCTGTATTGCTGTTTCCAACTGTTGTTTTGACCCTATAGTTTACTACTTTACATCGGACA |
| 1,67 | 1,8E-08 | THC2302184 | THC2302184 | GCATCTTCTTAATTAGAGGAATTTACCAAAGTATGAGTCTATAAGTACATGCAGTAAGGC |
| 1,67 | 3,8E-12 | NM_014708 | KNTC1 | CACGGGCCAGCTAGCAGGATTTTCACATCAAATTAGAAGTCTGATTTTGAATAATATCAT |
| 1,67 | 2,1E-06 | ENST00000355095 | ENST00000355095 | TATCGTCACAACTTACTACACATAAGATAATTCATACTGGAGAGAAACCCTACAAATGTG |
| 1,67 | 2,6E-14 | NM_033632 | FBXW7 | CATGATGGAGGAAAACAACCTTTAAAGGGATTGTGTCTATGGTTTGATTCACTTAGAAAT |
| 1,67 | 1,3E-10 | NM_000885 | ITGA4 | ATTTCAAGTAGCTTGCTACTTGGACTTATTGTACTTCTGTTGATCTCATATGTTATGTGG |
| 1,67 | 8,8E-04 | AK054921 | CDR1 | AGACTATCCATGTCTTCCAGAAAATCCTTGTCTTCCCTCAAATCCATAGCTTCCGAAAAA |
| 1,67 | 5,0E-12 | NM_178127 | ANGPTL5 | TGGTCTAGCAAATCTAAATGGCATTCATCACTTCTCTGGAAAATTGCTTGCAACTGGAAT |
| 1,66 | 1,1 E-09 | NM_003816 | ADAM9 | ATTTCCGTTTCCATCATTGAATAAGTCTTATTCAGTCATCGGTGAGGTTAATGCACTAAT |
| 1,66 | 2,0E-09 | NM_001565 | CXCL10 | GTCAAGCCATAATTGTTCTTAGTTTGCAGTTACACTAAAAGGTGACCAATGATGGTCACC |
| 1,66 | 7,8E-13 | NM_006406 | PRDX4 | ATCTTCCTGTGGGTAGATCAGTGGATGAGACACTACGTTTGGTTCAAGCATTCCAGTACA |
| 1,66 | 9,0E-14 | M_018665 | DDX43 | AGACAGAAGTATTGGACATGTTGGCAGTATGAAGAGACCGGACTGATTTGACTGATTCTT |
| 1,66 | 4,9E-06 | NM_018318 | CCDC91 | TAAAGGAAGCTTTGATTCAGCAATCTCAAGAACAGAAGGAAATATTGGAAAAGTGTTTGG |
| 1,66 | 4,3E-12 | NM_000110 | DPYD | GTAAAATCCCCTTCGCTGAAATTGCTTATTTTTGGTGTTGGATAGAGGATAGGGAGAATA |
| 1,66 | 4,6E-15 | BC024007 | CTBS | GCCCCTGTAAATTGGAATACATGATGATTGAACCATCTGTTATGAGAATATTACATTTCC |
| 1,66 | 2,2E-09 | NM_003357 | SCGB1A1 | GAAGAAGCTGGTGGACACCCTCCCCCAAAAGCCCAGAGAAAGCATCATTAAGCTCATGGA |
| 1,66 | 9,6E-09 | NM_017520 | HSMPP8 | AGAACAGGAAGCTAGAGAACAAGAACGCTTTCTTAGAGAAGAAAACTGTGCCTAAAAAGC |
| 1,66 | 3,1 E-14 | BC027178 | PRPF40A | AGTGAAGGGGAATTGGAAAAGCGCAGAAGAACCCTTTTGGAGCAACTGGATGATGATCAA |
| 1,65 | 6,2E-16 | NM_017934 | PHIP | GCTGTTGTTCGAAGACACCTCTTTAACTTTTGGAACTTCTAGTAGAGGACGAGTCCGAAA |
| 1,65 | 6,9E-14 | NM_000600 | IL6 | ATTGTTGTTGTTAATGGGCATTCCTTCTTCTGGTCAGAAACCTGTCCACTGGGCACAGAA |
| 1,65 | 3,3E-07 | NM_018136 | ASPM | ATCACAAATCCCCTGCAAGCTATTCAAATGGTGATGGATACGCTTGGCATTCCTTATTAG |
| 1,65 | 6,1E-10 | NM_004962 | GDF10 | GCATTGACACCATTCCCCACAGAGATAGTCATGCTGAGTGTGGGTTGTTTAAACATGCAT |
| 1,65 | 2,4E-04 | NM_002113 | CFHR1 | TTGGATTAATTTGTGAAAATGTAATTATAAGCTGAGACCGGTGGCTCTCTTCTTAAAAGC |
| 1,65 | 2,2E-08 | NM_139250 | CTAG1A | TGATTGTTTGTCGCTGGAGGAGGACGGCTTACATGTTTGTTTCTGTAGAAAATAAAACTG |
| 1,65 | 1,5E-14 | ENST00000371142 | ENST00000371142 | ATATTATGGTGATATCCGAACCTAGTGAATGGTATGCTTGGGTGTTTTCCATTGAGAGTG |
| 1,65 | 2,9E-09 | M_001012651 | NKTR | CTTCTAGGAGTAGATCTTATTCCAGATCATATACAAGATCACGTAGTCTAGCTAGTTCAC |
| 1,65 | 1,8E-13 | NM_022168 | IFIH1 | CTACGTCCTGGTTGCTCACAGTGGTTCAGGAGTTATCGAACGTGAGACAGTTAATGATTT |
| 1,65 | 3,9E-08 | NM_173531 | ZNF100 | ACAGAATGTGGCAAAGCTTTTAACCGGTCCTCACACCTTACTACACACAGGATAATTCAT |
| 1,65 | 5,2E-09 | NM_004119 | FLT3 | CGTCTGCGTTTACTCTTGTTTTCAAAGGGACTTTTGTAAAATCAAATCATCCTGTCACAA |
| 1,65 | 4,3E-14 | NM_001812 | CENPC1 | AAGGAAGGCCATCAGGAGGATTCGTGATTAGTGGAGTACTATCTCCAGACACAATATCGT |
| 1,65 | 4,4E-16 | NM_013338 | ALG5 | GGAGCTGGCTACAAATGGGTAAAGACCTACTTTTTATACGACTTCGATATTTGACTGGTG |
| 1,65 | 1,6E-05 | NM_024665 | TBL1XR1 | AGAGATATTTAGAACATATTACCCTGTGACTTACGTAGGAAACCTAATATGCTGAGTATC |
| 1,65 | 3,7E-07 | NM_001085 | SERPINA3 | ATAGGTGAGCTCTACCTGCCAAAGTTTTCCATCTCGAGGGACTATAACCTGAACGACATA |
| 1,65 | 3,5E-09 | NM_001147 | ANGPT2 | CAGTTTACAGACGCTGCTGTCACAACCAAGAATGTTATGTGCAAGTTTATCAGTAAATAA |
| 1,65 | 3,4E-12 | ENST00000334564 | ENST00000334564 | CAAGGACCATTACTATGGAACATGATAAGATTTACACAAGCGATAATTCAGTCTCTAGTT |
| 1,65 | 4,1E-11 | NM_004665 | VNN2 | AAAGAGCCTGGGTGTTTGGGTCAGATAAATGAAGATCAAACTCCAGCTCCAGCCTCATTT |
| 1,64 | 3,2E-12 | NM_022841 | RFXDC2 | AAATCTCTGTAGGACTTTCTGGGGCTTCAGTACCATTTACACCCACGATGAAGGGCTTAA |
| 1,64 | 1,2E-07 | XM_934182 | LOC647022 | GGTAACCTAAAGAAAAACAACCAGACAAGTGCCCAACGACACTTAAAAAGGTTATTTATT |
| 1,64 | 4,3E-08 | M_017641 | KIF21A | CATCAACTTTTTCTCCTACCATACTATCCTCAGACAAAGAAACCATTGAAATTATAGACC |
| 1,64 | 8,5E-12 | NM_001781 | CD69 | TGTGCAATATGTGATGTGGCAAATCTCTATTAGGAAATATTCTGTAATCTTCAGACCTAG |
| 1,64 | 4,6E-12 | NM_032373 | PCGF5 | TGGTATATTCAACTACAGCTTTCTAAGGATAGGACTACTTTCATGTCTAGTAATACACTG |
| 1,64 | 1,5E-09 | THC2407068 | THC2407068 | TCTGAAGCTCTTTATGATGCACCGGTGCATTTTTATTTAAAAAATAGATTGTGACTCCTC |
| 1,64 | 9,8E-08 | NM_002162 | ICAM3 | CGTCTTCGTGGCGGTGTTACTGACCCTGGGCGTGGTGACTATCGTACTGGCCTTAATGTA |
| 1,64 | 5,9E-13 | THC2344956 | THC2344956 | CTTGGGACATAGTGACTTGGGAGAGTCAGTTAAAGGAAAGTTACCCATCGCCTTTTCCAT |
| 1,64 | 3,1E-11 | THC2364621 | THC2364621 | GTCTTTCCAGCTCCAGGCCAACATTGTTAATATTTGGATTTTATGAATCTTTAGCACTTG |
| 1,64 | 5,2E-13 | NM_024715 | C5orf14 | GCTAATTCCAGGACAAGAGCAGGAACATGTGGAGTAGTGATGGTCTGAAAGAAGTTGGAA |
| 1,64 | 2,5E-07 | ENST00000288911 | ENST00000288911 | TCCAGGAAAAGTGTCTTCTCAGAAACAACCAGCTGAGAAGGCTACAAGTGACGACAAAGA |
| 1,64 | 4,4E-12 | NM_000497 | CYP11B1 | ATAAAGATGGTCTACAGCTTCATATTGAGGCCCAGCATGTTCCCCCTCCTCACCTTCAGA |
| 1,64 | 8,2E-10 | NM 006947 | SRP72 | GTAAACACTTGCCATCGTCAGATAGTATGTCTCTAAAAGTAGATGTTGAGGCTCTTGAAA |
| 1,64 | 2,2E-16 | NM_004598 | SPOCK1 | CTTTTTCTCAAAGTCACTGATGTTTGTTCCTGTTAAATGTATAGCATTGTAATGAGAGCC |
| 1,64 | 3,1 E-10 | NM 017957 | EPN3 | TCCCTCAGCTTCCTCCTTGGTCAACCTTGACTCGTTGGTCAAGGCACCCCAGGTTGCAAA |
| 1,64 | 1,7E-11 | NM_015032 | APRIN | ATCTAGTGCAATTGAATCCACACAGTCCACACCACAGAAAGGACGAGGAAGACCATCAAA |
| 1,64 | 2,7E-10 | 0 no_017554 | PARP14 | AAATATTAACATTTCCCTGGACCATAAGAGACCTTTGATTAAGGTTTTGGGAATTAGCAG |
| 1,64 | 1,3E-13 | M_018279 | TMEM19 | TTTGGTGGTTTAGCTGGATTACTAGGATCAATTGTGGACTCATACTTAGGGGCTACAATG |
| 1,63 | 1,6E-08 | M_001012506 | CCDC66 | TACCACTAAAAAACAGTAGCTATGAGAGAGAGAATTTGATCTCAGGAAGTAATCAAACAG |
| 1,63 | 4,1E-13 | NM_005755 | EB13 | TCTAGCTGAGAAATGGAGATGTACTACTCTCTCCTTTACCTTTACCTTTACCACAGTGCA |
| 1,63 | 8,8E-14 | NM_001165 | BIRC3 | TCCTATACATCGAAGGTGTGCATATATGTTGAATGACATTTTAGGGACATGGTGTTTTTA |
| 1,63 | 7,1E-04 | NM 033647 | HELB | AATCTCCACAAGTGTCTTCCAGACTTCAGAATTTGAGACTGAATAATTTAATTCCCAGGC |
| 1,63 | 1,6E-05 | ENST00000374279 | ENST00000374279 | AATGGTTTTGTGCAGTGAACAACACATGGCGAGGTACTAACTGAGAAACTTTTTCATGCT |
| 1,63 | 8,1E-08 | M_006910 | RBBP6 | CATCTTCCTCTCAGAAGGATGAAAAAATCACTGGAACCCCCAGAAAAGCTCACTCTAAAT |
| 1,63 | 8,6E-08 | THC2376215 | THC2376215 | TGTCAACTACTCTGAGAATGCAAAATTCTTGTTTTAAAATGTGTATCTTACAACTCCTTC |
| 1,63 | 8,OE-09 | NM_032229 | SLITRK6 | TCCATACCTTGTAAATAAGTGCCTTACGTGAGTGTGTCATCAATCAGAACCTAAGCACAG |
| 1,63 | 1,6E-14 | BF213738 | BF213738 | AAATCGAACAGGACAATGGGTAGATGGAGCTACATTTACCAAATCGTTTGGCATGACAGG |
| 1,63 | 7,8E-11 | NM_152527 | SLC16A14 | GAACTTTTTATCCAATGACTCAACTCTAATTACATCTAAGTTAGACTTGCTCACGTTCAG |
| 1,63 | 4,9E-13 | NM_004867 | ITM2A | CTAGTTGCTGTGGAGGAAATTCGTGATGTTAGTAACCTTGGCATCTTTATTTACCAACTT |
| 1,63 | 6,5E-11 | NM_001197 | BIK | TTTTCGTTTTTTCTAAAAGATGAATTCCTATGGCTCTGCAATTGTCACCGGTTAACTGTG |
| 1,63 | 5,3E-12 | NM_152349 | KA21 | GGAAAAAGTCTGTTTCTCTTCGATTTGATCTTCATTTAGCAGCCACTGATGAAGGGTGTT |
| 1,63 | 1,9E-04 | NM 007281 | SCRG1 | TTCGTGATTCCTTGCAACAATCAATGAGAATCTTCATGTATTCTGGAGAACACCATTCCT |
| 1,63 | 1,8E-11 | AB014594 | DOCK10 | TACAATAGTGCACACATTTTTCACTTTAAGGATCCAACATTGCTCTGAGGTTTTAGAAAC |
| 1,63 | 2,7E-08 | M_001001415 | ZNF429 | ATGTGGCAAAGCTTTTAACCGGTCCTCACACCTTACTAGCCATAGGAGAATTCATACTGG |
| 1,63 | 2,4E-12 | NM_052939 | FCRL3 | CAATCATGACCATTTGATCCAAGTGTGATCGAAAGCTGTTAATGTGCTCTCTGTATAAAC |
| 1,62 | 1,9E-12 | NM_002348 | LY9 | CACCACCACAACAGAATGATCTTGAGATTCCTGAAAGTCCTACCTATGAAAATTTCACCT |
| 1,62 | 1,5E-11 | M_016388 | TRAT1 | ATTTGGCTCCTATTGAAGATGGCTTCTAAGAAAACAAGATGCACAGAGGACACAGAAGGA |
| 1,62 | 1,5E-08 | NM 015723 | PNPLA8 | GTGCTACAGATACAGAAGAAGTCCATATAATGCTTGATGGCCTGTTACCTCCTGACACCT |
| 1,62 | 2,3E-11 | AK075484 | AK075484 | CTTTAAAAGGAGTGACATTTTCTAGGGAGGTAATTGTTGTGGATCTTGGGAATGAATACC |
| 1,62 | 4,4E-11 | NM_182556 | LOC283130 | CCCACGAGGCTTTGTTCTCCTGGGGTTGCTTCCTGGACCCTGAACAAGTTGTGCTCTCAT |
| 1,62 | 2,2E-11 | NM_004792 | PPIG | GTCCCTTCTCAAAAATAAAACAAAGCAGTCAGGACGATGAATTAAAGTCCTCCATGTTGA |
| 1,62 | 1,5E-11 | NM_181354 | OXR1 | CATTGAGGAAATGAAGACTGGATACTTCTGTATCTGTGAAGTTGGCACAGGTAACATTTG |
| 1,62 | 5,2E-06 | ENST00000377003 | ENST00000377003 | ATCGCAGTTAAAGCATGAAACACTTGAATTGGAAAAAGAACTCTGTAGTTTGAGATTTGC |
| 1,62 | 8,0E-14 | AK095791 | AK095791 | TAAAAGAGCAATCGCAGTTCGGTTTCACATGTATTGTTCTATAATCCAGGCAATAAATGC |
| 1,62 | 6,8E-10 | NM_032121 | RP11-217H1.1 | CATCTTTGTGTGGATGTGTATACTTTACGCATCTTTCCTTTTGAGTAGAGAAATTATGTG |
| 1,62 | 1,7E-11 | THC2280750 | THC2280750 | GAGTGTTGTGTCAATGAGAAACAAGACAGTTCATACTCCAGAACCTGTTTAAGAATTAGA |
| 1,62 | 1,7E-10 | BC037528 | BC037528 | TCACATGGACTTCATTCTGATAAAGGGTGTTTCTTCTTGTTTCACTGTCCATACCTTTGT |
| 1,62 | 2,8E-11 | NM_012097 | ARL5A | GAAGTAACTTGACCCAAAACACCTTTTATGTTTGCTTAGGGTATATTTTTTCAGTGTCTG |
| 1,62 | 7,4E-10 | NM_002913 | RFC1 | AGATGGCCTGTTGAATCTGATTCGGACTATGCCAGGCAAGAAATCCAAGTATGAAATAGC |
| 1,62 | 3,7E-12 | THC2350023 | THC2350023 | GATTTGTTCCAGTGTTGGAGCCCTTTTTAATGAAAATTCTCAACACCTACACTGGAAAAA |
| 1,62 | 8,2E-13 | M_017693 | BIVM | ATTTCATGAGTCTAGTAGTTCTTTGATTTATAGCAGGATCTTGCTTGCCTCATTTGTTTC |
| 1,62 | 2,0E-13 | THC2412604 | THC2412604 | GTGCTTGTGCATGCGAATATAAAAATTGATATTCATAGACTCGTTAACCACAGTGTTAGG |
| 1,62 | 1,3E-12 | NM_001010919 | RP1-93H18.5 | CAAATTTCATCACTGTATACTTTCAATCCGAAGGGCCAGTACTACAGCATGTTGCACAAA |
| 1,62 | 1,0E-10 | NM_019839 | LTB4R2 | CTTGCTCTGGGCCCCCTACCACGCAGTCAACCTTCTGCAGGCGGTCGCAGCGCTGGCTCC |
| 1,61 | 8,6E-10 | NM_003262 | TLOC1 | ATGGGGATTGTGAAGAGGATGAGGAAGAGGAAAATGATGGAGAAACACCTAAATCTTCAC |
| 1,61 | 1,7E-07 | NM_032872 | SYTL1 | GCAGCAGCTATGGGCTGCAGGTGCCCTGGATGGATTCCACACCTGAGGAGAAGCAGCTGT |
| 1,61 | 8,7E-12 | NM_001957 | EDNRA | GGTTGGTTTGATAAAGCAGTATTTGGGGTCATATTGTTTCCTGTGCTGGAGCAAAAGTCA |
| 1,61 | 2,1E-06 | NM 007139 | ZNF92 | ATGTGGCAAAGCTTTTAACCGGTCCTCAACCCTTACTAAACATAAAAGAATTCATACAGA |
| 1,61 | 5,9E-11 | NM_152332 | MTAC2D1 | TGATGATATGTATGCATCATTATAATATAGTATAGTTTCGCTGCCCTAAACATCCTCTGC |
| 1,61 | 7,2E-04 | BX119852 | BX119852 | AACGAGAGAGAGGTAAAGCCAACAAGCAGAACAGACCTACATTTGCAGCATCTCCAAGTT |
| 1,61 | 1,3E-08 | NM_014803 | ZNF518 | TGGATTTAGCAGACCTAGGCTTTCAAAAGATTCCATCAGAACTTTGCGGCTTTTCCCTTT |
| 1,61 | 5,4E-09 | AK091335 | AK091335 | TGTAGACTGAAGGAGTCTTTCAAAACACCCCAGCATTAAATCTCACTCTGCGCTGCTTCT |
| 1,61 | 1,2E-15 | NM_014445 | SERP1 | TCACCAATTAAGTGCAGTTTATATTCAGGTTGGATTATGCATGTTTAGGTAAACGAAAGC |
| 1,61 | 3,1E-14 | NM 006699 | MAN1A2 | AATGGAAGGGTCTTCTTCACCACTCCTTACCTTCTATGTGATGGAAAGACTAGAGCTTAT |
| 1,61 | 4,0E-07 | NM_005348 | HSP90AA1 | GAAGACTTTAGTGTCAGTCACCAAAGAAGGCCTGGAACTTCCAGAGGATGAAGAAGAGAA |
| 1,61 | 1,1E-07 | NM 012484 | HMMR | ACTATTTCTTCAGAGTTTGTCATATACTGCTTGTCATCTGCATGTCTACTCAGCATTTGA |
| 1,61 | 6,5E-04 | BC045756 | BC045756 | TGGGTGTTTTTTGGTTTTTGGTTTCTGGTTTACAATCTCGTCATTCAACAAAGATGGGAG |
| 1,61 | 6,0E-11 | BE272930 | BE272930 | AGAGGACTAGATTATGAATATGACTTCATTGATGACAGCCGTCCCCTCCTCTTCCTAAAC |
| 1,61 | 2,4E-12 | M_019891 | ER01 LB | ATGGACTGTGTTGGATGTGACAAATGCAGATTATGGGGAAAATTACAGACTCAGGGTTTA |
| 1,61 | 2,5E-12 | NM_003956 | CH25H | TTGTGGATGGTGTTGACTTCTGACTCTAAAAGCAATCAAACATGTTTCTGCTGGACAGTG |
| 1,61 | 2,9E-11 | NM_003469 | SCG2 | GATGGCATATGAAAACCTGAACGACAAGGATCAAGAATTAGGTGAGTACTTGGCCAGGAT |
| 1,61 | 2,0E-09 | NM_030627 | CPEB4 | GTATGTCAGTTAATTGTACTACTTGGCTGAATTTCCATATAGTTTTTACTGTGTATGGGG |
| 1,61 | 2,4E-08 | NM 033356 | CASP8 | AATCCAGCTATGAATATAGAGGGCTTATGATTCAGATTGTTATCTATCAACTATAAGCCC |
| 1,61 | 1,4E-14 | NM_002872 | RAC2 | CACTTACCTGTGAGAGTCTTCAAACTTTTAAACCTTGCCAGTCAGGACTTTTGCTATTGC |
| 1,61 | 3,1E-14 | NM_005830 | MRPS31 | ATATATTTCTGGAGAAACACCTGGAGAGCTTTCCAAAACAAGGACCAATTCGCCACTTCA |
| 1,61 | 2,3E-09 | NM_152997 | C4orf7 | GTCACGATAAACCTGGTCACCTGAAATTGAAATTGAGCCACTTCCTTGAAGAATCAAAAT |
| 1,61 | 3,9E-09 | M_017662 | TRPM6 | TGCCCAATTGCCAAACTAAAGACATCAGTTCATTGGTCAAATATTTGTTACCTGGAATGG |
| 1,61 | 9,6E-10 | AA541413 | AA541413 | TGTACAGAATCCTTTCCAGCTGTAAGTCATCAGCAAGTAAAAAATTTAGTATGGCAATAG |
| 1,61 | 5,1E-12 | NM_021237 | SELK | TCTGCAGGGAACTGGCCTGACTGACATGCAGTTCCATAAATGCAGATGTTTGTCTCATTA |
| 1,61 | 1,7E-10 | NM 016617 | UFM1 | TCCATACTCTTCCTATGAAGAGGGAATGCGTATGAATTAAGGCTACTACTGTCACAGAAG |
| 1,61 | 8,7E-13 | AF011794 | CCPG1 | AAGTCAGAAGAGCTCATATATATAATTCTAATGTCCCACCTATGTCCATTCCATGTACCA |
| 1,60 | 1,1E-11 | NM_030782 | CRR9 | CTGCCGCCAGCCAAGTGCAACTTGAATTGTCAATGAGTATTTTTGGAAGCATTTGGAGGA |
| 1,60 | 1,6E-09 | NM_005054 | RGPD5 | TTTGAAAAGTAACAACAGTGAAACTAGTTCAGTAGCCCAGAGTGGATCTGAAAGCAAAGT |
| 1,60 | 7,2E-07 | THC2269172 | THC2269172 | GCAGAGATCCACGAGGTATTGAGAGCAACGCGGAAAATAGTAGTGAACCCTGTAAAAATC |
| 1,60 | 7,3E-08 | NM 021038 | MBNL1 | ATCCTTTCAAACCCTCATGACTGACAAAAACTCCATGGGGCCAAATCTGCCTGAAGATCA |
| 1,60 | 7,2E-14 | NM_003263 | TLR1 | ATATTCCTCCTGTTGATATTGCTGCTTTTGGAAGTTCCAACAATGACTTTATTTTGCATC |
| 1,60 | 5,8E-10 | NM_005086 | SSPN | AATGTTTTGATTTGCTTTAAAAATGGCCTTCCTACACATTAGCTCCAGCTAAAAAGACAC |
| 1,60 | 2,5E-14 | NM_144778 | MBNL2 | ATCTTTCTGTAACACTTAAAGAATTCCCTCATTCATTACCTTACAGTGTAAACAGGAGTC |
| 1,60 | 2,6E-12 | NM_005863 | NET1 | TCTTTGAAAGGGGGGAGGAGGAGTAAAAGCCCGATTATAATGGTGATCAATTCAAGTCAG |
| 1,60 | 2,9E-06 | M_001039884 | FLJ44894 | AGAAATCCTACAAATGTGAAGAGTGTGGCAAAGCCTTTAACTGGCCCTCACAGCTTGCTA |
| 1,60 | 9,8E-09 | M_018293 | ZNF654 | CCTGATGAAAATGGTTCAGAAAGATCTGTCAATCAAGCAGTAGTGTGAAAAAAGCACTAT |
| 1,60 | 7,3E-10 | NM_000576 | IL1B | GGAATCAATTCAATTTGGACTGGTGTGCTCTCTTTAAATCAAGTCCTTTAATTAAGACTG |
| 1,60 | 1,7E-09 | NM_144973 | MGC24039 | TTCCGGCATTCTTACTAATAGTCTTTCTATTATACTATTGGATCTTTATTCATCCACCTA |
| 1,60 | 5,2E-12 | NM_174953 | ATP2A3 | CTCTGATGGACAGAATTTTTCTCTTAACTCAGCTTTTGCTACTTTGGCAAAAACTAGCGA |
| 1,60 | 2,4E-06 | AK021980 | AK021980 | CAGGCAGAATATGATCTGTGTCCAAAAGTGAACTTGAGTCAGGATTGAATCAATTTCAGC |
| 1,60 | 9,4E-12 | AL109696 | AL109696 | ATGAGGGAATTTTGGGAGAGCTACCACATCAGTACTTTGGCACGCATTAACTGTTCCACA |
| 1,60 | 1,3E-04 | NM_080632 | UPF3B | CGTTCTGATAGCGAACTTAAAGATGAAAAACCAAAGAGACCTGAAGATGAGAGCGGCAGA |
| 1,60 | 1,2E-11 | NM_000619 | IFNG | ACTTGAATGTCCAACGCAAAGCAATACATGAACTCATCCAAGTGATGGCTGAACTGTCGC |
| 1,60 | 5,8E-07 | NM_003429 | ZNF85 | TGCACACCTTACCACACATGAGGTAATTCATACTGGAGAGAAACCCTACAAATGTGAAAA |
| 1,60 | 1,3E-12 | NM 003414 | ZNF267 | TGTGATGAATGTGGTAAAGCCTTCAGCTATAGGTCATACCTCACTACACATCGGAGAAGT |
| 1,60 | 9,3E-06 | NM_182515 | ZNF714 | GTCTTCAACCCTTACAAAACATAGAAGAATTCATACTGGAGAGAAACCCTACAAATGTGA |
| 1,59 | 3,7E-14 | NM_002738 | PRKCB1 | CCTCACTTTGATGTTGTTTTGCAAGATGTTTGTGGAAATGTTCATTTGTATCTGGATCTC |
| 1,59 | 9,8E-09 | ENST00000381475 | ENST00000381475 | GCCAGATTTACCACCATTCATTCACTGAAAATATTTGCAGACAATTTTTCTGTTTGTCTG |
| 1,59 | 1,8E-14 | NM_015355 | SUZ12 | CTTGATTGGTAAGCTCTCCAAATGATGAGTTCTAGTAAACTCTGATTTTTGCCTCTGGAT |
| 1,59 | 2,4E-11 | NM_001050 | SSTR2 | AGGATTGCTCAGCATGAGTCCAATTCAGAGAACGGTGTTTGAGTCAGCTTGTCTGATTGA |
| 1,59 | 4,6E-11 | NM_014802 | KIAA0528 | GTAAGTGGTGACGCAGTGGTTTTTGTTCGTGAATCTGACTTAGAAGTGGTGTCATCTCAG |
| 1,59 | 2,0E-12 | NM_003121 | SPIB | CCTGTCCAAGGTTCCCTCTTGTCAGATCTGAGATTTCCTAGTTATGTCTGGGGCCCTCTG |
| 1,59 | 1,6E-08 | ENST00000373093 | ENST00000373093 | ACATTTTGTAACAATTTGGGTTTCATAGTAACTGTGTTACTTGCCAATCATTCTAGGCTG |
| 1,59 | 2,2E-08 | NM_015271 | TRIM2 | ACTCTTCAGCACATTCCTTTACTAAGCAGTTTAAAGCCGTCCTAGTGGAGCAAGCCCTAA |
| 1,59 | 3,9E-07 | NM_005502 | ABCA1 | CCAAAGAGCCATGTGTCATGTAATACTGAACCACTTTGATATTGAGACATTAATTTGTAC |
| 1,59 | 3,6E-12 | NM 000629 | IFNAR1 | TCCTGTTTAGGGAAAGAAAAAACATCTTCAGATCATAGGTCCTAAAAATACGGGCAAGCT |
| 1,59 | 2,0E-11 | NM_014584 | ER01L | ATTGTGTTGGTTGTTTTAAATGTCGTCTGTGGGGAAAGCTTCAGACTCAGGGTTTGGGCA |
| 1,59 | 1,8E-11 | NM_052954 | CYYR1 | TTGTATTAAGTGTCATAATAGCATCAATGTGCACATTACCAGTATAGACTGTTGGAGTTC |
| 1,59 | 3,4E-12 | NM_032941 | RECQL | GTGACAAAGTCCACGCAGAACTCTTTCAGGGCTGAATCGTCTCAAACTTGTCATTCTGAA |
| 1,59 | 5,2E-07 | NM_032413 | C15orf48 | AGTGTGCGGAAATGCTTCTGCTACATTTTTAGGGTTTGTCTACATTTTTTGGGCTCTGGA |
| 1,59 | 2,8E-14 | NM 006068 | TLR6 | CAGAGTGAGTGGTGCCATTACGAACTCTATTTTGCCCATCACAATCTCTTTCATGAAGGA |
| 1,59 | 3,0E-12 | NM_012092 | ICOS | AAGCTGTGCCTCGACACATCCTCATCCCAAGCATGGGACACCTCAAGATGAATAATAATT |
| 1,59 | 3,5E-12 | M_016649 | C20orf6 | CCAGAAAAGGATCGAAAAGTCAAAATTGACAAGAGATTTCGAGCCATGTTTCATGACAAG |
| 1,59 | 3,4E-12 | NM 004315 | ASAH1 | ATGAACTCGATGCTAAGCAGGGTAGATGGTATGTGGTACAAACAAATTATGACCGTTGGA |
| 1,59 | 4,8E-07 | THC2432288 | THC2432288 | GGAAAAAACCAGCCTTGAAGTGTCTTCTCAGAAACAACCAGCATTGAAGGCTATCTGTGA |
| 1,59 | 1,9E-09 | M_001009568 | SMPDL3B | AGGTGTCCCCATAAGCGCCATGTTCATCACACCTGGAGTCACCCCATGGAAAACCACATT |
| 1,59 | 8,7E-06 | NM_004719 | SFRS21P | ATCTGAAAGCAAAGTGTACCAACCTGTATCTTGTCCCCTAAGTGACTTATCTGAGAATGT |
| 1,59 | 1,3E-13 | ENST00000367001 | ENST00000367001 | TTTGATGTAGCTCTACCGATACTATGTGGTAATGCTATTTTGTTTTACTAACAAGCTCTG |
| 1,59 | 2,9E-09 | NM_005263 | GF11 | CTGTGGACAAGATGTCATTCATTCACTCAGCAAATGTTCATGGATCACCGGCTACCAAGT |
| 1,59 | 9,2E-09 | A_24_P417757 | A_24_P417757 | GAGCAGAGACATCCAGGAGAGGAATGGGTGAAATACAGTGATGATGAGGAATCTGATTCT |
| 1,58 | 4,9E-13 | M_016040 | TMED5 | GCTCTGATATGCATTTGGATGATTAATGTTATGCTGTTCTTTCATGTGAATGTCAAGACA |
| 1,58 | 2,2E-07 | NM_001242 | TNFRSF7 | AGCCACAACTGCAGTCCCATCCTCTTGTCAGGGCCCTTTCCTGTGTACACGTGACAGAGT |
| 1,58 | 5,2E-10 | NM_181783 | TMTC3 | GCAAAGGAATTAAAGGCTTTGCCAATTTTGGAGGAGTTACTCAGATACTACCCTGATCAT |
| 1,58 | 1,4E-09 | M_019600 | KIAA 1370 | CTTTTTGTACTGTTGAAACCACTTCATTGGACATGTTGCAATAGCAAAACCCCCAGTTAG |
| 1,58 | 7,4E-10 | NM 006267 | RANBP2 | CAACTTCAGAAGTTGAAGTGTCTAGCACATCTGAAACAACACCAAAAGCAGTGGTTTCTC |
| 1,58 | 3,5E-08 | NM_139211 | HOP | GGAACACATTTCCTTCTGAACTTACTTCCCTAAGTCACTTTCCTTATGTATCATCTAATA |
| 1,58 | 3,0E-07 | M_018151 | RIF1 | GGTTTGCTTAATCAAACAGAATGTGTGTCAGATAATCAGGTTCATCTTTCTGAATCTACA |
| 1,58 | 1,5E-12 | NM_052946 | NOSTRIN | ATGGTGGTTTGGATCTTTGAATGGGAAAAAAGGCCATTTTCCTGCCGCTTATGTGGAGGA |
| 1,58 | 6,5E-11 | NM_152835 | PDIK1 L | TGGTGCTAAGGGATACTTTGTCATTATGATGAAGTAAGTGTTAAGTGTCAGATAAATAGC |
| 1,58 | 1,2E-06 | NM 021023 | CFHR3 | TCAGGAAGTTACTGGGATTACATTCATTGCACACAAAATGGGTGGTCACCAGCAGTACCA |
| 1,58 | 1,3E-12 | NM_003142 | SSB | TTTGTTTGCGGGGCTTTTAAAAGGAAAACCGAATTAGGTCCACTTCAATGTCCACCTGTG |
| 1,58 | 2,0E-09 | NM_024689 | CXorf36 | CAGCACTGAGAACTTCCAGAAAGTGTTAGCCTTCTCCCAACTGTGTTATACCAACCACAT |
| 1,58 | 2,9E-12 | AK027214 | CNOT6L | GAGGTATATGCACTAACAAAACATTTTCCTCCTTTCATATACGCATGTTACTTAGCTATG |
| 1,58 | 9,9E-11 | NM_001323 | CST6 | TCAGCTCCTAAAGCACAACTGTGTGCAGATGTGATAAGTCCCCGAGGGCGAAGGCCATTG |
| 1,58 | 2,2E-07 | AK098043 | AK098043 | AGGGTATCTAGTTAGAGTTATATATATAGTTAGATACATTTGTTTCTGTTGATATTCAGT |
| 1,58 | 8,6E-06 | NM_006144 | GZMA | GAATGAATATGGTTTGTGCTGGAAGCCTCCGAGGTGGAAAAAACTCGTGCAATGGAGATT |
| 1,58 | 2,6E-10 | NM_005771 | DHRS9 | ACTCATTTAGATCGTGCTTATTTGGATTGCAAAAGGGAGTCCCACCATCGCTGGTGGTAT |
| 1,58 | 8,5E-12 | M_018297 | NGLY1 | AAGTAGTCTGTTGGTTCAGTGCATGCTTAGTTGGCAGTTACCACCCTGTGCTAGCATATT |
| 1,58 | 1,5E-07 | NM 015687 | FILIP1 | GGAAAAGGCTGAATTAAGCCTAAAAGATGATCTTACCAAGTTGAAGTCATTTACCGTGAT |
| 1,58 | 5,3E-11 | ENST00000358356 | ENST00000358356 | ATATTTAGCATGGATCCCGGTACACTTTCAATACTTAATAAATGGTCAATGTTATTCTTT |
| 1,58 | 5,3E-08 | NM_014234 | HSD17B8 | AGCTTGGACGACATGGGATCCGCTGTAACTCTGTCCTCCCAGGGTTCATTGCAACACCCA |
| 1,58 | 7,0E-09 | NM_007237 | SP140 | ACGGAAAGTGATCAAGCGTGTGGCACAATGGATACTGTGGATATTGCAAACAACTCCACT |
| 1,58 | 4,3E-14 | AK025669 | AK025669 | ACAACTACCTGTAGCTGGGGAGAACATCTTCACATGCCCCATAAACTCCATTTTGGCAAA |
| 1,58 | 4,3E-12 | NM_170736 | KCNJ15 | GCTGTTTCCACATCAGAACTCCCTTCAAACACAAAGATTGCTGTGAAAACGAAAATGTGT |
| 1,57 | 2,5E-10 | THC2314600 | THC2314600 | AGAAAGTAAGTTTCCAGAGCAAATCTGACCTAGCATTTGGTATGCTAGGCTCTGCTTTTC |
| 1,57 | 4,0E-06 | NM_004460 | FAP | TTTCTGGTACTCTGTGAAAGAAGAGAAAAGGGAGTCATGCATTTTGCTTTGGACACAGTG |
| 1,57 | 2,2E-11 | AK126324 | SMCHD1 | ACAAGTACCTGGGCATGAATTTCCATTTCGATTCAGATGGGACTGGAAACAACCATTCAA |
| 1,57 | 1,7E-08 | A_32_P67223 | A_32_P67223 | TCCAAGTCAGAAAAACTATTCATATAGGGATGGGCTATTAGCAAGAGTATATGAATAACG |
| 1,57 | 1,2E-07 | AK131420 | ZNF66 | AAACCCTATAAATGTATAGAATGTGGCAAAGCCTTCAACCGGTCCTCACACCTTACTACA |
| 1,57 | 2,2E-08 | BC030100 | BC030100 | AGCACAAAATTCAACTCCAACCTGCAGTTAGGATCCCAAAGAGATGTTGACATTTATAGC |
| 1,57 | 5,2E-05 | NM_014159 | SETD2 | TGGAACTGGGGTTGTGTATGATCGAACTCAAGGACAAGTACCAGATTCCCTAACAGATGA |
| 1,57 | 5,5E-10 | NM_005531 | IF116 | GACCAGGAAAAACAAGAAAGACATACTCAATCCTGATTCAAGTATGGAAACTTCACCAGA |
| 1,57 | 1,1E-10 | NM 001031716 | OBFC2A | ACATGTCATAAGTGGTACCCACTTCCCCTTTTTACTGTAGGGTGGATAACTCTTAGGATT |
| 1,57 | 1,7E-11 | NM_004180 | TANK | AGTTTTCCCACCATCCATTACATCCAGGGGGGATTTCCTTCGGCATCTTAATTCACACTT |
| 1,57 | 5,3E-07 | NM_033402 | LRRCC1 | TGGAGGCTCAAATTGAGAGTTTATCTAGAGAGAATGAATGTCTGCGAAAGACAAATGAAA |
| 1,57 | 1,1E-11 | AK096677 | AK096677 | ACTGCTCCCTTGTCTGATACCTGTATTTCTCCAAATGATAAAAAACTTCGTCGCTTAACC |
| 1,57 | 2,2E-08 | NM_153350 | FBXL16 | ATGCGGAAGATATAACCTTATATTTGGTAAGTGTTTCTTGTGCTATTTTATCACGTGACC |
| 1,57 | 2,6E-09 | NM_177457 | LYNX1 | ACGGAGACAACTGCTTCAACCCCATGCGCTGCCCGGCTATGGTTGCCTACTGCATGACCA |
| 1,57 | 2,1E-11 | NM 024561 | NARG1 L | CCAATTCGACTTCCATACATACTGCATGAGAAAGATGACCCTTCGTGCCTATGTTGACCT |
| 1,57 | 7,0E-13 | NM_030764 | FCRL2 | CATGACAGCTGGAGTTCTCTGGGGACTGTTTGGTGTCCTTGGTTTCACTGGTGTTGCTTT |
| 1,57 | 5,5E-05 | NM_006256 | PKN2 | AAGACCTCTTAAAAATAGCAACCCTTCATTTGCTCTCTGTGCCACCAATAGCTTCTGAGT |
| 1,57 | 3,7E-12 | NM 032383 | HPS3 | ACATCAGTTCACTGTTGTTGGAGACATGACAATCATTTTCATCCCAAGAACACTTTAAGG |
| 1,57 | 2,4E-09 | NM_014607 | UBXD2 | AGTGGACATACGGAAAGACTGTGACTTTATTTTGTAATGGGAGGAAGAAATTTTCTCAGA |
| 1,57 | 2,5E-13 | BC033025 | PPAPDC1B | AGATGACTGTTTCTACCTCATTTGACCATAACTGTACTTGTGAGCTTCTTTCCTTCCATT |
| 1,57 | 1,2E-09 | NM_002064 | GLRX | CTGATAAAACTTACAGCCCCCTACACCAAGAGTGTATCTGTGAAAGAGCTCCTACACTTT |
| 1,57 | 1,3E-08 | NM_014373 | GPR160 | AGGCACAGAATGCTTATTCTCGTCACTGTCCTTTCTATGTCAGCATTCAGAGTTACTGGC |
| 1,57 | 3,2E-06 | NM 020841 | OSBPL8 | TTATGGCTCTTCGAAATCATTTAGTTTCAAGCACACCGGCCACGGATTATTTTCTGCAAC |
| 1,57 | 1,1E-14 | NM_020240 | CDC42SE2 | GGTGCCAGTAAGCGTAGAGCGGAAATGTTGACTTTAGTTAACATTGGGTTTAGCATTTCC |
| 1,57 | 3,2E-04 | NM_003005 | SELP | AAATACCTCTTTATTTTTTGATTGAAGGAAGGTTTTCTCCACTTTGTTGGAAAGCAGGTG |
| 1,56 | 1,6E-09 | NM_006425 | SLU7 | CTATGACAAGGGATCTGAAGTGCATCTACAGGCAGATCCTACAAAGCTAGAGCTGTTGTA |
| 1,56 | 4,9E-10 | NM 002262 | KLRD1 | ATACAAAGAACTGCATAGCGTATAATCCAAATGGAAATGCTTTAGATGAATCCTGTGAAG |
| 1,56 | 1,7E-13 | NM_012224 | NEK1 | AAGATGGAGTCTCGAGTACTGTGGACCAACTTAGTGACATTCATATAGAGCCTGGAACCA |
| 1,56 | 4,1E-08 | AK123473 | ZNF292 | GGTGTGGAAAAACCCGGTACTGTGCACTATTCTTTCCCAGGAACCATTGGATAAGGATAA |
| 1,56 | 1,3E-10 | 0 no_000043 | FAS | ATGTCTATCCACAGGCTAACCCCACTCTATGAATCAATAGAAGAAGCTATGACCTTTTGC |
| 1,56 | 3,3E-10 | ENST00000233161 | ENST00000233161 | TGTTGTTGTTTTAGATGGGCTCATTACATAACGAGTTAATTGTCACTAGTAGGAGACTGT |
| 1,56 | 2,0E-11 | NM 014350 | TNFAIP8 | TGGCCAGCTATGTCCTCTAGGAAATGACAGACCCAACCACCAGCAATAAACATTTCCATT |
| 1,56 | 2,7E-09 | CR936613 | KIAA1109 | GTAACTTCTGAACCTCAGCATGTTACTCTAATAGTGTTTGGGATTGGCATGGTGAACCGC |
| 1,56 | 2,1E-09 | M_001012452 | FLJ32679 | AGAGAGATGCCATCAGAAAGTTTATCACCCTATAACAAAGCCAGGGGGCAGTGCCAAAGA |
| 1,56 | 3,8E-15 | AK022936 | AK022936 | GGTTGGAGTGAAGGTCTTATCTGCATTAATTTTGTTAATGCACATTGTAGATGTGGAGAA |
| 1,56 | 1,8E-10 | M13995 | BCL2 | ATCAGAGTTGTTGCTTCCCGGCGTCCCTACCTCCTCCTCTGGACAAAGCGTTCACTCCCA |
| 1,56 | 1,2E-04 | NM_013347 | RPA4 | AGAGAGAGACGGTGATGGATAAATTGACAACTCTGTAGGATTTACTAGCAAGCTAATGGA |
| 1,56 | 4,1E-11 | NM 007043 | KRR1 | GAGTTGGCAAAAGATTCTGAATTACGATCACAAAGTTGGGAGAGATTTTTGCCACAGTTC |
| 1,56 | 4,1E-13 | NM 014028 | OSTM1 | ACTGAAAATGTGCTGGGGTTTGTTCTGCTGTCACTGTTTATGCTGCTGGAACTTAGCACT |
| 1,56 | 2,5E-10 | NM_181659 | NCOA3 | GTCAGTCTGTCTCTTTTTCAGCTCAATTGTATCTGACCCTTCTTTAAGTTATGTGTGTGG |
| 1,56 | 1,4E-07 | ENST00000369697 | ENST00000369697 | ACTAGAGCAAAATTATGAGTGTCTAAGTATCTTAGTTATGCTTCATAAGATCTGACTGGG |
| 1,56 | 1,8E-12 | AK021804 | AK021804 | GGTATTGGAAGACTAGTGACTGTATTTAGTACCGGCTTTATATTTTACGATGTTCTTTCT |
| 1,56 | 2,4E-09 | NM_178862 | STT3B | GAATTGCCTTATGTATAAAATGTCATACTACAGATTTGGAGAAATGCAGCTGGATTTTCG |
| 1,56 | 2,1E-09 | NM_001778 | CD48 | CATCATGAGGGTGTTGAAAAAGACTGGGAATGAGCAAGAATGGAAGATCAAGCTGCAAGT |
| 1,56 | 3,6E-09 | NM_138290 | RPIB9 | TGAAATTTTATATTGTTCTGGTACATGTCTGAAATTCTATTGCTTGGAGAGAATCCCCTC |
| 1,56 | 5,8E-10 | NM_014716 | CENTB1 | GTTCACTTCTCCAAAGTCCGGTCTCTGACCCTTGACTCATGGGAGCCAGAACTAGTGAAG |
| 1,56 | 1,6E-12 | NM_024624 | SMC6 | TCCTTTCAGATGCCTGGATGAATTTGATGTCTACATGGATATGGTTAATAGGAGAATTGC |
| 1,56 | 3,6E-09 | NM_005126 | NR1 D2 | CTGCTTCTAAAGTTGCCAGATCTTCGATCTTTAAACAACATGCACTCTGAGGAGCTCTTG |
| 1,56 | 4,1E-12 | M_001004419 | CLEC2D | TTTCATGGTGCTAATATTACCTGTTCTCCCACTGCTAATGACATACCCGAGACTGAGTAA |
| 1,56 | 1,3E-07 | NM_000640 | IL13RA2 | ATACATGAAGACTTTCCATATCAAGAGACATGGTATTGACTCAACAGTTTCCAGTCATGG |
| 1,56 | 4,5E-13 | NM_012120 | CD2AP | CCAACCTTAGGTAAAACAAAAATATTGTAATCCTAGAAATTATCCTCCAGCTTTCTCACC |
| 1,56 | 1,4E-11 | NM_001673 | ASNS | GGACTGTGGGTAGATAGGGGAACAATGAGAGTCAACTCAGGCTAACTTGGGTTTGAAAAA |
| 1,55 | 4,4E-11 | BC067244 | BC067244 | CTTCCAAATCACTGGTTTGGGAGGGGGTGGATACATCCTCATTTTCTGTACATGATGCAT |
| 1,55 | 5,6E-11 | NM 004972 | JAK2 | GGATAACATGGCTGGATGAAAGAAATGACCTTCATTCTGAGACCAAAGTAGATTTACAGA |
| 1,55 | 4,4E-10 | NM_006846 | SPINK5 | ACATTTCCACCAAGTTTGAGCCCTCAAAATGTCCTGATTACAATGCTGTCTGTCCAACTG |
| 1,55 | 1,8E-12 | NM_003144 | SSR1 | TCCACACACTAAGCAGAAAACCAAGTTATCAGGACAGAGATATTTCCCAGTTACTCCTAA |
| 1,55 | 4,8E-09 | NM_000499 | CYP1A1 | GGTAAAACAGGGCCACATAGATGCTGATGGAGCCTTCCCAAGTTGTGCTTGAGCCAGGAG |
| 1,55 | 2,5E-10 | NM 003441 | ZNF141 | TAAAGAAGTGTGCAAAGCCTTCAATAGGTTCTCAACCCTTACTGTGCACAAGATAATTCA |
| 1,55 | 1,0E-10 | NM_175725 | IL5RA | AAACACTACAGAAGACAATTATTCACGTTTAAGGTCATACCAAGTTTCCCTTCACTGCAC |
| 1,55 | 1,2E-10 | NM_005625 | SDCBP | ACTGTCGCCTTAACTATGTTAAGCATCTAGACTAAAAGCCAAAATATAATTATTGCTGCC |
| 1,55 | 3,1E-04 | S71486 | S71486 | AAGTTGTATACAGTCTGCTCTTTCCTGGTTCTAAGAGAAGAGAGAGCTAAAGAAGAGGCC |
| 1,55 | 9,2E-10 | BX393727 | BX393727 | ATCAACATCCTCACAGACAATGAAAGCACACCGGAAGGACATGACCACAAGACAGATAAA |
| 1,55 | 1,7E-07 | THC2281706 | THC2281706 | CTGATGGTTCTCTTCCAAACAATGATCTAGAAAGATGCCCCGTGTCAAGTTGCTTGCCTT |
| 1,55 | 3,0E-06 | NM_021629 | GNB4 | CAATGTGACAGTGTTACATTTGGAGAGAAGTGTGAAATCTAACCAATCGCTAGCACATAT |
| 1,55 | 1,5E-08 | NM_002558 | P2RX1 | ACAACCCAGTGTACACACCACCTACGTGCACACAGCATCCTTCCACACTGTGTATGTGAA |
| 1,55 | 7,3E-11 | AB040942 | KIAA1509 | TACTGAGAAAGAAATGGGAGACTTTTGAATGTTAACAAGATCCATTTTAGGAGTGTTTGG |
| 1,55 | 8,3E-12 | AK094893 | LOC645431 | CGTATTTATTGTCAGCTCTTTAAACAAAAAGCACTCTATGAAGTGCTGTACTTTACAGTC |
| 1,55 | 7,1E-11 | NM_032505 | KBTBD8 | AAGTAAAAGGCACAATGGGTACTACAGAATTAAAATGTAGGTCTAACATAATGCCAGTTC |
| 1,55 | 6,9E-07 | ENST00000336749 | ENST00000336749 | TGTAAGTTCTGAATGGTTCCTTGCTGACTTTGGGTGACACATGTACCACATACTGGCTCA |
| 1,55 | 4,2E-13 | NM_032205 | PHF20L1 | AACCCATCCACTCTGGGCAGGTGACTGGAATAGCTGATTAAAACATAAATGCTGCTTTTA |
| 1,55 | 3,0E-12 | NM_182926 | KTN1 | ATGTTTTCACCCTTTCTACTTTGTCAGAAACACTGAACAGAGTTTTGTCTTTTCTAATCC |
| 1,55 | 6,4E-09 | M_016648 | LARP7 | TGGAGTTACTTCCCAAAAATGTTAATCACAGCTGGATTGAAAGAGTATTTGGGAAATGTG |
| 1,55 | 1,2E-08 | NM_005025 | SERPIN11 | AAGAGAGCCAATATGTGATGAAAATTGCCAATTCCTTGTTTGTGCAAAATGGATTTCATG |
| 1,54 | 1,3E-05 | BX112397 | BX112397 | TCTTAACCTAACCCTTAGTCATAAACTAGAACTCTGACCCTTTTCTAGTGAAGAGCTTTT |
| 1,54 | 1,2E-12 | NM_014673 | KIAA0103 | ACCCAGTCTTAAGGTTTCAAAAACTCTTTGACATTAGATTTCACAACTGCACAATTGAAC |
| 1,54 | 5,2E-09 | NM_080423 | PTPN2 | TGGGTCTTATCTCAGACCCCAGATCAACTGAGATTCTCATACATGGCTATAACAGAAGGA |
| 1,54 | 7,1E-12 | ENST00000381298 | ENST00000381298 | ATTGACCAAGTACATCAAGCAGGAGAGCTCTTCCTTCATTCTGTTATAGTCCACATCATT |
| 1,54 | 1,2E-09 | NM 006164 | NFE2L2 | CAGTAGTTTCACTTTAACTGTAAACAATTTCTTAGGACACCATTTGGGCTAGTTTCTGTG |
| 1,54 | 6,4E-10 | BC043564 | KCNA2 | CTACATTGCACAGGATGTATTGATATTATCATTCTTGCTGACATATTCTGGAAGCTTTCA |
| 1,54 | 5,9E-10 | NM_015633 | FGFR10P2 | CCACCAGATACAGAAATGTGCTTTAACATCAGTTGAAACCTAAATTTTCTTATGTTGTGG |
| 1,54 | 8,5E-10 | NM_174912 | AMDD | CCAGCAGACAAGCAGAGAAACAACTGGGGAATTTATTGACTCATTTAGTTATTCTTTCTA |
| 1,54 | 4,7E-12 | NM_145243 | OMA1 | GTGTAATTGTCCACCACTGTCTAATCCAGACCCTCGATTACTATTCAAACTCAGCACGAA |
| 1,54 | 5,5E-07 | BC002521 | ATRX | AAATCCAAGTCTTCAGGATCGTCACGATCAAAGAGGAAACCTTCAATTGTAAACAAAAAT |
| 1,54 | 8,3E-07 | M_018221 | MOBK1B | TTCAGAGCTGTGATTTTTGCAAAGTATTTTACCAACCTCCTCGATGGCTTTGATAAAGGT |
| 1,54 | 2,5E-10 | NM_145258 | LRRC44 | TCAGGAGTATTTGCTTGGGGTCATAGTTAAAGCCTAGTTCTCTCAGGTAAATACAGGGAT |
| 1,54 | 1,3E-12 | M_016824 | ADD3 | GGTGAGTTGGCATGCAAAAAATTACATTGATTACAGTGTGTTTTGGAGCTGGCTCTGTTT |
| 1,54 | 2,0E-10 | NM_018976 | SLC38A2 | ACAAAACCACGAGTCTCGGGTTAAGGGAAGTGACAATTTTATTCCATTCCAGAGAATGGA |
| 1,54 | 5,0E-04 | NM_030641 | APOL6 | GTCTGTGGTGAGCAAAGTTTGCCTTATTACACTGATAAAGTGTAATTACACTAATAAAGC |
| 1,54 | 9,6E-12 | M_001008388 | ZCD2 | ATTTGTGTCTTACTAAAGCAGCTTATTGTAGGTGTTGGCGTTCTAAAACGTTTCCTGCCT |
| 1,54 | 1,5E-04 | NM_000958 | PTGER4 | CCCAAACGTGGTTACATTAGCCATTCATGTATGTCAGAAGTGCAGAATTGGGGCACTTAA |
| 1,54 | 6,2E-08 | NM_014294 | TRAM1 | GTTCTTTTCAGTGTTCACATCTTGTTCCCCATTTCTCACTTGTGTCACCAGCTGTTTGTG |
| 1,54 | 1,2E-11 | NR_002819 | MALAT1 | TATTCATATTTACACGAGAACCTAATATAACTGCCTTGTCTTTTTCAGGTAATAGCCTGC |
| 1,54 | 4,8E-12 | NM_001568 | EIF3S6 | AAAGCTGAGGGAATGTGAATCAGTGCTTGTGAATGACTTCTTCTTGGTGGCTTGTCTTGA |
| 1,54 | 1,5E-07 | NM_001797 | CDH11 | AACTAATACGTGCCAGATATAACTGTCTTGTTTCAGTGAGAGACGCCCTATTTCTATGTC |
| 1,54 | 6,6E-12 | NM_015446 | AHCTF1 | TTGCCGGACCTTTCTGAACCAAACAATGAGCCTTTATTTTCTCCAGCGTCAGAAGTTCCA |
| 1,54 | 9,0E-09 | ENST00000361972 | ENST00000361972 | ATTGTGCTACTTTTGACCCATGTAATTTACCTAAAAGTTGTAATTGCTGACAGAGTACTG |
| 1,53 | 4,1E-10 | NM_001465 | FYB | CCTGCTCCTCCTAAACAATTGGACATGGGAGATGAAGTTTACGATGATGTGGATACCTCT |
| 1,53 | 7,2E-12 | NM_180989 | GPR180 | ACATTAGAGCTACAGGTTAAGACTAGAAAGTCTGAGTTATGTTTTAGGTATACATGATCG |
| 1,53 | 1,6E-06 | NM_002185 | IL7R | CATCCTGCTTCTACCATGTGGATTTGGTCACAAGGTTTAAGGTGACCCAATGATTCAGCT |
| 1,53 | 1,7E-06 | THC2439806 | THC2439806 | CATTAGGGAAAGTTTCGTGGGTGCATTAGAGTTCAAGTTTGTTGGCTCATTGTAGGACTG |
| 1,53 | 6,3E-08 | M_001003799 | TARP | ACAGATGTCATCACAATGGATCCCAAAGACAATTGTTCAAAAGATGCAAATGATACACTA |
| 1,53 | 1,6E-07 | NM_024498 | ZNF117 | AAACCTTACAAATGTAAAGAATGTGGCAAAGCCTTTAACCAGACCTCACACCTTATTAGA |
| 1,53 | 1,4E-06 | NM_133462 | TTC14 | TTCAGAGTCTTCTCGCAGTTCCAGAAGGCATTCATCTAGGGCATCCTCAAATCAGATAGA |
| 1,53 | 9,6E-08 | NM_207585 | IFNAR2 | TATGGGGACCATAGTATCATTCAGTGCATTGTTTACATATTCAAAGTGGTGCACTTTGAA |
| 1,53 | 3,1E-11 | NM_021105 | PLSCR1 | GTTTAGCTCTTACACTCTATCCTTCCTAGAAAATGGTAATTGAGATTACTCAGATATTAA |
| 1,53 | 3,2E-04 | M_001008224 | UACA | CAGACTCACAGTGTACTTCCCCAGGTATACCAGCCCATATGCAAAGCAGATCTATGTTAA |
| 1,53 | 8,9E-09 | NM_002519 | NPAT | TGCCATTAGCCGGCATACCACCATAAGAGAAACTCAATCAGAAAAGAAAGTTTCTCCAAC |
| 1,53 | 2,1E-09 | NM_138810 | TAGAP | TGCTTTTGTCATAGTTCCACTCTCTCAGATACATGTATCTAATGAAACTGAATAAATCCG |
| 1,53 | 5,2E-09 | AL355687 | AL355687 | ATATATATCTTTAAGAACTCGCTGTGTTAGGGAAACTGTTTACATATTGAGGTGTTCCTC |
| 1,53 | 8,5E-05 | NM_002666 | PLIN | AAGGGAACAATCAGATGCAAAAGCTCTTGGGTGTTTATTTAAAATACTAGTGTCACTTTC |
| 1,53 | 3,5E-09 | THC2403056 | THC2403056 | AGTTGAAATAGACATCTTTTCATGAACTCTGTAATATTTGAAATTATTGATAACTCTTAC |
| 1,53 | 2,2E-08 | A_32_P122595 | A_32_P122595 | TGAGCTAGTATGAATTTAATTTTTTAGAATGCGACGATGTCATCAGTTCTTAAGTAGGCC |
| 1,53 | 2,5E-12 | M_001023571 | IQCB1 | GTGTGGCTTTTATTGTAGAACTAAACTTAGCATAGTGTTCTGTTGTTTACATGAAGTGTG |
| 1,53 | 3,7E-10 | NM_005732 | RAD50 | TTGCACATGCTCTGGTTGAGATAATAAAAAGTCGCTCACAGCAGCGTAACTTCCAGCTTC |
| 1,53 | 3,4E-12 | NM_015423 | AASDHPPT | CGTATTCCTCTTAACGTGAACCGTCTGTTCATTGTTTTTACCTGTTTTCGTTTTAGCAAG |
| 1,53 | 5,6E-06 | BC039414 | BC039414 | AATGTTCAAAATGAGAAGACAATAACTGACAAGTCTGTGATTTTTGGGTTAGATGAGACC |
| 1,53 | 5,3E-09 | NM_001715 | BLK | TCGCACGGTCATCCGGAGTACTAAGCCCCAGTAAGGTGTTCAGGACTGGTAAGCGACTGT |
| 1,53 | 9,0E-10 | ENST00000360548 | ENST00000360548 | TGTCATTCTGTCAATGTTCATGACAAATTATCTGGAACTATACAGATTACATGGCAGTAG |
| 1,53 | 1,7E-11 | NM_177531 | PKHD1L1 | TATAAGATTGAATTTATACTGGATAATGTTGTTGGGGTAGAATCCAGAACTTTCAGCCTG |
| 1,53 | 1,2E-06 | NM_000237 | LPL | AACAGTCGATCAAGGGATGTATTGGAACATGTCGGAGTAGAAATTGTTCCTGATGTGCCA |
| 1,53 | 8,9E-12 | THC2270231 | THC2270231 | GAATTTTGTTACACTGGAGGAAGGGGAGACCTTGAGGAATATGGTCAGGATCTTCTCCAT |
| 1,53 | 2,6E-11 | NM_025228 | TRAF31 P3 | GGGTACATTACAGCTTGGGTTTTCCAACTGACTTAGGATTTCTGACTTTTTATTAATTTC |
| 1,53 | 1,9E-07 | NM_015384 | NIPBL | GAGAGCACCATAGTTGAGCCTAAACAAAATGAAAATAGACTGTCTGACACAAAACCAAAT |
| 1,53 | 1,1E-04 | ENST00000320876 | ENST00000320876 | CTACTTCTCGTAACATTGGGGTTAGAAGAATACAGATCAAATTGCTTTTTGATGAAACAC |
| 1,53 | 1,2E-11 | NM_018659 | CYTL1 | TAGTTGTATCTGAAACTGTTATGTATCTCTCTACCTTCTGGAAAACAGGGCTGGTATTCC |
| 1,53 | 8,5E-12 | NM_022763 | FNDC3B | TGGTGACAGTTTAGTGCCTCTGCCCATTGTCCATGATTTACACTAATTGTGAGCAGTCTT |
| 1,53 | 1,2E-10 | NM_004049 | BCL2A1 | TGTAACCATATTTGCATTTGAAGGTATTCTCATCAAGAAACTTCTACGACAGCAAATTGC |
| 1,53 | 2,7E-10 | NM_015364 | LY96 | TGAAGCTATTTCTGGGAGCCCAGAAGAAATGCTCTTTTGCTTGGAGTTTGTCATCCTACA |
| 1,53 | 3,0E-04 | NM_015180 | SYNE2 | AATCTGGGAGGCAGAAGCCAAATCTGTTTTGGATCAAGATGATGTGGACACCTCAATGGA |
| 1,53 | 2,3E-08 | NM_014857 | RABGAP1L | GCATTCTGTGTTTTGGTGAAAATCATGTACGACTATGGTTTGAGAGACCTCTACAGAAAC |
| 1,52 | 5,6E-07 | NM_175861 | TMTC1 | ACCAGACTAAGTGCCAGTATATATATGACTGATATTTTCGTGACTCATAGAAGGTGTCCA |
| 1,52 | 2,4E-10 | NM_005018 | PDCD1 | GCTGGGACAAGGGATCCCCCTTCCCTGTGGTTCTATTATATTATAATTATAATTAAATAT |
| 1,52 | 1,2E-09 | NM_001167 | BIRC4 | AGAGGAGAAGCTTTGCAAAATCTGTATGGATAGAAATATTGCTATCGTTTTTGTTCCTTG |
| 1,52 | 2,4E-10 | NM_014618 | DBC1 | GGAAATGAAAACGTTCTAGCAACTGTATAAAAGCGTTGGGCATGTTTGTTATTTCTATAC |
| 1,52 | 4,7E-10 | NM_024756 | MMRN2 | TGTGCCCCCACCTAAAATATCATCTTGAATTGTAATCCCTATAATCCCCACATCAAGGGA |
| 1,52 | 4,9E-10 | NM_006716 | DBF4 | AGACTAGTGAAGAGAAATCAGAATTTTTGGGTTTCACAAGCTACACAGAAAAGAGTGGTA |
| 1,52 | 6,5E-12 | NM_002830 | PTPN4 | CAGTGCTGGAATCGGAAGAACTGGGGTTCTTATTACTATGGAAACAGCCATGTGTCTCAT |
| 1,52 | 6,6E-11 | NM_207578 | PRKACB | GCACCTTAGAAGAATGACACCAGAAAACCTTATTATATCACAATATTCTGTTTTCCCCTT |
| 1,52 | 8,5E-07 | NM_014298 | QPRT | CTGTCAGGGCTGACTTCACCTCTGCTCATCTCAGTTTCCTAATCTGTAAAATGGGTCTAA |
| 1,52 | 8,9E-05 | BC062780 | BC062780 | AGATGGGAGAACAGAGGAGTAGTGACAGGAGTAGAAGCACTGTAGACCTTTTCATATCAT |
| 1,52 | 3,5E-11 | NM_024312 | GNPTAB | GCAACTGGAACATGGAGACATCACTTTGAAAGGATACAATTTGTCCAAGTCAGCCTTGCT |
| 1,52 | 1,9E-08 | NM_001117 | ADCYAP1 | GTAAATTTTAGAAACCCCTCTGTAGCCACTAGTAAGTAATTATGCACTAAATATGAACCC |
| 1,52 | 8,9E-12 | NM_004346 | CASP3 | TGCACCAAGTCTCACTGGCTGTCAGTATGACATTTCACGGGAGATTTCTTGTTGCTCAAA |
| 1,52 | 5,9E-09 | NM_032883 | C20orf100 | AAGTTACAGCTTATCCTACCGTGTGTGTGGTTTTGGGGTTTCGTTTGGGTTTGGGTTCTT |
| 1,52 | 3,3E-13 | NM_138459 | C6orf68 | TGAGTTCACCTGCTAGTACAATCTCCACAACTTGAATGGCATTGGTTGTTCTATAATTCC |
| 1,52 | 7,1E-12 | NM_002240 | KCNJ6 | GTACAATACCAGATCACTCAAAAAGGTGTGTCAAAGATTTTACCTGGGATATGACAAGCA |
| 1,52 | 8,4E-13 | NM_016265 | ZNF12 | GTGTTGGACTCATAATCTGAAGACTCACAGAATGGAAACCATGATTATAACAAGACCACA |
| 1,52 | 2,0E-13 | NM_005168 | RND3 | CTGTTTATTGTTGGCGCTTGTTGAAAACGAGCTTTCTTTCCCATGATAGTGCTTCGTTTT |
| 1,52 | 2,4E-09 | AL161993 | ASPHD2 | AGACCAGTTTGAGGCACTCACTCTAGAAATAGCCTGTGTTAGCTGATGTGTGAAAGCGTA |
| 1,52 | 1,1E-08 | NM_014395 | DAPP1 | AGAAGAATGAGATACTGATGTCCACAGTTCATTGGCAGAATCTAACCCCTTCTGTTATCT |
| 1,52 | 6,2E-12 | M_017699 | SIDT1 | TTTAAAGTCCCCAAATGTGTACTTAGCCTTCTGTTATTCCTTATTCTTTAAGCAGTGTTG |
| 1,52 | 3,9E-07 | AK074562 | AK074562 | TGAAAGTCATAGAAACAACTTAAAAGTAGAAAATGGTTAGGTGTTACGGCTTTCTCGTCG |
| 1,52 | 2,5E-11 | NM_020123 | TM9SF3 | GAAGACAGAAGTGTACTCTCTACAATGCAATTTACTGTACAGTTAGAAAGCAAAGTGTTA |
| 1,52 | 6,5E-08 | NM_206855 | QKI | AAGCTGTTGAATGAGTCTTAAAAATTATACTACTGTTAAGTGGACCAAGTTTGGTGAAGC |
| 1,52 | 2,2E-07 | M_018017 | C10orf118 | CAGCAAGCACGAAGAAAATTAGATCAGGTTGAGAGTGGAAGCTATGACAAAGAAGTCAGC |
| 1,52 | 2,4E-09 | NM_138467 | TYW3 | TTCATGTGTACACTTTATAGGAGAATAAGCAAGAGCTTAGGTAAGTTATAGTCCTTACCA |
| 1,52 | 3,4E-10 | BX537651 | BX537651 | GCTACAATTAGCTGCATTACTTGGTGCCAAAGAGCAGTGGGGAATTGTTGAGTTGCTGTA |
| 1,52 | 1,3E-07 | ENST00000264903 | ENST00000264903 | TGTGGTATGTTTAGGATGCCCACGGAATGAATTTTTCCTTATTCCAATTCAACCTTCTGT |
| 1,52 | 1,9E-04 | NM_004079 | CTSS | TCTGTTGGTGTAGATGCGCGTCATCCTTCTTTCTTCCTCTACAGAAGTGGTGTCTACTAT |
| 1,52 | 1,4E-07 | ENST00000377047 | ENST00000377047 | CTTGCTATATCATGGTTTGCTATGGCACAGACTCAGCTTAGCATGGGGACATTTTGAACT |
| 1,51 | 1,5E-05 | NM_015070 | ZC3H13 | CCAAGAGATGGACATGATGAAAGAAAATCAAAGAAGCGCTATAGAAATGAAGGGAGTCCC |
| 1,51 | 5,2E-09 | NM_152475 | ZNF417 | AAGATGCTTCATGGATACAAGAGTTAAAACTTCCTGTCCCATTTAGTGAAACAAAAGGTT |
| 1,51 | 7,6E-09 | NM_170672 | RASGRP3 | CATTAAGGCAAAGTAGTTCCAGTGATTTAAAATACGGTTCCAAATACGCTAAAACCAACT |
| 1,51 | 5,9E-11 | XM_496707 | FLJ12993 | AACAAGATTCTCCTGTCAAAAAGGCGTCTTACTGTAAATAGTCCAAGGTGACATTTGTTA |
| 1,51 | 1,5E-07 | NM_001289 | CLIC2 | GAGAGTGAGCATATCAGAGAGGCAAATTCTTAAAGAATGATTTTTAAAATCAGCTCTAGG |
| 1,51 | 6,2E-12 | NM_006777 | ZBTB33 | ATAAGTTTCCATATCCAAAGTTCAGAATTCATGTGAAATACTTCTTTGGGGCAAAAGTCC |
| 1,51 | 5,9E-11 | NM_152295 | TARS | CTGCAGGCGTAACTATTTTTGACCTAGTCAGTTTTTAAACAATGTGCATTTGAAGGAGTT |
| 1,51 | 8,1E-12 | NM_100264 | WAC | CATCAAGATTACGCGAAGAAGCGCATAACATGGGAACTATTCACATGTCCGAAATTTGTA |
| 1,51 | 1,4E-09 | NM_006506 | RASA2 | AAGAATTTATTCCCTTTTTACCCTCAGTTTACTTAAGCTGCAGAAGATGGAAGAGGCTTG |
| 1,51 | 6,9E-12 | CR749561 | ALDH1L2 | TCAAGGAAAATCTGGGCTAAGAGTAGGATATGAGGGATGATGGATAAGGCATGAGACATG |
| 1,51 | 1,5E-10 | NM_018429 | BDP1 | AAAGGTGGCAGAATTGAAACAAACTGGAAAAACAGACATTTCTCCAAGGGAAAACGAGCT |
| 1,51 | 1,2E-08 | XM_208438 | LOC286495 | TTTTCTCTTGGGATGTCCCCGTTTTGTTGTGATTGACAACTGTATTGCATTGAAGAAAGT |
| 1,51 | 7,3E-07 | BC015929 | NR1D2 | TTTAATGTATCTCTTTACATGATCTGAGAGAGGATTCAAGTTGATAGAAATAGCTGAGGG |
| 1,51 | 3,8E-08 | AK123297 | AK123297 | TGCAATATGGAGGAGAAATGATTAGACCTTAGGAAGTGCCAGTGGGTTGGTCCTTTCATG |
| 1,51 | 2,1E-07 | AK021543 | AK021543 | AGTTTGTACCCTTTTGATTCACGGCATGGCCCTATAGTTAATTAAGAGGTGGATAACTTA |
| 1,51 | 8,9E-12 | AK026687 | AK026687 | GTGAGGTTCTATCTGAAAATGTTATTTAGCTATCTTCTGGGACTATTTAATGAAAGTGGG |
| 1,51 | 8,3E-04 | NM_178544 | ZNF546 | ATAGCATCACTCAGTCCCTGTTAGACTTTAGAAGATTGATACTGATGCACTGCATCCCAA |
| 1,51 | 4,8E-05 | AF116677 | AF116677 | CTTTGGCTCATAGAATACTTATCTGATTAAAGAAAGCACATGAATTTGCATGGAGTACAG |
| 1,51 | 9,4E-12 | AK128714 | AK128714 | TTAGAGAAGCCCCCATACCTGGTAGAGCATGTACCATCTTACATGCTTAAATAACTCCAC |
| 1,51 | 4,7E-07 | THC2443199 | THC2443199 | GAATAAGAATTAAACTGAATGTGCATGTCATCATACTTCTGAAACTGACTTTGGTCAGAG |
| 1,51 | 2,3E-07 | NM_016218 | POLK | TGTGAAAGAAGACAGCTTAATAGTAGTTAGCTTAAGTAGTTTCTCCAAGTACTTTTGTGC |
| 1,51 | 8,5E-05 | NM_020801 | ARRDC3 | AGCAAAACAAAGTGCAATATTAAATGTTTGCTTTATAGATTATATTCTATGGCTGTTTGT |
| 1,51 | 5,0E-08 | NM_020711 | KIAA1189 | TGAAAGCCACAGAGTTAAGTGACCTCAGGTAACATAATGGTGATGGTTGGCCATTTGAGT |
| 1,51 | 1,9E-07 | AK093571 | SCML4 | CCACACAGAAACAGGGGATTTATTCCACAGTTAGCACCACAGATTGCGACTTGGGAAAAA |
| 1,51 | 1,2E-11 | M_018981 | DNAJC10 | TTGGGTGATTGATTTCTATGCTCCTTGGTGTGGACCTTGCCAGAATTTTGCTCCAGAATT |
| 1,51 | 3,8E-09 | NM_014810 | CEP350 | CTGTAGTCTTGTAGCCATAATGAGCAAATTGACTAAGAGAAGCAAAGGTTTCTTGGGGTT |
| 1,51 | 1,2E-09 | NM_173354 | SNF1LK | CCAACACAAAACTCCTTCCCCTTTTTAAAATGATTTCTGTTCTAATGCCATAGATCAAAG |
| 1,51 | 6,7E-07 | NM_003638 | ITGA8 | CCCATTATGGGTAATAATACTAGCAATACTTCTTGGATTGTTGGTTCTCGCCATTTTAAC |
| 1,51 | 4,1E-08 | THC2432207 | THC2432207 | CTTTAGGAAGCGGAAGACTAATGACCGAAGCTTCAAGACTTTGACATGTGTGAAAACATG |
| 1,51 | 1,6E-05 | NM_000165 | GJA1 | AGTACTGTAAACAGGCTTTAGTCATTAATGTGAGAGACTTAGAAAAAATGCTTAGAGTGG |
| 1,51 | 1,1E-08 | NM_177438 | DICER1 | CGGTCGCTGTTGCACCTGATAGTAAGGGACAGATTTTTAATGAACATTGGCTGGCATGTT |
| 1,51 | 6,3E-12 | NM_152729 | NT5DC1 | GTGGTGTTCTATGTGCCGTGTATGGTCATATTAGTAGCTATTTGTTGCAAGATACTTGAA |
| 1,51 | 2,2E-09 | NM_172003 | CBWD2 | GAAGAGAAACCTGATGGCCTTATCAATGAAGCTACTAGACAAGTTGCTTTGGCAGATGCC |
| 1,50 | 5,6E-04 | NM_001001971 | FAM13C1 | TATATCATTTTGTGTTATTTTATACGAAAACATGTTTATTATTTTCATTTTTGTAAAAGG |
| 1,50 | 8,0E-10 | AB011103 | KIF5C | TGGTGGTTTATATCAATAACGATGCTGTACTATAGTCCATGTAACAAAAGATCTGGAAGT |
| 1,50 | 1,1E-09 | NM_198329 | UBE1DC1 | AAAGCAAGAAGATTCTGTCACTGAGTTAACAGTGGAAGATTCTGGTGAAAGCTTGGAAGA |
| 1,50 | 2,0E-08 | NM_006059 | LAMC3 | CTGTGAACTCGACCCCGTGTGGATAGTCACACTCCCTGCCGATTCTGTCTGTGGCTTCTT |
| 1,50 | 1,2E-08 | NM_015393 | DKFZP56400823 | CACAGCCATGGTTTCCATTTCTAGATGAAAGGATGGCCTAGGACATAGGTCTCAAAGACT |
| 1,50 | 2,5E-10 | NM_030762 | BHLHB3 | ACAACAGTCTGACTATGAGTGAGGAAAATATCTGGGTCTTTTCGTCAGTTTGGTGCATTT |
| 1,50 | 2,4E-08 | NM_014648 | DZIP3 | GGATGTTATCCTGCTATCAACAAGGAATTGCTCTACAGTCAATAACAGGCAGTCAGCGTA |
| 1,50 | 8,0E-10 | THC2407737 | THC2407737 | AAGAAGGGACAGTTACACAGTAATTGGGAAAACTTCTGCAAGGACAGATGTGCATTTCTC |
| 1,50 | 1,0E-09 | AJ227863 | AJ227863 | GGTTTCCATGCCATTAACCGCTAGATCATTTGCATTCTCTCACAGAGTAAACGGAAAAAA |
| 1,50 | 1,5E-08 | NM_031292 | PUS7L | GGGCTTCAGTTTCAACATCTGTAAAATGGGCATGTTAACATTGCCTACCTCATAGGATTA |
| 1,50 | 2,3E-09 | NM_014450 | SIT1 | GCCAAGTCACAGGATGTGATCTACCCCGGACTTCCTATCTGAGCTTCAAGGGAGACATCT |
| 1,50 | 2,2E-13 | NM_022365 | DNAJC1 | GCTCGGTCTGCAGAGGAGCCGTGGACTCAAAATCAACAGAAACTTCTGGAACTGGCGTTG |
| 1,50 | 8,6E-09 | AK057962 | AK057962 | TCAGGAGCCTTGTTTTAAGACAGCCTGGCTGTCTGAGAGCCACCGGAGTCGAGACTGGAT |
| 1,50 | 6,7E-08 | NM_005356 | LCK | CCCACCCACATGTGACACATATGCACCTTGTGTCTGTACACGTGTCCTGTAGTTGCGTGG |
| 1,50 | 1,4E-07 | A_24_P473972 | A_24_P473972 | AATGCTGAATTAGGCAAAACCAATATTTAGTAGTAAATCCATTCCTGGTAGTATAAATCA |
| 1,50 | 3,0E-11 | NM_007114 | TMF1 | TCACTCATTTACAGCTAGAAATTGGCAATCTAGAAAAAACTCGATCAATAATGGCTGAAG |
| 1,50 | 2,5E-09 | THC2405019 | THC2405019 | TCCAAAATGGAAGTTGTAGACATTATCTGTAGTTTATGCACAACAATAAATTAGAAAGCC |
| 1,50 | 4,0E-09 | NM_016931 | NOX4 | AGTGCAATTTCTAAGACTTTGAAACTCAGCGGAATCAATCAGCTGTGTTATGCCAAAGAA |
| 1,50 | 1,1E-11 | THC2363646 | THC2363646 | GGGACTATGGGTAGATAGGGGAACAATGAAAGTCAACTCAGGCTAACTTGAATGTGAAAA |
| 1,50 | 8,9E-11 | NM_007315 | STAT1 | TAAAGTCAGTGCCCAACTGTTATAGGTTGTTGGATAAATCAGTGGTTATTTAGGGAACTG |
| 1,50 | 2,6E-12 | NM_148170 | CTSC | CACTCCCTTGGAAAACAGTAAACATCATTTTGGAATGTGAACAACCAGAGACTACACAGG |
| 1,50 | 8,4E-11 | NM_006010 | ARMET | GGATTCCCTTCCTTCTGTTGCTGGTGTACTCTAGGACTTCAAAGTGTGTCTGGGATTTTT |
| 1,50 | 2,4E-10 | ENST00000264777 | ENST00000264777 | GTGGGGAACCCTTACCTAAGTATGTGGATTGACAAATCATGATCTGTACCTAAGCTCAGT |
| 1,50 | 6,9E-09 | NM_014819 | PJA2 | GTTTGTTTCCTTTTAAGTACTGTTGATCAGTTGTGACACTTACTGGTTAAACTTACGTTG |
| 1,50 | 5,3E-09 | THC2283371 | THC2283371 | TTAGAAGTGAAGCAACTTGAACTCACTCTCCGAATGTACAAAATGTGGAATTGAAGACTG |
| 1,50 | 4,7E-13 | NM_000321 | RB1 | GTGCCAGAATTTTAGGAACTTCAGAGATCGTGTATTGAGATTTCTTAAATAATGCTTCAG |
| 1,50 | 1,4E-07 | NM_003437 | ZNF136 | CAAAAGAGAAAAGCTCTTGAATATAAGCAATGTCCACACGTTTTTAGCCAGCCCACATGC |
| 1,50 | 2,3E-05 | NM_001014975 | CFH | AATGGAACCAAGTTTGAGTCTTGCCAGGTCAATACTTGGGTCCTGAGTATGGTGACTAGT |
| 1,50 | 1,6E-10 | NM_052847 | GNG7 | CCCTGCCTCGGAGAACCCCTTTAAGGACAAGAAACCTTGTATTATTTTATAACTGTGTTC |
| 1,50 | 2,3E-12 | NM_005791 | MPHOSPH10 | ATTAAAGTTGTGTCAAATCTGCCAGCCATAACCATGGAGGAAGTAGCCCCAGTGAGTGTT |
| 1,50 | 3,7E-09 | AK026485 | AK026485 | TAATCAAATGGTAGGATAGGATATTAATCACTGTAGCTTAAAGCTAAGTTTCTCCCAAGG |
| -1,50 | 6,0E-09 | NM_001101 | ACTB | GCACCCAGCACAATGAAGATCAAGATCATTGCTCCTCCTGAGCGCAAGTACTCCGTGTGG |
| -1,50 | 6,1E-05 | NM_031449 | DKFZp76112123 | TGGGTGGGCTCTCAGATTCAGCTCTGTGTAAAGATTCTCTAGCGGGCTGCGCTCCCAAGT |
| -1,50 | 9,6E-10 | A_32_P208713 | A_32_P208713 | GTCAGGTGTGCTGGCCCTCAATGACCAACACAACAAAGATAAATTTATTGTTTTCTGAGA |
| -1,50 | 3,6E-08 | ENST00000371244 | ENST00000371244 | CTGATAGGATGAGTATCTTGATTTTTAGGTAAGATCACAGTTTCACAAATTGTGGTGTTG |
| -1,50 | 3,1E-09 | NM_020724 | RNF150 | CAATAGCAACAGAAATATAAATTATATTCCATTCCCAGAGAGAGAATGCGCTTTGGATTG |
| -1,50 | 5,1E-06 | NM_033315 | RASL10B | TGGGGGAAGGGTCGTGGGTGGGGAATTTATCACCAACATCCATTGTAGGGGGAATCTATG |
| -1,50 | 5,4E-09 | NM_024110 | CARD14 | GCAGAGGCATAAGCCCACATATGCTGTGACGCTGGCCACCTTTTCTCAGCTTCTGAGGCT |
| -1,50 | 3,4E-06 | NM_152335 | C15orf27 | TCTCATTCCAGCATGAGCGTTTCTGAGTCTCTTCAAGACGAATCTAGTTTTCACCTTCAC |
| -1,50 | 3,2E-12 | NM_020888 | KIAA1522 | GGTAAATGAGCTTGACCTAGAGTAAATGGAGAGACCAAAAGCCTCTGATTTTTAATTTCC |
| -1,50 | 2,0E-09 | NM_018059 | FLJ10324 | TAGGTTCTATTTATATGGCAGATGACGTGAAATTGTGATGTTTGTTACAGAGCTTTTATG |
| -1,50 | 1,6E-08 | NM_003564 | TAGLN2 | CTGGGAGTCACTGATGCTGCCTCTGCCTTCTGATGCTGGACTGGCCTTGCTTCTACAAGT |
| -1,50 | 1,5E-12 | AK026765 | C6orf59 | TTTTCTGGCAGTGTAAGGTCTACACCATTTTCCTCTCAGCATCAGAGAAGGCAGAAAGCA |
| -1,50 | 1,2E-09 | NM_005231 | CTTN | TTTTGAAGGTGGGGAGGGGAATATACACATTGCTTTTATATTTAATACTTTTGCTGATGC |
| -1,50 | 1,7E-09 | NM_003722 | TP73L | TCTGTTATGGGCTTTTGGGGAGCCAGAAGCCAATCTACAATCTCTTTTTGTTTGCCAGGA |
| -1,50 | 7,2E-09 | NM_004104 | FASN | ACGGAGGCCATATGCTTCTTCGTGCAGCAGTTCACGGACATGGAGCACAACAGGGTGCTG |
| -1,50 | 4,4E-10 | NM_138711 | PPARG | CCAAGGCTTCATGACAAGGGAGTTTCTAAAGAGCCTGCGAAAGCCTTTTGGTGACTTTAT |
| -1,50 | 4,8E-11 | NM_033452 | TRIM47 | CAGATCGGGCCCCTCAAGAAGTCCTGCATATCCGTGCTGAAGAGGAGGTGATGCCGGGCA |
| -1,50 | 1,2E-08 | BX640708 | LOC284454 | CACATGTGGGGCATGGAACAAGTTCCTTGTGGACCCAGAAGGGACACAAGCAGGTGTGCT |
| -1,50 | 3,2E-12 | NM_014567 | BCAR1 | CAGGAATCTGTATATATTTATGGCCGGGCAGGGTGTGGGGCCATGCCTCCTCAGGAGCCG |
| -1,50 | 1,6E-08 | NM_017451 | BAIAP2 | GGACTTTATCATCGGCAGACCTCAGAAGACAACACACAAAGGTTTCTTTTTTCTTAGCTT |
| -1,50 | 4,2E-08 | NM_032808 | LRRN6A | TTTCTCTGTAACTTGGGTTTCAATAATTATGGATTTTTATGAAAACTTGAAATAATAAAA |
| -1,50 | 2,2E-10 | NM_004265 | FADS2 | CTGATCGTAATGTTTATCATGTTACTTCCCCACCCCTACATTTTTTGAAATAAAATAAGG |
| -1,51 | 9,2E-04 | NM_014723 | SNPH | TAGATTTCTCCCAAGGGGATCACTTAGCTTCTCACTGACACACCCTTCCCAATTGCCACA |
| -1,51 | 5,2E-09 | NM_017982 | SUSD4 | CATCTCATGATGGAGACAGTAACCAAGGTAATGTTTTGTTTCAGCATTTTAAAAAGACTC |
| -1,51 | 2,7E-12 | NM_024927 | PLEKHH3 | GCAGCCTGGGCCAGGACTGCTCTGAGATTTGAGGGAAACATGGACCCTTTTGGCCCTGCA |
| -1,51 | 8,0E-10 | AB007877 | HSPA12A | TCTAAAATATCAGCTACTGGGGTCTCATTCACTACAAGATAAATTCAAACTTCACAGAAC |
| -1,51 | 2,4E-07 | NM_002627 | PFKP | GCCTGCATGTGCCTGCAGCCACCGTGGACTGTCTGTTTTTGTAACACTTAAGTTATTTTA |
| -1,51 | 1,3E-10 | NM_024307 | GDPD3 | CGGACCTCCTAAGTCCAGAAGCCTCGAGGTCTCCTGTTTCTCTTCCTGAAAAATAAATAT |
| -1,51 | 1,4E-05 | NM_019032 | ADAMTSL4 | CAAACGTGTATCACTTTTCAAAAAGAGGTTACACAGACTGAGAAGGACAAGACCTGTTTC |
| -1,51 | 3,0E-10 | AK054645 | RP11-138L21.1 | CTGCGGAGATAAGTCATTTTGGAATTCAGCTTCCTTCAACACTGAGACTTCATACCTTCA |
| -1,51 | 2,2E-11 | AK128234 | CEACAM19 | AAACAAAAACTCCCGCTTTCCCAGAGGGTCACTTGGCAGGGGAAAGAATCAGGAAATAAA |
| -1,51 | 2,2E-09 | NM_007098 | CLTCL1 | CATGTCACTCCAGCAGCACAGGGGACGCAATGGGAGGCAGGGACACCTGGACAATATTTA |
| -1,51 | 1,6E-07 | NM_001040272 | C9orf94 | GTTGGGTCTCCTTCTTCTTCACCACAAAAACAGGCTCTAAGAAATCATGTTACTAAAAAA |
| -1,51 | 2,8E-08 | NM_144669 | GLT1D1 | CAGGTCCAATGTTGTCTTAGATCTGATGATGCTGCTATTTACAAAACACTGATCGTCCGA |
| -1,52 | 8,7E-04 | NM_015444 | TMEM158 | AAATTGCTATTGCTGTAGTAAGAGAAGCTCTTTGTATCTGAACATAGTTGTATTTGAAAT |
| -1,52 | 3,4E-08 | NM_002246 | KCNK3 | GACTTCAGACTCACCATAATTGCTGATAATTACCCACTCTTAAATTTGTCGAGTGATTTT |
| -1,52 | 3,7E-13 | BC033222 | BAHCC1 | TCGGGGATCAGAACGATGTCAAGTGAAGAAAAATGCACGGGGGCATGCTGAGCCACTGGA |
| -1,52 | 2,5E-07 | AU185665 | AU185665 | GTTTTTGATTTGTCAGTTGCTTGGCTGAACTGATCAAGTCGATAGTTGCTTGACAAACTC |
| -1,52 | 1,2E-11 | AK024229 | FLJ14167 | GGAGAGTTTTCATGTTATTTTCAATACATAGCAGTATCTAAAGACGTAGGCGATGAGACT |
| -1,52 | 3,0E-09 | NM_002944 | ROS1 | GGAGATGGGTCTGATTAATAGCGTTGTTTGGGAAATAGAGAGTTGAGATAAACACTCTCA |
| -1,52 | 1,0E-05 | NM_001703 | BA12 | ATATATATATCTCTCTATTTTCACACTCCACTTTGGAACTACCCAGGAGCCAGCGCCCTC |
| -1,52 | 1,5E-06 | NM_000961 | PTGIS | TTGTTATGCCTTGCTATTTTAATAAAGATTCTATTTTCGTATAACATTGTCAAGTGGAAA |
| -1,52 | 1,5E-09 | NM_182645 | VGLL2 | AGACGCAAAGATACACTTTTTTCCTTTGGACTGTGAACATGGAAGCCTCAGCCTGAATGT |
| -1,52 | 3,8E-12 | NM_152264 | SLC39A13 | AAGTGGCCATCGAGAGGTCTGGATGGTTTTATAGCAACTTTGCTGTGATTCCGTTTGTAT |
| -1,52 | 1,1E-11 | ENST00000295989 | ENST00000295989 | GAAGGTTTCAAATGTGTCTAGTGTTCAGTATTGAGGACAAAGAAATACAAGTGGCAGGCC |
| -1,52 | 1,8E-09 | NM_001505 | GPR30 | AACAGCTGGGGACAACTGCGGTGATGATGTAAAAACCTTCCCATAAAATGTAAGAAAAGC |
| -1,52 | 3,5E-11 | NM_002998 | SDC2 | TACAGAACAAGATACCTGCTCAGACAAAGTCACCTGAAGAAACTGATAAAGAGAAAGTTC |
| -1,52 | 4,1E-09 | NM_002619 | PF4 | GACCTGCAAGCCCCGCTGTACAAGAAAATAATTAAGAAACTTTTGGAGAGTTAGCTACTA |
| -1,52 | 1,0E-06 | CR936831 | C80RFK32 | TTTAACTTCAATATGCTGTCTGGACATAGTGAGAACTAATGGCAATAAACGTCCAGCTGC |
| -1,52 | 3,0E-10 | NM_020299 | AKR1B10 | ATCACAGTGAACTTAGTCCTGTTATAGACGAGAATCGAGGTGCTGTTTTAGACATTTATT |
| -1,52 | 3,6E-12 | ENST00000339892 | ENST00000339892 | TGATCGAGGCCTGCCCGAACCCCAAGGCCAACAGAGAGAAGATGACTCAGATCATGTTTG |
| -1,52 | 1,5E-08 | NM_023107 | FGFR1 | GCATCTCTTACGAAGAGTTGGAAAAACAAATGCCATATATAAATTCTAAGCCATATGAGG |
| -1,53 | 3,5E-09 | NM_020707 | GUP1 | GACCGTGGCATCAACGACTGGCTTTGCAAATATGTGTATAACCACATTGGTGGGGAGCAT |
| -1,53 | 8,5E-09 | NM_004947 | DOCK3 | ATGCTGTGTATTTGTACAGGAATTTGAGCAAAAAATGTATAGAGTGTGATGTCCAATTGG |
| -1,53 | 1,3E-12 | BC052611 | PXN | CTTGCCTTGTTGAGGCTTTTTCCTTTCAGATGAGCTGTCCTTGACATTCTGATGTGGTGA |
| -1,53 | 2,4E-09 | NM_153344 | C6orf141 | AAATCTTATCCCGTTTACAAATGTCCTGGGAGTGCATCCAAAGTGAAACTTGGGTATCCT |
| -1,53 | 2,9E-04 | NM_173575 | STK32C | ATCTTCAAGACTGCCTCGATGCCATCCAGCAAGACTTCGTGATTTTTAACAGAGAAAAGC |
| -1,53 | 2,4E-08 | NM_002152 | HRC | CTATCTCTGCCCGCTGGTCTGCGAAACGGTCTGCGCTCCAGGAAGCTACGTTGACTATTT |
| -1,53 | 3,5E-12 | NM_018664 | SNFT | ATACCTGGGAGGAAGGCTTTTCCTTCACAATTGTATACAGGGGGCACCTGTGGCCAGGCC |
| -1,53 | 1,0E-06 | NM_201380 | PLEC1 | CAAGGGCAGAACACTAACCTGACCGTGGGCGGGGCCTTGCGGTATCCGCCCCCAATAAAA |
| -1,53 | 4,5E-07 | NM_053284 | WFIKKN1 | AAGCAGGCCTGCGAGCTGCTCAACCGCTTCCAGGACTAGCCCCCGCAGGGGCCTGCGCCA |
| -1,53 | 1,2E-08 | BC017937 | BC017937 | AAAGTCAGCCTCGCATTTGGAACCCATTTCCCTGAGTTCATTGCTGAATTCCAGAAGGAA |
| -1,53 | 8,8E-09 | NM_018125 | ARHGEF10L | GTGTGCGAGTGTGTGGGCTGGTGTGTGAATATCTATAAATAAGTATATATGGTGTATATT |
| -1,53 | 7,9E-11 | CR596712 | CR596712 | CATCTCTTGGTCTATGACGACGATGTTGGAGAGTTCCTACTCCGTGATAACTCAGTTCTA |
| -1,53 | 1,5E-12 | AK127004 | AK127004 | AAAGCAAATTGTTTGGTGTCTTTCTGTCCCACTACAGTATAGGCCCGGTTCAGACAGAGG |
| -1,53 | 1,5E-09 | NM_138440 | VASN | TATTCTGGGAAGATGTTTTTCAAACTCAGAGACAAGGACTTTGGTTTTTGTAAGACAAAC |
| -1,53 | 9,0E-06 | NM_198570 | UNQ739 | CTAGATGACTCTGGGAACTATCAGTCAAAGAAGACTTTTGATGAGGAATAATGGAAAATT |
| -1,53 | 1,1E-08 | NM_153370 | Pl16 | CTCCGGAAGGGAAAGGCTACGGGGCATGTGCCTCATCACACCATCCATCCTGGAGGCACA |
| -1,54 | 2,6E-09 | NM_013440 | PILRB | AGGTCTGAACTCCACTGAATTAAACCACTGGCATTTGGGGGCTGTTTATTATAGCAGTGC |
| -1,54 | 5,8E-08 | A_24_P247175 | A_24_P247175 | TGTCCACCTTCCAGCAGATATGGATCAGCAAGCAGGAGGAGTATGACAAGTCTGGCCCCT |
| -1,54 | 6,0E-07 | AK023086 | CNIH3 | ATACCTCTCAGTAAAGAAAGAAATAACAACTTCTATCTTGGGCGAGGCATTTCTTCTGTT |
| -1,54 | 1,6E-09 | NM_001825 | CKMT2 | GGTCGCAGATGTGTACGACATTTCCAACATAGATAGAATTGGTCGATCAGAGGTTGAGCT |
| -1,54 | 1,9E-08 | BC041879 | LOC390705 | TGCGGGGATGAGGACCTGGAGCCACTGTGACGCCACCCATGAGAACGCCGCTGCGGGCCG |
| -1,54 | 4,3E-11 | NM_003812 | ADAM23 | GCCTTGCACTGTTGGATTCTGGGTATGACATACTCGCAGCAGTGTTACTGGAACTATTAA |
| -1,54 | 5,8E-09 | NM_022359 | PDE4DIP | AAACAGCATCTGGATAAAACATGGGCTGATGTGGAGAATCTCAACAGTCAGAATGAAGCT |
| -1,54 | 5,2E-07 | BC022888 | CMYA3 | TTCCTAATGAAGAACCAAATATGTGTAAAAATATTGCAGAAAACACCCTTGTACCTGGAG |
| -1,54 | 2,0E-12 | NM_020836 | KIAA1446 | GCATGCCCTGCCAGTTAGTACTGTATTTTGCATTCATTAATAAAAGACACCGGTGGAAAG |
| -1,54 | 5,0E-13 | NM_024060 | AHNAK | GTGCATTTCTTTACTTGCAAGGGGACAGAGTGTGGGCTTAGGTTTGGGACTAGAGGGGGC |
| -1,54 | 4,4E-12 | NM_138809 | LOC134147 | AGAGAAGATTGCTCACCTGCAGACAAGCCCTACATTGACGAGGCCAGAAGGAATTTAATT |
| -1,54 | 1,7E-12 | NM_003383 | VLDLR | TCTAAACAAATAATACCCCCGTCGGAATGGTAACCGAGCCAGCAGCTGAAGTCTCTTTTT |
| -1,54 | 4,6E-10 | NM_006898 | HOXD3 | TGGTCATTTCTCAAAAACCACAGTCCTCACCACAGTTTATTGATTTCAAATTGTCTGGTA |
| -1,54 | 9,5E-11 | NM_023928 | AACS | CCAGCTTCCCTCCAAGGAATGAGTGGATTTCATACAGGATCTCTTTATTGCACAGACTGA |
| -1,54 | 1,0E-06 | AK094629 | AK094629 | AGCTATATTGTGCTCAAAGTATGTTACTGACATTTCTCCGTCGTTAAAAGGAACTGCTCC |
| -1,55 | 3,9E-11 | NM_198965 | PTHLH | AGCAGGAAAAGAAAAAACGGCGAACTCGCTCTGCCTGGTTAGACTCTGGAGTGACTGGGA |
| -1,55 | 1,3E-11 | AK024186 | OBSCN | CCTCGCTTCAGCCGGGCGATTCTTCCCCTCATTTGTTGCATTGTTTGCATTAATATGAAT |
| -1,55 | 9,5E-07 | THC2309459 | THC2309459 | GCGAGAGACATTTGACTCTTGTGTTTGTATCTCCTTTTTATGATTGCTGTACCTACCCAT |
| -1,55 | 1,5E-07 | NM_000156 | GAMT | CACCAGCCCTGGGCTGATCCCAGCTGTGTGTCACCAGAAGCTTTCCCGGCTTCTCTGTGA |
| -1,55 | 5,4E-12 | NM_004469 | FIGF | ATTCGTATGATCAGTACTGACTTTCTGATTACTGTCCAGCTTATAGTCTTCCAGTTTAAT |
| -1,55 | 2,8E-06 | BC013821 | BC013821 | GGACTTAGAGGAAACTCTGGGGCACAAGGAGGTGATGCCTGTCACTTGGACATGGGTGCA |
| -1,55 | 7,4E-12 | ENST00000361200 | ENST00000361200 | CATGAACATAGGACACGCTTCTGTCTGTAGAGGCTTCATATGAGACCCAGAAAGTCTATC |
| -1,55 | 2,4E-08 | AJ489257 | TRDN | AACAGAAGATTTAGTTAACACAATTGTATTTAATTATTTGCTTAATATGTATTTTTAAAA |
| -1,55 | 2,8E-08 | NM_004393 | DAG1 | TTGAGCATGACTTTTCTTGATGTCTGAAGCGTTATTTTGGGTACTTTTTAGGGAGGAATG |
| -1,55 | 5,0E-09 | NM_001003938 | HBM | AAGTTCCTGACTGGTGTGGCCGTGGTGCTGACCGAAAAATACCGCTGAGCCCTGTGCTGC |
| -1,55 | 7,5E-11 | NR_002330 | ST70T1 | ATTTTGACTGTGAAACGGCCACCTCTAACAGAAAGTCCATTTTAAACATTGTTTTGTTGT |
| -1,55 | 2,3E-08 | NM_002499 | NE01 | CTCTACCAGCTGTTAATCCATCACTCTGAGGGGGAGGAAATGTTGCATTGCTGTTTGTAA |
| -1,55 | 6,3E-11 | NM_024567 | HMBOX1 | CACAGACATCCTTTCCTCTCACAAGCTGTGTGACTTAGTAGATAAAATACTGCCTTCTGC |
| -1,55 | 3,0E-11 | NM_001039570 | KREMEN1 | CATCGTGGGTCTCATGCACGTCAAGACCTTCCCACATCCAAACTCAGCTTCCAGCAGGGA |
| -1,55 | 7,4E-08 | NM_005220 | DLX3 | AGACGGTGAACCCCTACACCTACCACCACCAATTCAATCTCAATGGGCTTGCAGGCACGG |
| -1,55 | 3,1E-07 | NM_001620 | AHNAK | GAAAAGTAACATTCCCTAAAATGAAGATCCCCAAATTTACCTTCTCTGGCCGTGAGCTGG |
| -1,55 | 5,4E-10 | NM_199461 | NANOS1 | TACAGCTCCTTATCGTTCTATGTACCAGGTATTTTATTACTGAACTAGCAACTAGCCTTT |
| -1,55 | 1,5E-08 | THC2294276 | THC2294276 | TTAGCCCCATTGCTAATTAAAAGCCCCATTAAAAGAGGGAAGTTCTTTTAGGTATCTACA |
| -1,56 | 1,4E-12 | NM_015193 | ARC | CCCACAGATTTTATTTTTGCACATAAGCCATAACCAATCCTCAAGGCTGGCACAGGCTTT |
| -1,56 | 1,8E-11 | NM_014181 | HSPC159 | GACACCGAGCCATTATACCCAGAATAAAATAATACATTTATGCTGGATTTTATTCAGACC |
| -1,56 | 4,1E-07 | AL713714 | AL713714 | TGAGTAAATCAACACTAGCACTTATGTCTATCCAGAAAATAATCAGGAGATTATCACAGC |
| -1,56 | 4,7E-08 | NM_014772 | KlAA0427 | CTCCAAGAAGCCAAGTCCCAGTCTGTTTTCTGGCATCAGACACCGGCCCGTGTTCCTTGT |
| -1,56 | 2,6E-09 | NM_022361 | POPDC3 | TATAGGAAAAAGATATCACTATGATATTCGGCTACCCAACTTCTATCAAATGTCAACTCC |
| -1,56 | 3,4E-04 | NM_152716 | FLJ36874 | TCTCCCATTCAGCAATCAACCAGACTAAGGAGTTTTGATCCCTAGTGATTACAGCCCTGA |
| -1,56 | 1,5E-08 | ENST00000324559 | ENST00000324559 | CTCTGTTGATTGACTCCAGTTGAAGGTGAGAAATCTGTACCAATCATTCAAAAAGGGAAT |
| -1,56 | 6,3E-12 | NM_015059 | TLN2 | ATCTTGTATTTTGTGGCCCAGAAAACTGAACGATTATTTTGTTCCTCCGTAGTCCAAAGG |
| -1,56 | 6,2E-07 | NM_033229 | TRlM15 | AGCCAGCGGGCTTTCGGGGAAAAAAAAGAAAAAGACATCTAAAATAAAATGTTTAAACTG |
| -1,56 | 1,0E-07 | NM_013239 | PPP2R3B | GCAAGCTGGCCAACGTCTTCTTCGACACCTTCTTCAACATCGAGAAGTACCTCGACCACG |
| -1,56 | 1,2E-11 | NM_078481 | CD97 | TCCTGGACTTTTCCTCTCATGTCTTTGCTGCAGAACTGAAGAGACTAGGCGCTGGGGCTC |
| -1,56 | 9,7E-10 | NM_172390 | NFATC1 | CTCTGGTGGTTGAGATCCCGCCATTTCGGAATCAGAGGATAACCAGCCCCGTTCACGTCA |
| -1,57 | 6,2E-12 | BE138567 | BE138567 | GGTGACTTTAAATCCAAGGTAAAAAACACGGCATGGGTATTAGTTTGAATAGGGAAAATG |
| -1,57 | 5,3E-11 | NM_002829 | PTPN3 | TCCGAATGTTTGGCCTTCTGAGAAAAGAGCTTCTAGTAATTGAACCATGGGAAACCCAGC |
| -1,57 | 1,9E-11 | NM_153486 | LDHD | CCCTTCACCTGGTGACAGGAACACTCCTTTCCTGGTATGGAACGTGAGCTCCCGTGACAT |
| -1,57 | 9,1E-07 | NM_002314 | LlMK1 | AGTTGAGCATCTAGGAAGTATTAAAACTGTGAAGCTTTCTCAGTGCACTTTGAACCTGGA |
| -1,57 | 3,5E-05 | NM_006133 | C11orf11 | CAAATTAATATAGCTTATTCTATAAATATATCTGTATATAAAGGTTTCTGTATATTGTAT |
| -1,57 | 3,5E-09 | NM_001007279 | RRP22 | AAAGGACTTGGCTATGAACTTGACTGGAAACACGCAGCCTGCTCCTGGAGCTTCACTGGA |
| -1,57 | 4,5E-10 | NM_007341 | SH3BGR | CCAGAAAATGGAAGCTATGAAGCAACATTTCAAATGTCTCTCCACAAACCAAACTCAACA |
| -1,57 | 2,0E-04 | NM_020062 | SLC2A4RG | CATGTTTGTTTTTGAAGCTCAGGTGTCTCACGTCTGGGCTGCACCAGGCGAAGAGAGAAA |
| -1,57 | 1,3E-10 | ENST00000367058 | ENST00000367058 | GACACTATTTAAGGGCCCGCCTCTCCTGGCTCACAGCTGCTTGCTGCTCCAGCCTTGCCC |
| -1,57 | 2,0E-10 | NM_014914 | CENTG2 | CCCTATGATGGTGTGTGTCAAATCAGTTGTAGCTAATCTGTCCAGGGAGAATACTGGCTT |
| -1,57 | 8,2E-13 | NM_002960 | S100A3 | GGTCTTCTGCGATCAGTTAACCCATTTTACCTAGGAGGCCCAGAGATGTGAGGGCTCCTT |
| -1,57 | 9,0E-07 | NM_052843 | OBSCN | ACGGGCCCTTCTTACCTCACTCAACTTCAGCCAGGAGGACTGGGTGGTGCTTGCAATGTT |
| -1,57 | 8,6E-15 | NM_032902 | PPP1R16A | GTCTGGCTGCAAAGACTATTTTTATCCTGCAACTCTTGATAAAGGGCTGTTTTGCCATGG |
| -1,57 | 1,1E-13 | NM_001008392 | CTDSPL | GCCTATTAGTAATGCTGATTTTATTGTTCCGGTTGAAATCGATGGAACTATACATCAGGT |
| -1,58 | 1,6E-08 | NM_002178 | IGFBP6 | TGCTGCAGCAACTCCAGACTGAGGTCTACCGAGGGGCTCAAACACTCTACGTGCCCAATT |
| -1,58 | 6,1E-05 | NM_002055 | GFAP | AATCTTCAGGGCACTGCTGCTGCCTTTAGTCGCTGAGGAAAAATAAAGACAAATGCTGCG |
| -1,58 | 1,6E-10 | NM_018948 | ERRF11 | TCAGTATTTCCTGGGGATTGTTTGCTTTTTAAGTAAAACACTTCTGACCCATAGCTCAGT |
| -1,58 | 5,8E-10 | NM_032895 | MGC14376 | TCATCCCAGCATTGCAATGGAGAAAAATGGGAAGCAATGGTTTGGTTGGGAATTTATTCC |
| -1,58 | 6,0E-11 | NM_003966 | SEMA5A | GGGCTGAGGTTCTAAAGGAATTGAAAAGAGACGATAAACATTGAATGAGGGTGAGGGCAT |
| -1,58 | 2,6E-09 | NM_002252 | KCNS3 | TGACTGAGACGACATGCATGTAAGATCCACAAAATGAGACAATGCATGTAAATCCATGCT |
| -1,58 | 5,0E-11 | NM_015124 | DIP | TCTGGGCAGATGTTTAATTTCTGTGACTAATCACTGAACTAGACGAATGTTAAATTTTTT |
| -1,58 | 2,1E-14 | NM_020127 | TUFT1 | TTTCTGTGTCTCAAGACTGGGCTCACATTCTGGCTTTGTCCATAACAATGCTCTGGGATT |
| -1,58 | 2,1E-04 | AK129956 | AK129956 | CCTTCCGAGAATAAGTATTACCTTTAAACAATATCAGCGCACACACATAGCTGCATGTTC |
| -1,58 | 1,6E-05 | BC054888 | BC054888 | AAGCCAGTCTTTTCTGCAAGGTCTGTTGACCAAGAGCATATTTCCCCTCTGTTGTACATC |
| -1,58 | 1,4E-07 | NM_001554 | CYR61 | ACCAAGAAATCCCCCGAACCAGTCAGGTTTACTTACGCTGGATGTTTGAGTGTGAAGAAA |
| -1,58 | 8,8E-10 | NM_020433 | JPH2 | ATGGCCCACAGCTCTGCATCTGCATCTGAGCTTCTCAAGACTCCTGGAGCATGAGGGGAA |
| -1,59 | 3,6E-09 | CR622844 | CR622844 | ATACAAAGTGCAAAAAAGCTTGGCTCCTTCTGTATTCTTTAAAAACAAAACAACAACAAA |
| -1,59 | 7,3E-10 | NM_014575 | SCHIP1 | AGTTCCTTTTTAGATGTGCTATTAACATTCTGTTGGATTCAGAGGGTTCCTTGAAAGTTT |
| -1,59 | 4,5E-09 | M_016835 | MAPT | AGTTTGCCATGTTGAGCAGGACTATTTCTGGCACTTGCAAGTCCCATGATTTCTTCGGTA |
| -1,59 | 8,3E-09 | NM_007197 | FZD10 | CTTCACAGTGCCAGGAAAGAGTGGTTTCTGCGTGTGTATATTTGTAATATATGATATTTT |
| -1,59 | 7,5E-10 | NM_012278 | ITGB1 BP2 | ATTGAGCAGCAGGAGGCTGAAGGAGGGGAGAACAAAATTGTCCAAACCATGCTGTTTTTT |
| -1,59 | 1,7E-11 | NM_199165 | ADSSL1 | GGGTTGGTGTTGGCAAGTCAAGAGAGTCGATGATCCAGCTGTTTTAGTCGCAGACTGAGC |
| -1,59 | 3,5E-09 | NM_004058 | CAPS | AAATCTGTAAAACATCAACCCCCAATCAGAAGATGGCAAATGGGGAATAAAAATAGCAGG |
| -1,59 | 7,2E-13 | NM_145659 | IL27 | GGAGTTGCTGCTGCTGTCCAAGGCTGGGCACTCAGTCTGGCCCTTGGGGTTCCCAACATT |
| -1,59 | 5,0E-08 | NM_173167 | UNC45B | GCAGAAGTGGTTCAGACAGCCCGAGAATGTCTCATCAAGTGCATGGATTATGGTTTCATT |
| -1,59 | 2,7E-11 | NM_145064 | STAC3 | CAAGCACAAGCAGCCTGGCTTCCAGCAGTCTCATTACTTTGTGGCTCTCTATCGGTTCAA |
| -1,59 | 7,5E-10 | NM_004468 | FHL3 | AGGGCAGCAGTTCACCTCCCGGGATGAAGATCCCTACTGTGTGGCCTGTTTTGGAGAACT |
| -1,59 | 1,0E-11 | NM_207477 | FLJ27365 | CAAGTCCACTTGGGCATGGGGAGCTGAGAGCTAGCATGCCGTTTAACTTGGCAGGAAGCA |
| -1,59 | 4,1E-09 | A_23_P20578 | A_23_P20578 | GGGGCTACGTGCCAGGGTATAAGTTCCAGTTTGGCCACACATTTGGCCATCTCACCCATG |
| -1,59 | 1,0E-09 | NM_139166 | ABRA | CACAGACGAGATGGCAAGATCCAGGTTACTTTTGGAGATCTCTTTGACAGATACGTTCGT |
| -1,60 | 7,8E-12 | AL831818 | SYNPO | TAGCTGTAACAATCTGTGAGGATTGAGAGGATAGGCTGGTGCTTTGAAGAAAGGCCTTGT |
| -1,60 | 4,5E-11 | THC2431711 | THC2431711 | AGACTTTTTGCCATATCTGACTGAATATAGCCAGTCATTTTTTCCCTTAAATCAAGTCAC |
| -1,60 | 2,5E-11 | THC2238625 | THC2238625 | CCTGCTGCTGCTCTTCATACACCAGGATATCCAGCATCTGCTTCAGCCGCCAGGGAATGT |
| -1,60 | 2,7E-10 | NM_198501 | SMTNL2 | TCCCTCTGGTGAATTCGATACATCGGCTTTACAGGGTTACAGTGATTACCAAGTGTTTTT |
| -1,60 | 7,7E-16 | NM_015689 | DENND2A | CACAAGAAATAACTCTCAGCCTCAAGGGAAAACTTCCTCCTAGTGCAGCCCTATTCTTTA |
| -1,60 | 8,2E-11 | NM_194463 | RNF128 | CTGAATGGATGAAACCATTGCATTCTTGTACACTGATTTGAAATGCTGTAAATATGTCCC |
| -1,60 | 5,2E-12 | NM_152372 | MYOM3 | CCGGATGCACTGGGGCTATCTAACAGTACTGGCATCTGATAGGTAGAGGTCAGGTACGCT |
| -1,60 | 1,9E-07 | NM_032608 | MY018B | AACCTCTAGGCAGTGACCCATTCAGCTGGAAACTCCCAAGCCTCGACTACGAACGCAAGA |
| -1,60 | 8,5E-11 | NM_152336 | FLJ32310 | TTCTGTGCCTAAGTGCATATGTGCGACTGACCTGTTGACCTGTTACGAACTAGCTTTTGA |
| -1,60 | 3,8E-08 | NM_001945 | HBEGF | CCCAGTGGAAAATCGCTTATATACCTATGACCACACAACCATCCTGGCCGTGGTGGCTGT |
| -1,61 | 3,7E-06 | NM_002586 | PBX2 | TGTATCTTCCTCAATTTCCCCATAAATGAGGTATCTTTTTGTCACACCAAAATCAAGGGG |
| -1,61 | 1,8E-13 | BC045676 | C17orf72 | ACAGCCAACTGGAAAGATATAAAAGTTTGGGTCTGTCTCCTCTCCTTCAGAAATGAAATA |
| -1,61 | 2,0E-14 | NM_152237 | GAS2L1 | AGCCCGGTGTTCTGAAGACCTGTGGCCCAGCAGAGCCTCTGACAGTAAAGTTTTGCTCCA |
| -1,61 | 1,8E-11 | ENST00000256367 | ENST00000256367 | CCTAGTCTCCTGATGTCTGTGCTCGTTATGCTATGCCATGTTGCTTCAGTCAAACAAGAA |
| -1,61 | 6,4E-11 | NM_005194 | CEBPB | CACGTGTAACTGTCAGCCGGGCCCTGAGTAATCGCTTAAAGATGTTCCTACGGGCTTGTT |
| -1,61 | 5,6E-11 | NM_145038 | C2orf39 | CTGTCTTTATAGCCTGTCGAAATAAGGAGCCAGAGGAGTTACCTGTGTCCTGCATTATGA |
| -1,61 | 2,8E-09 | AF227517 | AF227517 | TATAATTTGACCCTAGTTAGAATGGTTATCTGAGGAAGTGGCCTGTACGATTTCTGCTTT |
| -1,61 | 2,8E-11 | NM 007148 | ZNF179 | ACTTCTAGGCTATGAAACTGACTTCTAGGCTATGAAACTTACAGGGTATGGGTGGGCACA |
| -1,61 | 4,7E-15 | NM_032525 | TUBB6 | AAGCCTGAAATTGTGCCGTGTTGCCTTATATGAATATGCAGTATGGGACTTTGAAATAAT |
| -1,61 | 1,2E-06 | AB209463 | CSNK1 D | TTGTATCGCCACTGTATTTGTGTACTTTAACAATCGTGTAAATAATAAATTCATAATGAC |
| -1,62 | 1,6E-10 | 0 no_000507 | FBP1 | TAATGCCACTGGTGTTAAGATATATTTTGAGTGGATGGAGGAGAAATAAACTTATTCCTC |
| -1,62 | 3,3E-13 | M_001017402 | LAMB3 | GCCTTTAGTTCTCCACTGGGGAGGAATCCTGGACCAAGCACAAAAACTTAACAAAAGTGA |
| -1,62 | 3,1E-08 | M_001024858 | SPTB | ACCTGCTCAACCGTGCTTCCACCAACCAGAGGAAACCCATTTACTAGTTTTTCTAATAAA |
| -1,62 | 9,8E-12 | NM_032726 | PLCD4 | CAGCAAAGATTAGGGAAAGAGACTTGACCCCAGGACTGTACTACGACTCTTAAGAGAACA |
| -1,62 | 4,8E-08 | NM_080621 | SAMD10 | AAGCTTCCTTCGGGAAAATGTCCTAGCTGCTGCTGAGGCTTGAACCCAGACCCCAGCACT |
| -1,62 | 1,2E-08 | M_016223 | PACSIN3 | CCTGGACCTTTCCAGCAGTGAGAAGTTCCATGAACTCCACCGTGACTTGCACCAGGGCAT |
| -1,63 | 5,0E-08 | NM_012339 | TSPAN15 | ATCTCTGGCAGTGCCTTGGCGGTGGTATTCAAGGCAGTTTTGTAGCACCTGTAATTGGGG |
| -1,63 | 2,8E-11 | NM 001004439 | ITGAL1 | GACACATGAAAGGCATCAGTCCCCCTGTGCATAGTACGACCTTTACTGTCGTATTTTTGA |
| -1,63 | 7,0E-11 | AK023606 | ADAMTSL4 | AAACCGTCAACCCATTTAGCTTATCCCTTGGCCAAAAAGTGTATGAGATGTGCCTGGATG |
| -1,63 | 3,1E-07 | NM_001927 | DES | CCAGCCCAGGGTGGACTTAGAAAGCAGGGGCTACAAGAGGGAATCCCCGAAGGTGCTGGA |
| -1,63 | 4,4E-11 | NM_199162 | ADPRHL1 | TCCCTGTGATGAGGTTCTCTCTCAGAGAGTCTTGGAAAAGAGACCACTTGCTCTTGTTTA |
| -1,63 | 3,4E-11 | NM_176072 | P2RY2 | GCTCCCATGGGCTAGGAGCCAGTGTGAGGCTGTAACTTATACTAAAGGTTGTGTTGCCTG |
| -1,63 | 7,8E-13 | NM_020927 | KIAA1576 | TAATCGCAAATTACTCCAGATGGTTAAAGAAAACCCTTTCAGAATACAGAACGAGAAAAC |
| -1,63 | 3,1E-09 | AB040888 | ODZ3 | AAATCATCAAGGACAAAGGCCTCGACCTGTTGCGCTGGGCCGTCTGTTCCTTCTAGGCAC |
| -1,63 | 3,5E-10 | NM_003970 | MYOM2 | ACCCGTCTAAGGGAGAAAGCTAATGTTTTCCACAAGACTGAACAACGTGTATTTACACGA |
| -1,63 | 2,4E-14 | NR_002942 | LOC340508 | GGATGAGCTTTGCTATTTAATTACTAATATTATTGAATGCCTTAGAGGAGGCCAGAGCAG |
| -1,63 | 1,8E-10 | NM_012266 | DNAJB5 | GCTGGGGGAAGACTGCTTCAGCCAGATCCTGGGGTGGGGTCTTTAGGTTTTCTCACTTTG |
| -1,63 | 2,4E-10 | NM_001104 | ACTN3 | CAGCTCAACGAGTTCCGAGCATCCTTCAACCACTTTGACAGGAAGCGGAATGGGATGATG |
| -1,63 | 1,1E-10 | NM_001299 | CNN1 | AGAGACCCAGCGGACACACGCGGTTTCGTTTGCAGCGACTGGCATACTATGTGGATGTGA |
| -1,64 | 1,6E-13 | NM_002632 | PGF | AATGTCACCATGCAGCTCCTAAAGATCCGTTCTGGGGACCGGCCCTCCTACGTGGAGCTG |
| -1,64 | 1,5E-11 | NM_014370 | STK23 | TAGAGCAGGCCACACAGTTCAGCGCCTTTCTGCTGCCCATGATGGAGTACATCCCCGAAA |
| -1,64 | 5,2E-11 | BU160948 | BU160948 | TTTTTTGCCTGTGTGAATTCTACTTTTTAGCAAAAATAAAGCCCCCCAAAGGATGTGCAA |
| -1,64 | 6,5E-11 | NM_001231 | CASQ1 | GTGTCTCTTCCATCACTCTCCATACTCTTTCTTGTGATTCTCCTCTAGCCATATATATGG |
| -1,64 | 1,9E-13 | NM_080647 | TBX1 | TGTAGATACTGTAGATACTGTAGATACCGCCCCGGCGCCGACTTGATAAACGGTTTCGCC |
| -1,65 | 1,3E-13 | NM_021979 | HSPA2 | TTTTTTGTTTGTTTCTTTGAAATGTCCTTGTGCCAAGTACGAGATCTATTGTTGGAAGTC |
| -1,65 | 7,5E-15 | NM_018205 | LRRC20 | TCTGTCTTGGAAAGTGTGGGCAGCCACAGATGCCCCCAAATCAGAGCTCACAGTGGGTGA |
| -1,65 | 2,7E-08 | NM_003098 | SNTA1 | TCCTGCGACAGCCCTTCGAGAAGCTGCAGATGTCTTCAGATGACGGTGCCAGTCTCCTTT |
| -1,65 | 1,0E-10 | NM_001179 | ART3 | GGAAGCTTGAAGACCATGGTGAGAAAAACCAGAAGCTTGAAGACCATGGTGTGAAAATCC |
| -1,65 | 7,1E-09 | NM_002704 | PPBP | GACAGTGACTTGTATGCTGAACTCCGCTGCATGTGTATAAAGACAACCTCTGGAATTCAT |
| -1,65 | 6,9E-15 | NM_138409 | C6orf117 | GTGGCCTGAGTTATGTAAACAAGTGAGCAACACAGCTTTAATTTCATGGAGGAATCAAAG |
| -1,65 | 8,3E-09 | CR622769 | LRRC38 | ATATAATAGGCCTCAGCAACCTTGGGATGTTATACTGGGAACTAATAAACTCGTGTGCAA |
| -1,65 | 8,9E-13 | NM_001897 | CSPG4 | TGGGTTTATTTAAGGGAGATTGCAAGGAAGCTATTTAACATGGTGCTGAGCTAGCCAGGA |
| -1,66 | 2,8E-12 | NM_006877 | GMPR | GAGGAACGGACGGAAGCTCAAGCTCTTCTACGGGATGAGCTCTGACACCGCCATGAACAA |
| -1,66 | 1,8E-12 | NM_015157 | PHLDB1 | CAACTGGGGCTTTTGTATAAGAAGACTTTAATATTCTGTAAGGAGCTTGGTCCTGTGAGT |
| -1,66 | 3,7E-11 | NM_015270 | ADCY6 | CACTAGCCTGAATTTTGCTTGAAGATCACTTTGTCTTGGAAATGACTAGAGAGGCAGAGG |
| -1,66 | 2,5E-12 | NM 025249 | KIAA1683 | GCTCCCTGCAGTGGGGACTTCGTGGGAGGCACTCATGGCTCTCTGGGTCTAATGAATAAA |
| -1,66 | 8,5E-10 | NM 003803 | MYOM1 | ACTTGGGTGTGAATGGTTTGGGTTAAGGATGAGACGTCTTCATGCTTTCTCCTCCCTATT |
| -1,66 | 7,5E-07 | NM_001005291 | SREBF1 | GGCTCCTGTGCTACTTTGCCTTTTGCAAACTTTATTTTCATAGATTGAGAAGTTTTGTAC |
| -1,66 | 2,4E-10 | NM_002471 | MYH6 | ATCAACCAGAAGAAGAAGATGGAGTCGGATCTGACCCAGCTCCAGTCGGAAGTGGAGGAG |
| -1,66 | 1,8E-10 | NM_000476 | AK1 | CCAGCGGGCGTGTGGACGACAATGAGGAGACCATCAAAAAGCGGCTGGAGACCTATTACA |
| -1,67 | 7,9E-10 | NM_015069 | ZNF423 | TTTCCGCCGCAGACTACATTTCTAGAGTTAGAGAAACCTGCTTTTTAAGGCTATTGTCCT |
| -1,67 | 7,5E-11 | NM_001042 | SLC2A4 | TGTATATATATAGATACTTCTATAAAGTCACTGCTGAAGACAAGCATCCTATTGTGGAGG |
| -1,67 | 3,5E-12 | AF131834 | AF131834 | GCGATCTCCCAGTGAGCCATCACGATGCCCTTTTCAAATAAATGTTAATGTTGTCACCAC |
| -1,67 | 6,2E-12 | AK023797 | KlAA0672 | GATGTCTTTCAGGACAGTGTCCAAATGTATGTCTGTACACTTAGTATGGTATTCCCCAGT |
| -1,67 | 2,7E-11 | NM_000127 | EXT1 | ACGACTACTCCATGGTGTTGACAGGAGCTGCTATTTACCACAAATATTATCACTACCTAT |
| -1,67 | 2,7E-05 | NM_002966 | S100A10 | AGAAAAGTTAAATACCAGATAAGCTTTTGATTTTTGTATTGTTTGCATCCCCTTGCCCTC |
| -1,67 | 8,7E-15 | AK098354 | AK098354 | ACCCTGGATTAATGTAAATAGCTTTTTGGGGAACGGATTCTAATGTCACGTATGTGACCG |
| -1,67 | 1,8E-13 | NM_022144 | TNMD | GGAGATTTGTGATAACGTGACCATGTATTGGATCAATCCCACTCTAATATCAGTTTCTGA |
| -1,67 | 3,9E-08 | AB095949 | ADAMTS16 | GAAATGGGAAAAATGAAATTCCTGCTAAGGTGCTTCTATCTCTTTCAGATTCATGCATTG |
| -1,67 | 2,0E-11 | NM_005159 | ACTC | AACCGGGAGAAGATGACTCAGATCATGTTTGAGACCTTCAATGTCCCTGCCATGTACGTG |
| -1,67 | 1,3E-06 | NM_012156 | EPB41L1 | AGACATTGTATGCTTATGGTTTCTCATTATGAAGGTGCAGCTTGTAGGAGGTTTGTACGG |
| -1,68 | 5,0E-06 | NM_032777 | GPR124 | AAAGCTTTGTATTATTCTTCCACATATGCTGGCTGCTGTTTACACACCCTGCCAATGCCT |
| -1,68 | 5,8E-08 | NM_153020 | RBM24 | TTTTGCCTGTGTAGATTTCATTTATTGGTAATTCATTAGTTTAACACTATTATGTAGTTT |
| -1,68 | 1,9E-07 | NM_001449 | FHL1 | CTGTAAAATGGCATTTGAATCTCGTTCTTTGTGTCCTTACTTTCTGCCCTATACCATCAA |
| -1,68 | 2,9E-09 | NM_015432 | PLEKHG4 | GACTTGATGCCTTTTGAATAACTTTCAATAGAATTGTCTAAAATTATCTTACTGGTTGTT |
| -1,68 | 7,8E-11 | NM_000710 | BDKRB1 | TTCATCCCATAGGAAAGAAATCTTCCAACTTTTCTGGCGGAATTAAAACAGCATTGAACC |
| -1,68 | 2,1E-04 | NM_021939 | FKBP10 | CAGACTGGGCTGTAGTTAGCTTTTCATCCCTAAAGAAGGCTTTCCCTAAGGAACCATAGA |
| -1,69 | 3,2E-12 | NM_172311 | SALF | AAGCCATTGGTGATGCAGAGTGGTAAACCTTGTGAGCTCAGTACATCTATTTTGTGAACA |
| -1,69 | 1,6E-11 | NM_004235 | KLF4 | CGTTCCAGTGCCAAAAATGCGACCGAGCATTTTCCAGGTCGGACCACCTCGCCTTACACA |
| -1,69 | 1,5E-11 | NM_003196 | TCEA3 | ACCACCACTGACCTCTTCCAGTGCAGCAAATGCAAGAAGAAGAACTGCACCTATAACCAG |
| -1,69 | 3,1E-12 | AK123912 | AK123912 | GTAAAATGGAACCAAATCGCCTCTTCAATGGATTTGGTCCCCTTCAACCAGCTGTAGCTA |
| -1,69 | 3,3E-14 | NM_004653 | SMCY | TCCTACTATCTCCTGGCCTGGACCTCAGAAGGAGCTTTTTGCCTATCTATAATTTTTCAC |
| -1,70 | 1,7E-07 | NM_206862 | TACC2 | TTGCATTTTCCTAGTATAATTCATAGCAAGTTGACCTCAGAGTTCCTGTATCAGGGAGAT |
| -1,70 | 7,8E-11 | NM_005309 | GPT | GGATGACCATTCTGCCCCCCTTGGAGAAACTGCGGCTGCTGCTGGAGAAGCTGAGCAGGT |
| -1,70 | 9,7E-08 | NM_017625 | lTLN1 | TCCTCCCAACCATGAGATCCCAAGGATGGAGAACAACTTACCCAGTAGCTAGAATGTTAA |
| -1,70 | 6,0E-12 | A_24_P919283 | A_24_P919283 | CTCAGAGAAGCCAGTCAAATCTCCCCTTGTTGAGAACATTTTGACAGCATAAAATTGCTG |
| -1,70 | 3,2E-13 | NM_021046 | KRTAP5-8 | TGCAAGCCCTGCTGTTCCCAGTCCAGCTGCTGTAAGCCCTGCTGCTGCTCTTCAGGCTGT |
| -1,70 | 4,5E-11 | NM_016300 | ARPP-21 | TAGGTATGCCTTTATAGCTTAGCTAGTGACATGAATTCATCAAGGTAAGATTTTCTCCTA |
| -1,70 | 6,1E-10 | NM_020386 | HRASLS | TTGGGAGGAGGAAAAGAAACCTGGGGTGAATACTTATTTTCAGTGCATCATTACTGTTCC |
| -1,70 | 6,1E-08 | NM_052886 | MAL2 | CTAGAATTTAGGTGTGAGATTTTTTGTTTCCCAGGTATAGCAGGCTTATGTTTGGTGGCA |
| -1,70 | 5,7E-12 | NM_031886 | KCNA7 | CTCAAAGGGCATTTAGAGGAAGAAGGCTGAAATCACTAACACATATAGGGCTTCCTTTGG |
| -1,70 | 2,2E-09 | NM_198271 | LMOD3 | TCAAGTGTCTAGCACATACTACATCTAAGAGGCTAAACTCTGATAGGAAAAGCACTTTAA |
| -1,71 | 5,6E-05 | NM_002421 | MMP1 | ACATGTGCAGTCACTGGTGTCACCCTGGATAGGCAAGGGATAACTCTTCTAACACAAAAT |
| -1,71 | 3,0E-08 | NM_002081 | GPC1 | AGGTCCCCGGTTGCTGGTCAGGTCCCCATGGCTTGTTCTCTGGAACCTGACTTTAGATGT |
| -1,71 | 9,7E-12 | AK055821 | AK055821 | GACAAACAGCTCATAAGCCCGTACTTTTACATACTCACTTCCTGAATTGCATATTGAAAA |
| -1,71 | 2,0E-12 | ENST00000216214 | ENST00000216214 | CAGCTGGATCAAAAGGGCTTCTTTGGAATTAGGTTGTTTTAGTAACTTCTGTTCCAAAGA |
| -1,72 | 3,4E-08 | NM_001020818 | MYADM | TTTTTTAAATCCTTGGAGCTTCTGGTTCCTATCAGTTCCTGTTGTTAATCGTAGAACCGT |
| -1,72 | 2,5E-11 | NM_001458 | FLNC | AATGTCACCTACACTGTCAAGGAGAAAGGGGACTACATCCTCATTGTCAAGTGGGGTGAC |
| -1,72 | 1,3E-12 | NM_006366 | CAP2 | GGGGAGAGTGCCAACAATTTCCATTAATAAGACAGAAGGTTGCCACATATACCTCAGTGA |
| -1,72 | 1,5E-11 | NM_002340 | LSS | GTTACTGAAAGTCACTTTTATGAGCATCTGCCTTAATAAACAGACATTGATTCCCTTATC |
| -1,72 | 4,1E-13 | A_32_P171043 | A_32_P171043 | CACAGTGACTAGTCATATACTGGAGTAGCTCTGTTGTTCATTATTTGTAACAGGTCCATG |
| -1,72 | 1,3E-13 | NM_178568 | RTN4RL1 | AGCATCCACAGTGTTAGTCCAAAGGGTCGGACCGTGTCGTCAGCCTAGCGTTTGGTCAGT |
| -1,72 | 2,4E-12 | NM_017911 | FAM118A | TGTTTTGACCACTTTCCAGGATATGTGCAAGACCTTGCCACTCAGATCTGCAAACAGCAA |
| -1,73 | 1,6E-06 | NM_000037 | ANK1 | ATCATAGTCCGTGTTCAAAGTGTAAACTGTACAGTAATCAGCCTTGTGTATATGAAAAAC |
| -1,73 | 8,8E-13 | NM_002135 | NR4A1 | AATGACAGATTCTGACATTTATATTTGTGTATTTTCCTGGATTTATAGTATGTGACTTTT |
| -1,73 | 1,1E-12 | AK022045 | AK022045 | TGTAATCAGTTCATCTTACCTTGCCGGGCTCTGAAAGAATGAGTGTGTGGGATCAGATTT |
| -1,74 | 6,1E-10 | NM_018687 | LOC55908 | CTGAATCTGCCTGGATGGAACTGAGGACCAATCATGCTGCAAGGAACACTTCCACGCCCC |
| -1,74 | 3,2E-12 | NM_002607 | PDGFA | GTTTACCTAATATTACCTGTTTTGTATACCTGAGAGCCTGCTATGTTCTTCTTTTGTTGA |
| -1,74 | 2,3E-10 | ENST00000330255 | ENST00000330255 | ACCGTGAGCCCGACTTTCATCCGGTCGTACCACGTGTATCCAGAGCAAGGAAACTGGGAA |
| -1,74 | 1,5E-14 | NM_004997 | MYBPH | AAGGCGCAGAGATTGGAGTGCCCTGCGGAGTTGCACACTGGGTGGGAAGCACTCAAATAA |
| -1,74 | 2,0E-10 | NM_130808 | CPNE4 | GAGGCCTGGATCTGTTAAGCCCTTGTATTGTTAACTTTTTACAAAGAAACACAGATAACA |
| -1,75 | 8,1E-08 | ENST00000257704 | ENST00000257704 | AGCTGCACCTACCACAGCCAAGTGTATGCCAATGGGCAGAACTTCACGGATGCAGACAGC |
| -1,75 | 6,9E-13 | AK022486 | FLNB | TGTGTTCCAAGTTCTTTCTATAAAGAATCCTTCCAGAGGGAAGCCACTGTGAGTGAAAAT |
| -1,75 | 2,3E-09 | NM_001670 | ARVCF | GAGAAAAAGCATTAAAAATAAGCCAAAATAAGACTATTGGTAAACACCTAAATTTTTGTA |
| -1,75 | 3,2E-12 | NR_001544 | CYorfl4 | CCAGAACTGTGGGCAATAAATGTCTGTTGTTTATTACCTGTCCAGTATCTTTGGTATTTT |
| -1,76 | 2,1E-09 | NM_173608 | C14orf80 | TTCCAAGGCCAGGTGGCCACAGGGACGATGCAGATGCAGAGCCCACGTCACATGCTCGCT |
| -1,76 | 1,4E-07 | NM_025225 | PNPLA3 | CTTGACTACTAAAAACGTCTCCATGGCGGGGGTAACAAGATGATAATCTACTTAATTTTA |
| -1,76 | 4,5E-13 | NM_004273 | CHST3 | GTGGAAGCTGCTCTTTACCTCAAAAGCACAAAGCAGAATTGGCAACTTCACATGTCTCGA |
| -1,76 | 2,9E-12 | AK090474 | LOC441245 | TGCTCCTTCAGCATAAGGATAGTCTGCTGCTGCCACCCCGGGAAGGAGGTGAACGTCAGG |
| -1,77 | 1,2E-13 | NM_025135 | FHOD3 | TCCAGATGTAAACCCCAAACTTGTACACAAAAGAAAGCACAGATTGTTTACCTGTTGTGG |
| -1,77 | 5,6E-11 | NM_001681 | ATP2A2 | CTCTTTTTCCGTTACTTGGCTATTGGCTGTTACGTCGGCGCTGCTACCGTGGGTGCTGCT |
| -1,77 | 6,1E-12 | AK024388 | FGFR1 | CGCTCATTGGTGTTCATTCAGGAATACAGATACCTAGATTCACACACGTGGTGTGTTAAC |
| -1,77 | 1,1E-13 | CR608907 | CR608907 | AGAAAAAGAAAGATGTGTAAAGTAACAGAGAGAGGTGGCTATGGTGTAGAGACCTCTTTC |
| -1,77 | 9,6E-13 | ENST00000382496 | ENST00000382496 | TTGATTACCTATGGTTATCTGCAATTACCTACGCTTATCTTATCAGACCCTAGATGCCAT |
| -1,77 | 5,5E-13 | NM_002206 | ITGA7 | AGGAGGTTGTGTCACTGACTCAGGCTGCTCCTTCTCTAGTTTCCCCTCTCATCTGACCTT |
| -1,77 | 3,7E-07 | NM_005279 | GPR1 | TTCCCTTTGCTAACAATGAGTATTTGCTACTTGTGTCTCATCTTCAAGGTGAAGAAGCGA |
| -1,78 | 5,3E-11 | NM_002616 | PER1 | TTCTCAGAACTCAGATGTGGCTAGACCAACCAGTGGGAAACTGCCCCAGCTTCTCCCACC |
| -1,78 | 4,5E-12 | NM_001039906 | FLJ30064 | AAACATCAGTCAGTGCGTAGAACTGGTAAATGTCAGAGAAATGTATCACTTATCAGAGCG |
| -1,78 | 6,9E-11 | NM_001040167 | LFNG | AAGCGAATGATAAGGGAAAAGTTCTCAGGGAATTGAAGTGTTGTTGCTATGGTGACGTCC |
| -1,79 | 2,0E-12 | NM_001017421 | FKSG30 | AACTGGGACGACATGGAGAAGATCTGGCACCACACCTTCTACAATGAGCTGCGCGTGGCT |
| -1,79 | 5,6E-12 | NM_020478 | ANK1 | CTCTTGGCATGAATGACTGACTGTAGACGCATGACCTCCAGGCTTCAATCCTGCCTCTTG |
| -1,80 | 3,0E-14 | NM_000727 | CACNG1 | ATGAACATTGCAGGGACAACCTGTGTTTGCCAGCTGGGTGTTCCGTGTAAATAGCCAGCC |
| -1,80 | 1,4E-16 | NM_024786 | ZDHHC11 | AGAGTCAATACCAGAGAACCAGAGGCATCCTCTGTATTTTAATGAACTCTGCATTTTAAT |
| -1,80 | 1,1E-14 | NM_020689 | SLC24A3 | TTGATTCTGTTGCACATTTTGCACTGGTTTATGGCGATTGTTTTCTTGGACGGATAGTGT |
| -1,80 | 2,6E-11 | AK097227 | SPOCD1 | TGGAGGTGGCTGATAAGAGTAGAGTTTCAAAATCTCTTTAAACCTTCCTAAAGCAATGAT |
| -1,81 | 3,6E-10 | NM_033317 | DMKN | AGTTCTCCAGGAAGGAGGAGCTTCCTACTTTTGAGTTTCTCTGTGGAAATAAAACATGAA |
| -1,81 | 5,8E-13 | AK123855 | C1orf170 | CTGGGTGGGGGTCCTCAAGACTGTGGGAGACCCTGGCTGCTGAGCAGAGAACACATGGAT |
| -1,81 | 2,3E-15 | NM_133371 | MYOZ3 | GAAAAGGAGCCTGGTGGGGAGACCACTGCACCCAAAACAAATCCTTTCTTCTTCTGAGAA |
| -1,82 | 3,4E-15 | NM_002514 | NOV | TAGGCTGCAAAATTAAATCTAGACATTCTTTTAATGCCACCACACGTGTTCCGCTTCTCT |
| -1,84 | 2,7E-16 | NM_005685 | GTF2IRD1 | TCGGATATGTATCGATGCCTTTTAGTTTTTCCAATGATTTTTACACTATATTCCTGCCAC |
| -1,85 | 1,2E-11 | NM_000540 | RYR1 | GAGTCTTATGTCTGGAAGATGTACCAAGAGAGATGTTGGGATTTCTTCCCAGCTGGTGAT |
| -1,86 | 1,5E-10 | ENST00000377444 | ENST00000377444 | CTGGAACCCTCATTTTCCCCACTCTCAATGTCCCAGTGTCCAGCGTGACTAAGGACACGG |
| -1,86 | 1,7E-12 | NM_005061 | RPL3L | TTGCTGGTACCAAGAAGCGGGTCATTACGCTGAGAAAGTCCCTCCTGGTGCATCACAGTC |
| -1,86 | 7,0E-10 | NM_173201 | ATP2A1 | GTCCTCAAGATCTCACTGCCAGTCATTGGGCTCGACGAAATCCTCAAGTTCGTTGCTCGG |
| -1,86 | 3,3E-10 | NM_002686 | PNMT | CACCTTCAGACAGGCGTAGATGATGTCAAGGGCGTCTTCTTCGCCTGGGCTCAGAAGGTT |
| -1,88 | 2,1E-14 | NM_033655 | CNTNAP3 | ACTACCTTCACTGGCATTTCCATAGTCCTGGAATCCAGAGCCAAGTGGCCTATCTAAAAT |
| -1,88 | 1,1E-14 | NM_033342 | TRIM7 | CGTAACATACCAGTTAGGGCCTGCGGAAGCATCTTGTAATGGAACACATTACTATTTCTG |
| -1,88 | 6,0E-12 | NM_133379 | TTN | GATCTGAGTGCTTTCATATTTTCCAAATTATGTGGATCTAATAAACTTCCAAACAGGTCC |
| -1,89 | 8,3E-13 | NM_012309 | SHANK2 | CGTTCCTGTAACTTGTTCTACATTCCACAGTCTTTACCGTTTTATGTTCAAAATTACAAC |
| -1,89 | 1,8E-11 | NM_024074 | TMEM38A | TTCACAGAATCCTGGCAGCAGCTCCAGTCAAGAATGTCACTGGTTGGCATGATATTCTTA |
| -1,89 | 2,5E-10 | NM_138569 | C6orf142 | CACTGTTCTAGCCTTTAATGCCTTCTACTTAATATTAAGCTGACCGCAATACTAACGTGC |
| -1,90 | 3,4E-14 | NM_032578 | MYPN | AAAGAAATCCTCTGTGGGGTCACTTTAAAAACTACTAGCTTCAACTACCACTTACAAAAT |
| -1,90 | 3,5E-12 | NM_003311 | PHLDA2 | TTTATTTCGCTGGTTCTTTGTAGTCACATATTTTATAGTCTTAATATCTTGTTTTTGCAT |
| -1,90 | 2,8E-15 | NM_000930 | PLAT | TGAACAACTAGGCTTCAGCATATTTATAGCAATCCATGTTAGTTTTTACTTTCTGTTGCC |
| -1,90 | 9,8E-13 | AK131277 | B4GALNT3 | TCACCCATGGTGCATTGGACACTGGATCTTGAACTCCAGAGTCTGTTTAGGGAGGTGAGC |
| -1,91 | 4,5E-12 | NM_001033602 | KlAA0774 | TTCTAACTTGTGCCTAAGAATTATTGGGAAATGAAAATGCATTTCTATCTAGCTTCCCAG |
| -1,93 | 1,8E-09 | NM_152888 | COL22A1 | GACCAGACACTTGGCAAATTTACCTCTTTCTTCAAAAGAAAAACTTTAAGAAAATGAGCC |
| -1,93 | 2,1E-07 | BC045778 | LOC283824 | TGAACTATCTGAAATTGACCAGTAATCAAAGTTCCAATCATCTGAATGCTTTTCCTTGAG |
| -1,93 | 2,5E-12 | NM_000290 | PGAM2 | ACTACAACTCCATTAGCAAGGAGCGTCGGTACGCAGGCCTGAAGCCCGGGGAACTCCCCA |
| -1,93 | 4,1E-13 | NM_003837 | FBP2 | CAGAGATGGTAGCTATGAGTATGCAAAAGGTAAATCCACTTAATCACATACAGAAGAGCA |
| -1,94 | 1,9E-15 | AK126298 | AK126298 | TTTGCCTCATTCCATACTGATCATCCCAGCTGAACAATTTGAAAACTGTTCTGCCTTTTT |
| -1,94 | 1,1E-11 | AB033021 | FBXO40 | ATGTGTGATGTCAAATCAAACTGAAATGAAGAATGAGATTGAGTATATCTGTGGTGACTG |
| -1,94 | 1,7E-12 | NM_153426 | PITX2 | AATAGTTTCTCTGGTGGATGCAATGATGTTTCTGAAACTGCTATGTACAACCTACCCTGT |
| -1,95 | 8,0E-13 | NM_006741 | PPP1R1A | GACCGCTGTCCCTTATTTCAACCCGTTAGCAACAATGGATAGAGAACACAGTGGCTATTA |
| -1,96 | 1,6E-11 | NM_003226 | TFF3 | TGTCCCTTTGCTCCCGGCAAGCTTTCTGCTGAAAGTTCATATCTGGAGCCTGATGTCTTA |
| -1,97 | 9,8E-05 | NM_000484 | APP | AGTGAAGATGGATGCAGAATTCCGACATGACTCAGGATATGAAGTTCATCATCAAAAATT |
| -1,97 | 7,1E-11 | AB016898 | LOC653483 | GCTGCTGCGTGTTTCTTCCAGGGTGATTCCTTTCCGCTGGGTCACTTCAAAGCCGACCTT |
| -1,97 | 7,8E-13 | NM_001958 | EEF1A2 | CTCTGGCAAGAAGCTGGAGGACAACCCCAAGTCCCTGAAGTCTGGAGACGCGGCCATCGT |
| -1,98 | 2,8E-15 | NM_000231 | SGCG | ATGTGCCAGTGAAAGTGTTTGGACAAAAACTACATGATCTCAAAATGCACGTGGATGTGA |
| -1,99 | 5,4E-12 | NM_005331 | HBQ1 | TGGAAAGGACCTTCCTGGCTTTCCCCGCCACGAAGACCTACTTCTCCCACCTGGACCTGA |
| -1,99 | 1,1E-12 | NM_147175 | HS6ST2 | TCCCATGATTTCAACTGTAAACATCTGTCCATTGGTGTAGCTTTACAAACCATTCACTGA |
| -2,00 | 1,1E-14 | NM_007174 | CIT | AGGAAGAGAACACAAGGAATGATTCAAGATCCACCTTGAGAGGAATGAACTTTGTTGTTG |
| -2,00 | 1,1E-11 | NM_153610 | CMYA5 | CATGTGAGCAAAATCGACAAGAAAACCTTGACTTTACAGAGCAGTGTGTGAGTAAACAGA |
| -2,00 | 4,1E-16 | NM_138370 | LOC91461 | GTGAGTTTACCAGCCTAGCTATGGGACTGCTGGCTCCTAGTCCAGGAATCATGGGGGTAT |
| -2,01 | 8,7E-14 | NM_018723 | A2BP1 | TGACAAAACCATAAAAACCTTCCAATGTGGGGAGAAAGGAAGCTTTCCGAGGCCTGAGTA |
| -2,07 | 1,2E-10 | NM_005609 | PYGM | AAGTCAGCGCCTTGTACAAGAACCCAAGAGAGTGGACGCGGATGGTGATCCGGAACATAG |
| -2,08 | 3,5E-12 | NM_007026 | DUSP14 | GTTTAATAAACTGGTTCTGCTCTCTTCTGAATCTCATGCCTTTGGCACCTTGGTAGGTGC |
| -2,09 | 2,6E-13 | NM_004165 | RRAD | TTTTTTCACAGCCCGGGTGTGCCTGCCCTGGAGGGAGGCTCTTCAGTGCGGTAGCTATTT |
| -2,12 | 8,7E-16 | NM_032854 | COR06 | ACTGGCATGAGGAGTTAGGTTCATCACAAATACACACACACTGCCCCCAACCCTCTGCCG |
| -2,12 | 1,6E-10 | NM_014476 | PDLlM3 | AAACATACACTTAGCTATGTTTTGCAACTCTTTTTGGGGCTAGCAATAATGATATTTAAA |
| -2,15 | 1,2E-14 | NM_006308 | HSPB3 | TTCCTGTTCAGATGACATGGGGAAGATGATGGTTCAGCCACTGGTACTACGAGAATGTTT |
| -2,15 | 2,7E-15 | ENST00000332074 | ENST00000332074 | AATGGAAGACAGTTCCCGACCTTCTACCCCACAGAGATCAGCTCATGAGAATCTCATGGA |
| -2,16 | 1,0E-13 | NM_014859 | KlAA0672 | TTTTGATGTTGCAGCTTTGCTCACTTCCTGGCAAGGGCAGGTCATGCCTCAATTTGTAAT |
| -2,18 | 2,4E-16 | NM_002469 | MYF6 | TTTTTTGGAACTGCGAGTGGCTTAGGTCTAGCCTCATTTTGTTTTTGTTTGGTTGGTTTT |
| -2,20 | 6,3E-18 | NM_000635 | RFX2 | AAGCTCACAGTTAACGGATTGTAATGCCCGGCTTTTCCTCACTCCTGGTAACCAATACAA |
| -2,20 | 3,6E-14 | NM_002200 | IRF5 | GAGCACTTAGGTATCATATCAGATGCTCAAGGCTGGCAGCTACCCCCTTCTTGAGAGTCC |
| -2,22 | 1,7E-12 | NM_013353 | TMOD4 | AGGCCATGACCCGAAACAATGAACTACGTCGCCAGCAAAAGAAGAGATAACACTGCATTT |
| -2,23 | 4,3E-16 | BC015836 | BC015836 | GGCTGGATTGTGGGCTGCATCATAATGATTTATGTTGTCTTCTCTTAGAAAGGCAAGAAG |
| -2,23 | 8,1E-17 | NM_001007271 | DUSP13 | GATTTCCCTGACCCAATTCAGAGATTCTTTATGCAAAAGTGAGTTCAGTCCATCTCTATA |
| -2,24 | 1,2E-19 | NM_006732 | FOSB | TTTTTACAATCTGTATCTTTGACAATTCTGGGTGCGAGTGTGAGAGTGTGAGCAGGGCTT |
| -2,25 | 5,6E-14 | NM_000517 | HBA2 | CACAGACTCAGAGAGAACCCACCATGGTGCTGTCTCCTGCCGACAAGACCAACGTCAAGG |
| -2,26 | 2,7E-14 | NM_006783 | GJB6 | AGAAGACCGTGTTTACCATTTTTATGATTTCTGCGTCTGTGATTTGCATGCTGCTTAACG |
| -2,27 | 1,3E-16 | NM_021116 | ADCY1 | AGAGTGATGTGGGCAAGGGTGTCAATTTTCTCGCACAATACAAACTCACTGAGGATGCTT |
| -2,28 | 1,5E-13 | NM_080874 | ASB5 | CAGATATTAAAGCTGCTTTTGGGTAAAACAGGTTTCCACCAGTATTTTCCCTGAGCTAGA |
| -2,30 | 1,7E-14 | ENST00000284767 | ENST00000284767 | TTAACTTAAACATAGGGATAATGGCAAGCCACTCATAATGTTGTCATTTGAAGATCACAC |
| -2,31 | 5,8E-15 | NM_024875 | SYNP02L | ACAGCATCTTGTGAAAGTTATGGAGCATGAAAAGACTGAAGGGCCAGGACAGTTTGCATG |
| -2,32 | 3,0E-19 | NM_004816 | C9orf61 | GAGGATTATTGTTGATTCCATTTACTCATGCTTGCAAAACTAGAGACCCCTAAGGCAGAA |
| -2,32 | 1,3E-12 | NM_004004 | GJB2 | GTCATAGCACCTAACAACATTGTAGCCTCAATCGAGTGAGACAGACTAGAAGTTCCTAGT |
| -2,33 | 3,7E-16 | NM_004660 | DDX3Y | CACTGATAGGAAGGTCCACATCCACAAAGTTTCTCTTGAGTTTTGTTATGTGTTTTGCTG |
| -2,34 | 5,7E-14 | NM_001007176 | C8orf22 | AAAGACTATCGCTGGAAGTGGCACTTGCTACCTGGTGCATCTTTGAAAAGTGTGCCTTTA |
| -2,35 | 2,8E-15 | NM_006790 | MYOT | TAACCCAGAAGGAGAATTTCAGCGTTTGGCAGCTCAATCTGGACTCTATGAAAGTGAAGA |
| -2,38 | 1,8E-15 | NM_032588 | TRlM63 | ACGATGTGCTGTATTTCAGTGTCTATCCCAGACATACGGGGTGGTAACTGAGTTTGTGTT |
| -2,40 | 4,5E-15 | NM_004533 | MYBPC2 | CCAAGACAATTGGTGGTGGAGTCCTGACCCCAATCCCCAACCTCCCAGGACTGTGTTCTT |
| -2,40 | 2,4E-13 | NM_021245 | MYOZ1 | GCAATGCCCTATGGTGGATATGAGAAGGCCTCCAAACGCATGACCTTCCAGATGCCCAAG |
| -2,41 | 3,4E-10 | NM_003063 | SLN | CAATATCTGCTATGCTGTAGTGCTAGGATTGATTATGTGTTCTCCAAAGATGCTGCTCCC |
| -2,44 | 7,9E-16 | NM_001976 | EN03 | CTCGGAGCGTCTGGCCAAATACAACCAACTCATGAGGATCGAGGAGGCTCTTGGGGACAA |
| -2,52 | 4,9E-09 | NM_001008 | RPS4Y1 | CAACTTTATCAAATTTGATACAGGCAATTTGTGTATGGTGATTGGTGGAGCCAACCTCGG |
| -2,52 | 3,8E-10 | NM_001039567 | RPS4Y2 | TGTTGGTGTGATCACAAACAGGGAAAGACATCCTGGTTCTTGCGATGTGGTACATGTGAA |
| -2,56 | 1,4E-17 | NM_033118 | MYLK2 | CAGGAGAATTAGGAAGGCCATGGGGCAGCCTCCAGTCTGCTCTCAGCTTGTGCCTTGTAA |
| -2,56 | 3,6E-20 | NM_006789 | APOBEC2 | ACAGCCTCAGACCCGAGGTTTAGATTTCTGAAATATGCATTTTATGTTAAGTTGGGTATT |
| -2,57 | 2,7E-09 | NM_004543 | NEB | TCTGCAGGACTTACAGATCCTGCAGTCAATGTTTCGGTTTAGACTCTCCACTGTTACCTA |
| -2,67 | 1,4E-10 | BC107798 | TNNT1 | TCCGAGCGTAAGAAGCCTCTGGACATTGACTACATGGGGGAGGAACAGCTCCGGGAGAAA |
| -2,69 | 7,0E-21 | NM_144994 | ANKRD23 | ACACACCTTTTTATACCAATCAGTATCCTCTGTTCATTAAAACTGGCTATCCATTAAAAA |
| -2,71 | 2,8E-13 | NM_000366 | TPM1 | GTGCTTTCTATTGTACAGAAGCTCTTCGTTTCAGTGTCAAATAAACACTGTGTAAGCTAA |
| -2,75 | 3,7E-12 | NM_006757 | TNNT3 | GACAAGGCCAAGGAGCTCTGGGAGACCCTGCACCAGCTGGAGATTGACAAGTTCGAGTTT |
| -2,77 | 7,1E-13 | NM_003280 | TNNC1 | CAGCGGCACGGTGGACTTTGATGAGTTCCTGGTCATGATGGTTCGGTGCATGAAGGACGA |
| -2,98 | 3,1E-11 | NM_003279 | TNNC2 | GACTTCGACGAGTTCCTGAAGATGATGGAGGACGTGCAGTAAGGAGTGGACAGTCGCCTC |
| -3,14 | 2,9E-16 | NM_005181 | CA3 | AGACAGCAAGAATTGAGCTAATAATATGTTTTAACTCTTAACACCAGCAAGAAGTCAGTC |
| -3,20 | 4,1E-14 | NM_003673 | TCAP | AGGCAAGGATGGTGGGGCCCCCAGCACCCTCTGTTAGTGCCGCAATAAATGCTCAATCAT |
| -3,24 | 1,4E-20 | NM_014332 | SMPX | GTGTGGTTTTGAGGAGGGATATGATTTTATGGAGAATGATATGGCAATGTGCCTAACGAT |
| -3,27 | 2,3E-13 | NM_133378 | TTN | CTGACAACCCTGATCATCATGGACGTACAGAAACAAGATGGTGGACTTTATACCCTGAGT |
| -3,29 | 5,8E-15 | NM_019885 | CYP26B1 | TTTGTTTTGATATGAAACTGGTACCGTGTGAGTGTTTTTGCTGTCGTGGTTTTAATCTGT |
| -3,31 | 1,8E-14 | NM_000432 | MYL2 | AAGGAGGCTCCGGGTCCAATTAACTTTACTGTGTTCCTCACAATGTTTGGGGAGAAACTT |
| -3,52 | 5,2E-18 | NM_006063 | KBTBD10 | GCTGGGATGTTGAAGGAAATACGTTATGCTTCAGGAGCTAGTTGCCTAGCAACACGTTTA |
| -3,52 | 6,5E-17 | NM_206819 | MYBPC1 | AGGATGCCACCATGACTAAAGAGAGTGCAGTGATCGCCAGGGATGGTAAAATCTACAAAA |
| -3,64 | 6,1E-20 | BX647448 | CMYA3 | TTTGAGGAACTTGATGTAAACATGGTGTTCAGAAATCTCGTGTCTATCTCAATGGGATAT |
| -3,69 | 7,6E-16 | NM_013292 | MYLPF | GAGGAGTTGGATGCCATGATGAAGGAAGCCAGCGGTCCCATCAACTTCACCGTCTTCCTG |
| -3,81 | 2,8E-14 | NM_198060 | NRAP | GGCCAGAGAGGAAGTTTGTTCACCAGAGACAGGCTTCAGATGGCTTTGATTTCGGCAAGC |
| -3,96 | 8,9E-17 | NM_003282 | TNNI2 | GGAGCGGGACCTGCGAGACGTGGGTGACTGGAGGAAGAACATCGAGGAGAAGTCTGGCAT |
| -4,25 | 1,4E-20 | NM_001103 | ACTN2 | TCCCATCAGAATGCAATAAAAGCGGAAGTCACAGTTTGTTTCCTGGAAACTTTGACAAGC |
| -4,33 | 5,5E-18 | NM_003476 | CSRP3 | TATGTGTTTCTCCTCAGAAGTGATCAGGTCTTTACTGAATGTTAGAAGAGGCCTTTGGAA |
| -4,69 | 4,6E-14 | NM_005963 | MYH1 | TTATCTAACTGCTGAAAGGTGACCAAAGAAATGCACAAAATGTGAAAATCTTTGTCACTC |
| -5,01 | 1,6E-16 | NM_079422 | MYL1 | AGAAAATTGAGTTTGAACAATTTCTGCCTATGATGCAAGCCATTTCCAACAACAAGGACC |
| -5,43 | 1,7E-17 | NM_203377 | MB | GGAGTTCATCTCGGAATGCATCATCCAGGTTCTGCAGAGCAAGCATCCCGGGGACTTTGG |
| -8,36 | 8,1E-16 | NM_000257 | MYH7 | CCGTGACATTGGCACGAAGGGCTTGAATGAGGAGTAGCTTTGCCACATCTTGATCTGCTC |
| -12,08 | 2,6E-18 | NM_001824 | CKM | ACACTCGGAGCTTGTGCTTTGTCTCCACGCAAAGCGATAAATAAAAGCATTGGTGGCCTT |
| -12,49 | 1,1E-17 | NM_017534 | MYH2 | CGGGAGGTTCACACAAAAGTCATAAGTGAAGAGTGATCATGTCCTGATGCCATGGAATGA |
| -15,31 | 2,5E-18 | NM_001100 | ACTA1 | CCGCAGTCACTTTCTTTGTAACAACTTCCGTTGCTGCCATCGTAAACTGACACAGTGTTT |

**Table 4. Transcripts displaying a more then 2 fold differential expression between SpA and RA in synovial tissue (FC: fold change).**

| **FC** | **p.value** | **Systematic Name** | **Gene** | **Probe Sequence** |
|---|---|---|---|---|
| 7,14 | 1,0E-21 | NM_001192 | TNFRSF17 | GATCTCTTTAGGATGACTGTATTTTTCAGTTGCCGATACAGCTTTTTGTCCTCTAACTGT |
| 6,97 | 4,7E-18 | AY172962 | AY172962 | AAGATAGCAGCCCCGTCAAGCGGGAGTGGAGACCACCACACCCTCCAAACAAAGCAACAA |
| 6,96 | 1,0E-21 | NM_144646 | IGJ | TTGGGTGATGTAAAACCAACTCCCTGCCACCAAAATAATTAAAATAGTCACATTGTTATC |
| 6,28 | 1,9E-19 | NM_006235 | POU2AF1 | TTTTCTGGGAAATGACTTTTCTGGGAAATGACAGTTTCTTTGACATATTTTCTTTGCCCA |
| 5,80 | 1,1E-19 | B1521983 | B1521983 | GAAGTCACTTATGAGACACACCAGTGTGGCCTTGTTGGCTTGAAGCTCCTCAGAGGAGGG |
| 5,72 | 5,5E-19 | AW136683 | AW136683 | TCTGTCATAAGTGTAGCAGGTCTCTGTAGCACTGTCTTCATCACAGATATTGCTCTGGGT |
| 5,66 | 5,6E-18 | X57802 | X57802 | ATTCTCTGGCTCCAACTTTGGGAACACAGCCACTCTGACCATCAGCGGGACCCAGGCTAT |
| 5,47 | 1,1E-17 | NM_020070 | lGLL1 | TCCAAGCCAACAAGGCTACACTGGTGTGTCTCATGAATGACTTTTATCCGGGAATCTTGA |
| 5,33 | 1,4E-17 | NM_001013618 | CTA-246H3.1 | AACAAGGCCACACTGGTGTGTCTCATGAATGACTTCTATCTGGGAATCTTGACGGTGACC |
| 5,15 | 9,4E-18 | NM_014792 | KlAA0125 | CCATTTTAAAGATGGCTACTTAGGACCATATGGATGTTGTACTGATGTCATTTGACCACG |
| 5,15 | 1,0E-21 | NM_198440 | DERL3 | AGGCCTAAGAGGCTTCTGGCAGCTTCCATCCTACCCATGACCCCTACTTGGGGCAGAAAA |
| 4,92 | 1,3E-18 | NM_031281 | FCRL5 | TGGCATATGGACTGAAAGAAACTATGCTATTGGATCTCCTGGATCTCCAGCTTGCTGACT |
| 4,80 | 4,2E-16 | ENST00000216649 | ENST00000216649 | CTCAAGAGTCGAGTCACCATATCAGTGGACACGTCCAAGAACCAGTTCTCCCTGAGGCTG |
| 4,70 | 1,0E-19 | NM_006419 | CXCL13 | CAAGAGGCAAAGGAATCCATGTAGTAGATATCCTCTGCTTAAAAACTCACTACGGAGGAG |
| 4,67 | 1,3E-18 | ENST00000377233 | ENST00000377233 | TTGTATTGGTACCTGCAGAAGCCAGGCCAGCCTCCACAGCTCCTGATCTATGAAGTTTCC |
| 4,53 | 2,8E-14 | NM_016459 | PACAP | GGACATGTTTGCACTACTTGGGGGAGTTTGGAGAAGACCAGATCTATGAAGCCCACCAAC |
| 4,48 | 9,4E-18 | AF076205 | AF076205 | CCTCCAGTCTGAGGATGAGGCTGACTATTACTGTCAAACCTGGGGCACTGGCATTGGGGT |
| 4,39 | 3,1E-17 | ENST00000327926 | ENST00000327926 | TATTTGAATTGGTACCTGCAGAAGCCAGGCCAGTCTCCACAGCTCCTGATCTATGAGGTT |
| 4,38 | 0,0E+00 | NM_006228 | PNOC | GCCACTGCCATAACTTGTTTGTAAAAGAGCTGTTCTTTTTGACTGATTGTTTTAAACAAC |
| 4,35 | 5,4E-14 | BC073764 | IGKC | TCAGGGTATTAGCAGCTGGTTAGCCTGGTATCAGCAGAAACCAGAGAAAGCCCCTAAGTC |
| 4,29 | 4,3E-19 | ENST00000379877 | ENST00000379877 | GTGCAGCTGCAGGAGTCGGGCCCAGGACTGGTGAAGCCTCCGGGGACCCTGTCCCTCACC |
| 4,27 | 3,1E-16 | BX110985 | BX110985 | CTCTGTGGGGTAGAGATGAAAAGGCTCACTCAGTTCCAGCCCCTGCAGATCCCCCAGGAC |
| 4,24 | 7,2E-17 | BC070333 | IGHV1-69 | CAGGCCCCTGGACAAGGGCTTGAGTGGATGGGAAGGATCATCCCTATCCTTGGTATAGCA |
| 4,19 | 0,0E+00 | THC2347909 | THC2347909 | GGAAATGATGTTTGTCTAAAATGGCCAGAGACAACTTTTATAAGCTTCCAGGAAGTGGAG |
| 4,16 | 1,4E-11 | ENST00000377226 | ENST00000377226 | GAAAGCTCCTAAGCTCCTGATCTATGATGCCTCCAGTTTGGAAAGTGGGGTCCCATCAAG |
| 4,15 | 7,0E-20 | NM_000582 | SPP1 | TTCCACAGCCATGAATTTCACAGCCATGAAGATATGCTGGTTGTAGACCCCAAAAGTAAG |
| 4,10 | 5,9E-11 | ENST00000259219 | ENST00000259219 | TGATCTATGCTGCATCCAGTTTGCAGTCGGGGGTCCCATCTCGGTTCAGTGGCAGTGGAT |
| 4,08 | 4,5E-17 | ENST00000312946 | ENST00000312946 | GGGCAGTCTCCACAGCTCCTGATTTATAGGGTTTCCAATCATCTTTCTGGGGTCCCAGAC |
| 4,06 | 7,3E-15 | BC012876 | BC012876 | TCTGACACCTCAGCCTCCCTGGCCATCACTGGACTCCAGGCTGAAGATGAGGCTGATTAT |
| 3,86 | 4,3E-18 | NM_014479 | ADAMDEC1 | TGACTTAGTTCTGCCCTTTGGAGAACAAAAGAAAGCAGTCTTCCATCAAATCACCTTAAA |
| 3,80 | 0,0E+00 | NM_002460 | lRF4 | TTTTGTATTGATTTTCACAGCTTTGAGGAACATGCATAAGAAATGTAGCTGAAGTAGAGG |
| 3,75 | 2,3E-17 | NM_012390 | SMR3A | CACCCTATGGTCCAGGGAGAATTCAATCACACTCTCTTCCTCCTCCTTATGGCCCAGGTT |
| 3,66 | 1,3E-14 | AF063695 | AF063695 | CTGTCAGGTGTGGGATAGTACTAGTGATCATTATGTCTTCGGAACTGGGACCAAGGTCGC |
| 3,63 | 1,2E-20 | BC009479 | TTLL3 | GCTATATCCGCTTTTCCACGCAGCCCTTCTCCCTGAAGAACCTGGACAAGTGAGCCCCTC |
| 3,63 | 9,1E-18 | X57818 | X57818 | ACCCTCAGTGTCCGTGTCCCCAGGACAGACAGCCAGCATCACGTGCTCTGGACATAAATT |
| 3,63 | 4,0E-21 | AJ252276 | AJ252276 | AGATGGTGAAACAATATACGCAGAGAAGTTCCAGGGCAGAGTCACCATAACCGCGGACAC |
| 3,62 | 7,1E-14 | AY998685 | AY998685 | AATAAGAACTACTTAGTTTGGTACCAGCAAAGACCAGGACAGCCTCCTAAAATGCTCATT |
| 3,55 | 4,8E-20 | NM_017773 | LAX1 | TCGCACATGACTGTTTATAGCAGCTTCATTCATAATTACCAAAAACTAGAAATAACCCAG |
| 3,54 | 8,5E-13 | A_24_P384604 | A_24_P384604 | GGTTCAATGGCAGTGGGTCAGGCACTGATTTCACACTGAAAATCAGCCGGGTGGCTGAAG |
| 3,53 | 1,8E-10 | ENST00000328419 | ENST00000328419 | AGCCTGATGCCTGAACAGTGGAGATCCCGCAGCAGCTACAACTGCTGGGCCATGCATAAA |
| 3,51 | 2,6E-13 | ENST00000379884 | ENST00000379884 | ACACAGTCTACATGGAGCTGAGAAGCCTGAGATCTGAGGACACGGCCGTGTATTACTGTG |
| 3,51 | 5,1E-14 | ENST00000331195 | ENST00000331195 | ATACTGGTACCAGCAGCTCCCAGGAACGGCCCCCAAACTCCTCATCGAAAGGAATAATCA |
| 3,50 | 5,9E-13 | ENST00000360623 | ENST00000360623 | TCTCAGTTATTTATAGCTGTGGTAGCACATACTACGCAGACTCCGTGAAGGGCCGATTCA |
| 3,47 | 5,3E-15 | THC2428956 | THC2428956 | CATAGTTATAACCACCAACGTCACTGCTGGTTCCAGTGCAGGAGATGGTGATCGACTGTC |
| 3,46 | 6,5E-11 | ENST00000295410 | ENST00000295410 | GACAGAGTCACCATCACTTGTCGGGCGAGTCAGGGAATTAGCAATTATTTAGCCTGGTTT |
| 3,45 | 6,1E-14 | AJ399872 | AJ399872 | CAGTTTATTACTGTCAGCAGCGTCGCAATTGGCCTTCTTTCGGCGGAGGGACCCAGCTCA |
| 3,42 | 1,2E-15 | D83692 | D83692 | ACAACAGAATACGCCGCGTCTGTGAAAGGCAGATTCACCATCTCAAGAGATGATTCCAAA |
| 3,41 | 2,9E-17 | ENST00000361266 | ENST00000361266 | TGCAATCTGGGTCTGAGTTGAAGAAGCCTGGGGCCTCAGTGAAGGTCTCCTGCAAGGCTT |
| 3,35 | 3,1E-16 | ENST00000360329 | ENST00000360329 | ACATTGGGAAGGGATGGAGGATGGACGGAGGAAGGACGTGCAGTTGCAGCTCTTTCTGCA |
| 3,35 | 1,0E-21 | NM_018456 | EAF2 | CAGGATTCCTGATATAGATGCCAGTCATAATAGATTTCGAGACAACAGTGGCCTTCTGAT |
| 3,30 | 2,0E-11 | ENST00000379895 | ENST00000379895 | ATGGTATGATGGAAGTAATAAATACTATGCAGACTCCGTGAAGGGCCGATTCACCATCTC |
| 3,27 | 2,0E-14 | BC023973 | IGLV6-57 | AACTCTGCCTCCCTCACCATCTCTGGACTGAGGACTGAGGACGAGGCTGACTACTACTGT |
| 3,27 | 8,0E-15 | XM_372630 | LOC390712 | CTGTGAAGGGCAGATTCACCATCTCCACAGACAACTCAAAGAACACGCTCTACCTGCAAA |
| 3,26 | 2,5E-16 | AF103312 | AF103312 | AATGAACAGCCTGAAAACCGAGGACACGGCCATGTATTACTGTACTAGCGCGCCAAATAT |
| 3,25 | 4,9E-18 | NM_006875 | PlM2 | GGTGAGGGGACCCTACTTTGTTATCCCAAGTGCTCTTATTCTGGTGAGAAGAACCTTAAT |
| 3,25 | 6,4E-16 | AB063923 | AB063923 | CCTCAGTGAAGGTCTCCTGCAAGGCTTCTGGTTACACCTTTACCAGTCATGGTATCACCT |
| 3,24 | 7,8E-12 | NR_001564 | XIST | AATATTTCAATGCCTATTCTCTGCAAGGTACTATGTTTCGTAAATTAAATAGGTCTGGCC |
| 3,22 | 1,8E-13 | ENST00000328018 | ENST00000328018 | CAATAGCCTGGAAGCTGAAGATGCTGCAACGTATTACTGTCATCAGAGTAGTAGTTTACC |
| 3,22 | 2,8E-11 | ENST00000359488 | ENST00000359488 | TAGGAGACAGAGTCACCATCACTTGCCGGGCAAGTCAGACCATTAGCAGCTATTTAAATT |
| 3,21 | 1,2E-11 | BX640624 | IGHA1 | TCGGTGATCAGCACTGAACACAGAACTCACCATGGAGTTTGGACTGAGCTGGGTTTTCCT |
| 3,21 | 1,5E-11 | ENST00000283657 | ENST00000283657 | GGCCACCATCAACTGCAAGTCCAGCCAGAGTGTTTTATACAGCTCCAACAATAAGAACTA |
| 3,20 | 1,5E-12 | AF471475 | AF471475 | ACATTAGTGATAGTGGTAGTACCATGTATTACGCAGACTCTGTGAAGGGCCGATTCACCA |
| 3,19 | 9,1E-15 | BC095489 | IGKC | ACATCCAGATGACCCAGTCTCCATCCTCCCTGTCTGCATCTGTAGGAGGCAGAGTCACCA |
| 3,18 | 3,2E-11 | XM_942451 | LOC652791 | GCACAAGCTACGCAGACTCCATGAAGGGCCAATTCACCATCTCCAGAGACAATGCTAAGA |
| 3,18 | 9,5E-16 | L04336 | L04336 | GTTTCCTGCAAGGCTTCTGGATACACCTTCACTAGCTATGCTATGCATTGGGTGCGCCAG |
| 3,17 | 1,1E-17 | NM_017709 | FAM46C | CCTATCCACATCTTTCCAAGATAGACACTAACATGTCATGTCCCAAACATTAGCACGTGG |
| 3,17 | 1,0E-21 | NM_000096 | CP | TCACGGCCATAGCTTCCAATACAAGCACAGGGGAGTTTATAGTTCTGATGTCTTTGACAT |
| 3,17 | 1,6E-11 | BC034142 | IGKV1-5 | GATTTTACACTGAAAATCAGCAGAGTGGAGGCTGAGGATGTTGGGGTTTATTACTGCATG |
| 3,15 | 2,8E-19 | NM_033014 | OGN | AACTAATGATCACAGCTATTATACTACTTTCTCGTTATTTTGTGTGCATGCCTCATTTCC |
| 3,12 | 5,9E-19 | NM_012261 | C20orf103 | AGAAAACGACTAATGTAACTATGCAGAGTTGTTTGGACTTCTTCCTGTGCCAGGTCCAAG |
| 3,11 | 6,7E-15 | AF035035 | LOC647460 | GTCTGAAGATTTTGCAACTTATTACTGTCAACAGTATTATAGTTTCCCTCCGACGTTCGG |
| 3,11 | 1,3E-13 | A_24_P204574 | A_24_P204574 | GAGGATGAGGCTGATTATTACTGTGCAGCATGGGATGACAGCCTGAGTGGTCCCACAGTG |
| 3,11 | 1,4E-19 | NM_033280 | SEC11 L3 | TTCAAGTATGCTCTTTTGGCTGTAATGGGTGCATATGTGTTACTAAAACGTGAATCCTAA |
| 3,09 | 2,0E-21 | NM_012081 | ELL2 | TGTCTTTTCAAAGTGCTGCCAGTTGAAAAGGGAAGCATTATGTTTACAAATCTGTTTTGA |
| 3,09 | 6,5E-11 | ENST00000379879 | ENST00000379879 | CTTCAAATGAACAGCCTGAGAGCCGAGGACACGGCCGTGTATTACTGTGCGAGAGACACA |
| 3,08 | 2,7E-18 | NR_001442 | FER1L4 | GAAGTCTTTCTTACCCATGTGAGCTACCCCAGAGTCTAGTGCTTCCTCTGAATAAACCTA |
| 3,06 | 2,3E-08 | NM_001040077 | IGHG1 | TGAATGGCAAGGAGTACAAGTGCAAGGTCTCCAACAAAGCCCTCCCAGCCCCCATCGAGA |
| 3,06 | 5,6E-15 | AF267875 | AF267875 | TGAGGACGAGGCTGACTACTACTGTCAGTCTTATGATAGCAGCATCGGGAATGTGATATT |
| 3,03 | 5,1E-14 | U21252 | U21252 | CCAGAGATGATTCCAAGAACACGGCGTATCTGCAAATGAACAGCCTGAAAACCGAGGACA |
| 3,03 | 1,0E-08 | NM_005570 | LMAN1 | TTGACTACCATTTTCCTGTGTACTTCATCTATTTGTGTACAAAATGATGTCGTTTTGAGG |
| 3,03 | 2,7E-17 | NM_001006946 | SDC1 | ACCCTGGGCCCTGGGCTGGAATCAGGAATATTTTCCAAAGAGTGATAGTCTTTTGCTTTT |
| 3,02 | 7,4E-10 | Y11328 | Y11328 | GTGGCACATACTATGCAGACTCCGTGAAGGGCCGATTCACCATCTCCAGAGACAATGCCA |
| 3,02 | 8,6E-11 | ENST00000360102 | ENST00000360102 | TGGGTCCGCCAGGCTCCAGGGAAGGGACTGGAGTGGGTTTCATACAGTAGTGGTAATAGT |
| 3,01 | 1,4E-15 | A_24_P203886 | A_24_P203886 | ATCTCCTGCAGGTCTAGTCGCAGCCTCCTGCATAGTAATGGAAACACCTATTTACATTGT |
| 3,00 | 3,9E-18 | NM_006398 | UBD | ATTTGGGTGGGATGGGTAGGATGAAGTATATTGCCCAACTCTATGTTTCTTTGATTCTAA |
| 3,00 | 9,3E-10 | ENST00000383048 | ENST00000383048 | AGTTTGGGCTGAGCTGCCTTTTTCTTGTGGCTATTTTAAAAGGTGTCCAGTGTGAGGTGC |
| 2,99 | 5,7E-18 | NM_005079 | TPD52 | AACTGCTTACTCAACACTACCACCTTTTCCTTATACTGTATATGATTATGGCCTACAATG |
| 2,99 | 2,0E-16 | X01147 | X01147 | TGGGCATCACCGGACTCCAGACTGGGGACGAGGCCGATTATTACTGCGGAACATGGAATA |
| 2,98 | 8,9E-14 | NM_000450 | SELE | GATGTGATAAGGATCAGAACAGCAGAGGTTCTTTTAAAGGGGCAGAAAAACTCTGGGAAA |
| 2,97 | 4,2E-19 | AF343666 | AF343666 | GGAGGAACCTCTTGGAGAAGCCAGCTATGCTTGCCAGAACTCAGCCCTTTCAGACGTCAC |
| 2,97 | 4,4E-15 | AK026408 | AK026408 | TACTGTGGTACATGGCACAGCAACTCTAAGACTCACACAGTGCTCCAGACCCATGAGGAA |
| 2,96 | 1,9E-15 | THC2321023 | THC2321023 | GGTGCCTTCCAGCCTGACCCCTCCTCTGAGCACCACCTGCCAGTGTGACAGCTCCATCTG |
| 2,94 | 2,6E-10 | ENST00000307840 | ENST00000307840 | GCAGCCTGCAGCCTGAAGATATTGCAACATATTACTGTCAACAGTATGATAATCTCCCTC |
| 2,94 | 3,8E-14 | AF067420 | IGHA1 | TCTATGATAGTAGTGGTCCCTTTGACTACTGGGGCCAGGGAACCCTGGTCACCGTCTCCT |
| 2,94 | 1,4E-12 | AY003763 | AY003763 | GCTACAAGTTTGACTACTGGGGCCAGGGAACCCTGGTCACCGTCTCCTCAGCATCCCCGA |
| 2,93 | 6,3E-14 | BC008026 | MGC16025 | GCCTCTGACCCTGATGAAATAGCTTCGGTGGCATTTGCATCAAGATCATGTTAGTGTCAT |
| 2,92 | 2,4E-13 | BC032451 | BC032451 | AGCAGCTCCTTAGCCTGGTACCAGCAGAAACCTGGCCTGGCGCCCAGGCTCCTCATCTAT |
| 2,92 | 7,7E-15 | ENST00000354689 | ENST00000354689 | CTGTGAAAGGCAGATTCACCATCTCAAGAGTTGATTCAAAAAACACGCTGTATCTGCAAA |
| 2,92 | 3,2E-15 | ENST00000358917 | ENST00000358917 | AGGGGAAGGACTGTGTCCCTGTGTGATGCTTTTGATATCTGGGGCCAAGGGACAATGGTC |
| 2,91 | 4,1E-11 | AB063751 | AB063751 | CTGGATTCATCTTCAGTAATTACACCATGAACTGGGTCCGCCAGGCTCCAGGGAAGGGGC |
| 2,89 | 7,0E-21 | NM_000439 | PCSK1 | GAGTTTAACATGTGTGGTCTTGGTATTCTTAAGGGAACTTCCACATTATACATTTGATGT |
| 2,88 | 5,2E-16 | BC022362 | | CAGCAGAGGCCAGGCCAATCTCCAAGGCGCCTAATTTATAAGGTTTCTAACTGGGACTCT |
| 2,87 | 6,8E-16 | NM_001040075 | LOC651928 | TGCTAATGCTCTGGGTCCCTGGATCCAGTGGTGATATTGTGATCGCCCAGACTCCACTCT |
| 2,87 | 8,4E-20 | NM_002281 | KRT81 | TTGAAAGACCCCTCCCACTCCTGGCCTCACATTTCTCTGTGTGATCCCCCACTTCTGGGC |
| 2,86 | 5,4E-14 | NP102468 | NP102468 | TTACCATCAGTAGCCTGGAAGCTGAAGATGCTGCAACATATTACTGTCAGCAGGGCAATA |
| 2,86 | 5,0E-21 | NM_001775 | CD38 | TGAAAAATCCTGAGGATTCATCTTGCACATCTGAGATCTGAGCCAGTCGCTGTGGTTGTT |
| 2,85 | 1,9E-11 | AF035034 | AF035034 | GCAGGGTGGAGGCTGAGGATGTTGGGGTTTATTACTGCATGCAAGGTACACACTGGCCTC |
| 2,85 | 7,9E-11 | BC024289 | BC024289 | GCCAAGAACACGCTGTATCTGCAAATGAACAGTCTGAGAGCCGAGGACACGGCTGTGTAT |
| 2,84 | 5,5E-10 | AF471454 | AF471454 | ATGCAGACTCCGTGAAGGGCCGATTCACCATCTCCAGAGACAATTCCAAGAACACGGTGT |
| 2,83 | 3,9E-13 | A_24_P110487 | A_24_P110487 | CAGGATTTTATGGTGATAGTGGTTACACAAACTACGCAGACTCTGTGAAGGGCCGATTCA |
| 2,82 | 7,4E-14 | Z18824 | Z18824 | CTCCAGGGAAGGGGCTGGAGTGGGTTTCATACATTAGTAGTAGTAGTAGTACCATATACT |
| 2,82 | 1,0E-21 | NM_002610 | PDK1 | TGGACACGAAGTGATTTTTGTAACCTGAGCAGTTAATGAATGTGCCAACATTTTCTAGGA |
| 2,82 | 5,1E-14 | NM_002922 | RGS1 | GAAGTCCCATTAACTTAAAGTATATGTTTTCAAATTGCCATTGCTACTATTGCTTGTCGG |
| 2,81 | 1,0E-13 | AJ245002 | AJ245002 | TTTGACTGGTTATTATAGAAGGGGGTTTGACTACTGGGGCCAGGGAACCCTGGTCACCGT |
| 2,80 | 1,7E-12 | XM_496145 | LOC440361 | ATTAAAAGGTGTCCAGTGTGAGGTTCAGCTGGTGCAGTCTGGGGGAGGCTTGGTACATCC |
| 2,79 | 1,4E-12 | NM_006274 | CCL19 | AGTGTGAGTGTGAGCGAGAGGGTGAGTGTGGTCAGAGTAAAGCTGCTCCACCCCCAGATT |
| 2,78 | 1,8E-08 | NM_002416 | CXCL9 | TGTGACCCACTTACCTTGCATCTCACAGGTAGACAGTATATAACTAACAACCAAAGACTA |
| 2,78 | 2,2E-09 | AK128476 | IGHA1 | TGCTGAGTTGGGTTTTCCTTGCTGCTATTTTAAAAGGTGTCCAGTGTGAGGTGCAGCTGG |
| 2,76 | 1,8E-14 | NM_021181 | SLAMF7 | GGAGACCTCCCTACCAAGTGATGAAAGTGTTGAAAAACTTAATAACAAATGCTTGTTGGG |
| 2,75 | 1,7E-10 | AJ519285 | AJ519285 | CAATTCCAAGAACACGCTGTATCTGCAAGTGAACAGCCTGAGAGTCGAGGACACGGCCCT |
| 2,75 | 2,3E-16 | BX648200 | BHLHB8 | CCCGTGCCCCCACTGTAATTGTGCTGAAAATGTTTCTCAAATGACCCAAATTGGCTCTTA |
| 2,75 | 3,4E-16 | AK000347 | LAX1 | ATTTGTAATTTTGTAAACCCTGTCTCTCATATTTTGTAAAAATACCAAAATTAGCGGTGG |
| 2,73 | 9,7E-17 | DB362335 | DB362335 | CATCTTCCATTCCTTCCCAAATTATGGAAGTAAGGTTCTTCTCACCAGAATAAGAGCACT |
| 2,72 | 5,3E-17 | NM_001770 | CD19 | TACATGCCAGTGACACTTCCAGTCCCCTTTGTATTCCTTAAATAAACTCAATGAGCTCTT |
| 2,72 | 4,6E-15 | NM_003116 | SPAG4 | CGATTTCGCGGTCTTTGGCCTCCAGGTTTATGATGAAACTGAAGTTTCCTTGGGGAAATT |
| 2,71 | 5,5E-16 | CR621698 | CR621698 | ATGTTGCTCCCCTCTAGTCACTAAGATGCCACATATTTCTACTTGAGTGAACAGATTGCA |
| 2,70 | 5,6E-07 | BC067092 | IGKC | TCACTATCAGCAGCCTGCAGCCTGAAGATTTTGCAACTTACTATTGTCAACAGGCTAACA |
| 2,69 | 2,3E-07 | ENST00000331696 | ENST00000331696 | CAGCAGCCTGCAGCCTGAAGATTTTGCAGCTTATTACTGTCAACAGAGTGACAGTACCCC |
| 2,68 | 2,6E-05 | BC030813 | BC030813 | GTGGGTCTGGGACAGAGTTCACTCTCACCATCAGCAGCCTGCAGTCTGAAGATTTTGCAG |
| 2,66 | 3,8E-15 | NM_001783 | CD79A | TGATTGTAGCAGCCTCGTTAGTGTCACCCCCTCCTCCCTGATCTGTCAGGGCCACTTAGT |
| 2,63 | 3,3E-18 | NM_012328 | DNAJB9 | ATTTCTTTCTTAGTTGTTGGCACTCTTAGGTCTTAGTATGGATTTATGTGTTTGTGTGTG |
| 2,61 | 3,4E-15 | AF035040 | AF035040 | CACCCTTAACTGGGGATACGAAAAAGATGATGCTTTTGATATCTGGGGCCAAGGGACAAT |
| 2,59 | 1,5E-18 | NM_005080 | XBP1 | CCTTTTTGGCATCCTGGCTTGCCTCCAGTTTTAGGTCCTTTAGTTTGCTTCTGTAAGCAA |
| 2,58 | 7,6E-16 | NM_001077 | UGT2B17 | TTTCCTGCGACAAAACGTCTTTTCACAACTTACCCTGTTAAGTCAAAATTTATTTTCCAG |
| 2,58 | 4,5E-16 | NM_007029 | STMN2 | TATAATGGATCATGCGATATCAGGATGGGGAATGTATGACATGGTTTAAAAAGAACTCAT |
| 2,54 | 3,1E-14 | ENST00000327436 | ENST00000327436 | CTGGAGTGGATGGGGAGCATCTATCCTGGGAACTCTGATACCAGATACAGCCCATCCTTC |
| 2,52 | 8,9E-17 | NM_152866 | MS4A1 | GAGCTCACACACCATATATTAACATATACAACTGTGAACCAGCTAATCCCTCTGAGAAAA |
| 2,48 | 4,1E-07 | ENST00000295339 | ENST00000295339 | AGCAGCCTGCAGCCTGAAGATTTTGCAGTTTATTACTGTCAGCAGGATTATAACTTACCT |
| 2,48 | 5,7E-15 | BC031698 | FLJ40330 | AAACCTTCAAGCTGAGTGCAGAAAGCGCCGTCTTTTACTAGAAGACAGTAAAAGGTTGGT |
| 2,48 | 4,8E-16 | NM_005409 | CXCL11 | TAAGAAAGGCTGGTTACCATCGGAGTTTACAAAGTGCTTTCACGTTCTTACTTGTTGTAT |
| 2,45 | 1,6E-09 | NM_003299 | HSP90B1 | AAGAGATTTTCCTGAGAGAACTGATTTCAAATGCTTCTGATGCTTTAGATAAGATAAGGC |
| 2,44 | 1,1E-13 | NM_001870 | CPA3 | GCCAAGTATATCCTCAAGCATACTTCCTAAAGAACTGCCCTCTGTTTGGAATAAGCCAAT |
| 2,40 | 1,5E-14 | ENST00000283694 | ENST00000283694 | GAGCTTGGGTGGATGGGACGGATCAACCCTAACAGTGGTGGCACAAACTATGCACAGAAG |
| 2,39 | 9,7E-18 | NM_015187 | KlAA0746 | CTGCTAAGGTAGTGAATAAATCAGTAATGCAATATTGTGGGTCCAAACTACTCTTTGCAC |
| 2,39 | 5,5E-18 | NM_021928 | SPCS3 | GAATGTCACTTTGACCCTGTCTTGGAACGTCGTACCAAATGCTGGAATTCTACCTCTTGT |
| 2,38 | 4,8E-15 | AW977527 | AW977527 | GATAAGACATCATTTCCTGCACTACTGGACACACTCAATGAGGTAACTGTTGTTGAACAA |
| 2,38 | 1,4E-06 | ENST00000310579 | ENST00000310579 | GCAGATTACACTCTCACCATCCGCAGCCTGCAGCCTGAAGATTTTGCAAATTATTACTGT |
| 2,36 | 1,8E-08 | THC2275252 | THC2275252 | GTCACCGACCCGCCCTTACTCACATGCCTTCCAGGTGCAATAAAGTGGCCCCAAGGAAAA |
| 2,36 | 1,7E-14 | NM_005065 | SEL1L | CATTATTTCAGTGTGCATAAGTTCTTAATGTCAACCATCTTTAAGGTATTGTGCATCGAC |
| 2,35 | 6,4E-11 | BC018749 | IGLV2-14 | CATCACTGGTCTCCAGGCTGAGGACGAGGCTGATTATTACTGCAGCTCATATACAAGCAG |
| 2,34 | 3,6E-08 | AY062331 | AY062331 | AGCCTGAAGATTTTGCAGTTTATTACTGTCAGCAGCGTAGCGCCTGGCCTCTCACTTTCG |
| 2,34 | 1,6E-16 | NM_016594 | FKBP11 | TTCTCACTTGGCCTATGGAAAACGGGGATTTCCACCATCTGTCCCAGCGGATGCAGTGGT |
| 2,33 | 1,7E-12 | NM_021777 | ADAM28 | CTTAGAAGCTTCGAACTCAAAATCATGGAAAGGTTTTAAGATTTGAGGTTGGTTTTAGGG |
| 2,32 | 6,2E-18 | NM_002201 | lSG20 | CAGGGACTAGAGGCTTTCGGCTTTTTGGGACAGCAACTACCTTGCTTTTGGAAAATACAT |
| 2,32 | 1,9E-17 | DQ100868 | DQ100868 | GAATGATGATAAGCGCTACAGCCCATCTCTGAAGAGCAGGCTCACCATCACCAAGGACAC |
| 2,32 | 2,4E-10 | NM_002078 | GOLGA4 | GTGAAATGGAGCAAAAAGTAAAATCTTTAACCCAAGTCTATGAGTCCAAACTTGAAGATG |
| 2,29 | 6,1E-13 | NM_144616 | JSRP1 | GAGAGGCCTCGGAGAGAGGGGAAGCCGCGGAAGGAGAAGCCGCGGAAGGAGGAGAGACCT |
| 2,28 | 2,1E-09 | NM_001884 | HAPLN1 | GCTTCGTGGGTTTCCCAGATAAAAAGCATAAGCTGTATGGTGTCTACTGCTTCAGAGCAT |
| 2,28 | 2,6E-12 | NM_003123 | SPN | TCCTCACCCACCTCTTCACTCTGAATCCTCATGAGGCTTCTCAGCCCTGGATTTCCTGCT |
| 2,28 | 1,7E-15 | BC107852 | BC107852 | GAGGAAAACAACGCCCCAGCTGGGAAGCCTGAGAACACTTAGCCTTCATGAGTGTCCCCA |
| 2,27 | 1,4E-15 | AK074473 | C20orf82 | TATCATTCTGCTAAACGGCCCCAAAACAGTAGAATTTCTGCTCATGTCCTAGCAGGTTCA |
| 2,27 | 6,9E-18 | NM_014333 | IGSF4 | GAAAGAGTACTTCATCTAGATCAGCCTTTTTGTTTCAATGAGGTGTCCAACTGGCCCTAT |
| 2,26 | 8,9E-17 | NM_022567 | NYX | GAAGTCCTTTGTTTTCTACCACAATCCTCCTCCTCCTCTCCAGGGGCCTGGAAACACTAG |
| 2,26 | 1,1E-14 | NM_015208 | ANKRD12 | TCATCCCTGAAACATCAAATTCTGATATGCAAACCAAAAAGGAATATGTAGTTTCAGGTG |
| 2,25 | 1,4E-17 | ENST00000379969 | ENST00000379969 | TTTTCAGTTGTTACAGAGCTCAGCAGCTGTGGTTGCCCCTGTTCTACACCAATTTCAGTT |
| 2,25 | 2,6E-17 | NM_198491 | FAM92B | AGGGATCTACTGGATTTTAGAGCCAAGATGCAAGGAGTTTATGGGCATTATGACACTCGG |
| 2,24 | 2,2E-15 | THC2404289 | THC2404289 | AAGCTAATGCGAAATCATTTCTCTCTGATTTACCTGAGGAGGTTAACAAGGACCCATCAA |
| 2,24 | 2,2E-09 | NM_002345 | LUM | TTTTTATTTCTACTGTCAAATGATGTGCAAAACCTTTTACTGGTTGCATGGAAATCAGCC |
| 2,24 | 5,0E-17 | NM_030926 | ITM2C | ACTCTTAAATGCTTTGTATATTTTCTCAATTAGATCTCTTTTCAGAAGTGTCTATAGAAC |
| 2,23 | 8,8E-18 | CR620293 | CR620293 | CAACAAAGGAGCGTCACTTGGATTTTTGTTTTCATCCATGAATGTAGCTGCTTCTGTGTA |
| 2,23 | 1,3E-15 | L06610 | L06610 | TGAGAGGAAAGTTCTTCACCATGGACTGGACCTGGAGGGTCTTCTGCTTGCTGGCTGTAG |
| 2,23 | 1,5E-15 | ENST00000321715 | ENST00000321715 | TCCGGCTTCTTCGTCTTCAGCCGCCTGGAGGTGACCAGGGCCGAATGGGAGCAGAAAGAT |
| 2,22 | 5,4E-15 | NM_030776 | ZBP1 | ATGTTTGAGTCCCAACAAAATTCATATCAAAACATAATCCCAACTGGGTGCAGTGGCTCA |
| 2,21 | 3,2E-15 | NM_033285 | TP53INP1 | GGGAGGTTAGATGTGTGTTTCAGGCTTGGAGTGTATGAGTGGTTTTGCTTGTATTTTCCT |
| 2,21 | 3,9E-15 | NM_006280 | SSR4 | GCCTCCTCAGGAAGGCTCAGAGGAATAACGAGGACATTTCCATCATCCCGCCTCTGTTTA |
| 2,21 | 2,2E-14 | NM_182568 | FLJ36492 | CTCATAGGCATCAGAAAGAAAAGGATCTCTTTCATGAAAATTGCATGTTGCAGGAAGAAA |
| 2,20 | 7,2E-14 | NM_013244 | MGAT4C | TAGATGTTGGGGAAAACGTTATGCCTAGCAAACAAAGGAGACAATGTTCTAGTTACTTAA |
| 2,20 | 4,6E-17 | NM_024641 | MANEA | AGATTTGTGGTTACCTATACCACGCTAGGTGTTTTGACATGTTTAGTGTTTCTGCTTTAC |
| 2,18 | 2,8E-15 | NM_001012978 | NGFRAP1L1 | TCTTGAGGTTAATAATCATAAAATCCCTGCTTTCTAAATTCGCATTTTTCCTGGTGTACC |
| 2,18 | 6,9E-09 | ENST00000322032 | ENST00000322032 | GTAAACCCACCCACATCAATGTGTCTGTTGTCATGGCGGAGGCGGATGGCACCTGCTACT |
| 2,18 | 3,7E-15 | NM_014383 | ZBTB32 | AGGAGCGAAGGTTAACAGTAGGGGAGATTGTCGATCTCATCACCATAATAAAGAGTTTCC |
| 2,18 | 5,1E-14 | NM_013250 | ZNF215 | AAGTTGCAGTTCCTAAGAACCTATTGATGATGTTCAGTGCAGACTTACTGTACCTTTGGG |
| 2,17 | 9,4E-13 | NM_013377 | PDZRN4 | TCGGTAGAGTATGATTGCCTCGTTCAATGTGGCGTTTTTATATATATTTTGTGACTCTTT |
| 2,17 | 1,1E-15 | NM_005084 | PLA2G7 | AAAGCATTTAGGACTTCATAAAGATTTTGATCAGTGGGACTGCTTGATTGAAGGAGATGA |
| 2,17 | 6,6E-12 | THC2339566 | THC2339566 | CTGGATCATAGGTTTTGAGTTCATAATCCAGTAATAACAGCTTTCAGCTTTCTATGAGTA |
| 2,17 | 9,0E-14 | NM_032855 | HSH2D | GAATCCGAGCCCTTTTCCCATATCATCTGTTTGTTCTGTTGTCTAAAAGCACACTGCAAG |
| 2,16 | 1,3E-13 | NM_000867 | HTR2B | TCTACTAGATACGCTTCTCCTCACTGAAAATGAAGGTGACAAAACTGAAGAGCAAGTTAG |
| 2,16 | 5,3E-15 | NM_000626 | CD79B | CGCTGAAGGATGGTATCATCATGATCCAGACGCTGCTGATCATCCTCTTCATCATCGTGC |
| 2,16 | 1,1E-11 | NM_000092 | COL4A4 | TCCTAAAAGTTCAACCTGTTCATCTTGAACTTGGCCTGAGAACATTTTCTGGGAAGAGGT |
| 2,16 | 1,6E-15 | NM_024563 | C5orf23 | TGTTTGGATATAAATGTGTATGTGTCCTTGTAAATGTTTCTATCAAGCAAGAATGCCACG |
| 2,16 | 3,2E-12 | AK025047 | AK025047 | CCTCTCACACCCCTCCAACCTCATTTTGAGAGAACAATTCACATTTTAATTTTTGATGGC |
| 2,15 | 1,7E-17 | AK098753 | C22orf35 | TGGCTGAGATGATACCCGACCCTCTAGGGAAATTCTTAGAGTAACTTCTAGGAAATGTCA |
| 2,13 | 3,6E-16 | NM_022136 | SAMSN1 | CTCTGGTTGCTATATCTCATCAGGAAATTCAGATAATGGCAAAGAGGATCTGGAGTCTGA |
| 2,12 | 1,5E-14 | NM_152446 | C14orf145 | GGCCAAATATCCTACTGTGATCACTTCAGTCATATCCTGGACCGTCTTTGGTTTACTGTG |
| 2,12 | 1,8E-15 | THC2287925 | THC2287925 | GTCCCTTCTCCCTAACACTCCAGTTCAAGATAGCAACCTAAATTCTGAGTTGATTATTCT |
| 2,12 | 1,5E-14 | NM_003292 | TPR | ATGATGTTTCTCTTGCATCAACTCCAAAACGTCCAAGTACATCACAGACTGTTTCCACTC |
| 2,12 | 1,6E-09 | NM_004887 | CXCL14 | GTGGTACAACGCCTGGAACGAGAAGCGCAGGGTCTACGAAGAATAGGGTGAAAAACCTCA |
| 2,12 | 7,9E-13 | NM_012108 | BRDG1 | CCATACAATTTCATTATCTTATCAGAATGAAACCAATTCACAGGGAAACTAGAGACTACG |
| 2,11 | 2,0E-14 | NM_001362 | DIO3 | AAGTTGGGGTGTCTGCTTTGGGACCAGAGGAAGATAGCTTGAGAGGCATTGGCGAGGTTC |
| 2,11 | 1,7E-06 | ENST00000379913 | ENST00000379913 | CACAAGCCCAGCAACACCAAGGTGGACAAGAGAGTTGAGTCCAAATATGGTCCCCCATGC |
| 2,11 | 2,9E-16 | NM_022154 | SLC39A8 | GGAAAATGATTGACAAAGCCCAACAATGATCTCAGGAATTACATTTTCCAACAGACCAAA |
| 2,11 | 3,7E-10 | THC2380963 | THC2380963 | TATTGAGAGGGGCCTCGACCCCTCCTCATCCCCTCAGTGAAAACAATAAAGACAAATTTT |
| 2,11 | 3,1E-11 | NM_002927 | RGS13 | ATTAACAAACTTCTTTATCACATGTATCTTCTACATGTAAAACATTTCTGATGATTTTTT |
| 2,10 | 2,2E-12 | NM_001890 | CSN1S1 | CCTATGCTGTTTGGTACTATCCACAAATCATGCAGTATGTTCCTTTCCCACCGTTTTCCG |
| 2,10 | 1,9E-17 | NM_018284 | GBP3 | GAGATGCCAAGGTGAAAGTACCCAACTTCAAAATGAGATACAAAAGCTACAGAAGACCCT |
| 2,10 | 1,7E-16 | NM_014398 | LAMP3 | ACCATGTTGACTTTCCTCATGTGTTTCCTTATGACTCAGTAAGTTGGCAAGGTCCTGACT |
| 2,09 | 2,8E-16 | NM_002009 | FGF7 | GGAGGACTTAGTTTTTCATATGTGTTTCCTTAGTGCCTAGCAGACTATCTGTTCATAATC |
| 2,09 | 1,4E-12 | CR594705 | CR594705 | ATCCACTCTCTAGAGTCCAGTGTACTTTAGACTTCATCTGAGTCCAATACATGTACCACA |
| 2,09 | 5,6E-14 | NM_003810 | TNFSF10 | GCAACAATCCATCTCTCAAGTAGTGTATCACAGTAGTAGCCTCCAGGTTTCCTTAAGGGA |
| 2,08 | 4,2E-10 | NM_152687 | FLJ33641 | CCCAAATGCCTAGAACATCACATAAGGCACTAAATGCCTCATGTTTTACTGACGGGAATT |
| 2,07 | 2,2E-15 | XM_926222 | LOC441368 | AGAGTTCTACATTTATTCCAGCATTACCAGGTGAAACTCTCACTTACCTATGGAAAATCC |
| 2,07 | 5,3E-13 | NM_003558 | PlP5K1B | TTCCTATGGACTTTTGCATTATTTCATTGTGCATGCATCCAGTGATTATACATAAGCAAC |
| 2,06 | 7,3E-09 | AW979273 | AW979273 | GGATATTTGGTATGCTGGCAATGGTAATTCAAATTGGCGTTTAAGAATAATCTTGGCCGG |
| 2,06 | 7,9E-11 | NM_005711 | EDIL3 | ACATGACTGCCTATCAGTAGATTGATCTGTATTTAATATTCGTTAATTAAATCTGCAGTT |
| 2,06 | 1,2E-12 | NM_000139 | MS4A2 | TCAATGTCATCTTCTCCATGAAGACCACTGAATGAACACCTTTTCATCCAGCCTTAATTT |
| 2,06 | 4,4E-14 | NM_003037 | SLAMF1 | AGGCGCAGAACAGAGCGTTACTTGATAACAGCGTTCCATCTTTGTGTTGTAGCAGATGAA |
| 2,06 | 2,1E-16 | NM_014674 | EDEM1 | TCCTTGCACACTTCAGTGTTTCTCTCCTGTTCAATAAAATGCCCTGTTAAGGTATAATTT |
| 2,06 | 2,9E-13 | NM_000587 | C7 | CAGATACAAACTATTTCTATCCTGAGTAGTAATCTCACACTTCATCCTATAGAGTCAACC |
| 2,04 | 2,4E-10 | THC2314833 | THC2314833 | AGGCATTGAAGGCCACGAGTTACATTTGTTGGCAAAGATGAGTATGCAAGTCAACAAGTC |
| 2,04 | 6,1E-07 | NM_022137 | SMOC1 | TATTCTCTCTCTTATTGTAAGTTTTTGGATCTGCTACTGACAACTTTTAGAGGGTTTTGG |
| 2,04 | 6,8E-16 | AF124170 | AF124170 | AAGCAGAACCAACATCGCAAGTAATACTGTGAACTGGTACCAGCACCTCCCAGGAATCGC |
| 2,03 | 1,2E-09 | NM_018084 | KlAA1212 | TACTGGAACAGGAAAATGAACATCTGAATCAAACAGTGTCTTCCTTAAGGCAGCGGTCCC |
| 2,03 | 2,6E-14 | BC042469 | LOC644846 | AAATGTGAAGTCTGGCTTTGAAGAGGGTGTATAACACACATAATTTACTGTGCATCAGTC |
| 2,03 | 2,7E-09 | NM_006475 | POSTN | AGGAAGTTGCAAGCCAACAAAAAAGTTCAAGGATCTAGAAGACGATTAAGGGAAGGTCGT |
| 2,02 | 6,9E-14 | NM_006533 | MIA | CTGGCAAAGTCGATGTGAAGACAGACAAATGGGATTTCTACTGCCAGTGAGCTCAGCCTA |
| 2,02 | 5,1E-10 | NM_201269 | ZNF644 | TTGGAAAGACGAAATGGGATGCTCACAAATCTCCAATCTGGGTCTGAATGAGATGATGCA |
| 2,01 | 9,2E-17 | NM_001951 | E2F5 | GCACTTTAAGTTTATCACATTTTGTTGACTTCTGACATTCCACTTTCCTAGGTTATAGGA |
| 2,01 | 4,3E-11 | NM_020939 | CPNE5 | TGGTTCTGTGCCCGTCTCTGAGACAGTCTCTGTGTGGAATTTGCCTTAAACTGAAGTAAA |
| 2,01 | 1,0E-15 | NM_016570 | ERGIC2 | AGAAAGGGAACGTATCATTAACCATGCTGCAGGCAGCCATGGAGTCTCTGGGATATTTAT |
| 2,00 | 2,9E-13 | NM_181453 | GCC2 | AAGAACCTTCAAGAAAAGAATGGAGTATACTTACTTAGTCTCAGTCAAAGAGATACCATG |
| 2,00 | 3,1E-12 | NM_144590 | ANKRD22 | AATCCTTGTGACCACACCGATGGAGATACAGAAAAAGTTAACGACTGGATTCTATCTTCA |
| 2,00 | 2,7E-13 | NM_006495 | EVl2B | CACAAAACGTACATCAATCATTTCACTTACACCCTGGAAACCAAGCAAAAGCACACTTTT |
| -2,01 | 8,7E-14 | NM_018723 | A2BP1 | TGACAAAACCATAAAAACCTTCCAATGTGGGGAGAAAGGAAGCTTTCCGAGGCCTGAGTA |
| -2,07 | 1,2E-10 | NM_005609 | PYGM | AAGTCAGCGCCTTGTACAAGAACCCAAGAGAGTGGACGCGGATGGTGATCCGGAACATAG |
| -2,08 | 3,5E-12 | NM_007026 | DUSP14 | GTTTAATAAACTGGTTCTGCTCTCTTCTGAATCTCATGCCTTTGGCACCTTGGTAGGTGC |
| -2,09 | 2,6E-13 | NM_004165 | RRAD | TTTTTTCACAGCCCGGGTGTGCCTGCCCTGGAGGGAGGCTCTTCAGTGCGGTAGCTATTT |
| -2,12 | 8,7E-16 | NM_032854 | COR06 | ACTGGCATGAGGAGTTAGGTTCATCACAAATACACACACACTGCCCCCAACCCTCTGCCG |
| -2,12 | 1,6E-10 | NM_014476 | PDLlM3 | AAACATACACTTAGCTATGTTTTGCAACTCTTTTTGGGGCTAGCAATAATGATATTTAAA |
| -2,15 | 1,2E-14 | NM_006308 | HSPB3 | TTCCTGTTCAGATGACATGGGGAAGATGATGGTTCAGCCACTGGTACTACGAGAATGTTT |
| -2,15 | 2,7E-15 | ENST00000332074 | ENST00000332074 | AATGGAAGACAGTTCCCGACCTTCTACCCCACAGAGATCAGCTCATGAGAATCTCATGGA |
| -2,16 | 1,0E-13 | NM_014859 | KlAA0672 | TTTTGATGTTGCAGCTTTGCTCACTTCCTGGCAAGGGCAGGTCATGCCTCAATTTGTAAT |
| -2,18 | 2,4E-16 | NM_002469 | MYF6 TTTTTT | GGAACTGCGAGTGGCTTAGGTCTAGCCTCATTTTGTTTTTGTTTGGTTGGTTTT |
| -2,20 | 6,3E-18 | NM_000635 | RFX2 | AAGCTCACAGTTAACGGATTGTAATGCCCGGCTTTTCCTCACTCCTGGTAACCAATACAA |
| -2,20 | 3,6E-14 | NM_002200 | lRF5 | GAGCACTTAGGTATCATATCAGATGCTCAAGGCTGGCAGCTACCCCCTTCTTGAGAGTCC |
| -2,22 | 1,7E-12 | NM_013353 | TMOD4 | AGGCCATGACCCGAAACAATGAACTACGTCGCCAGCAAAAGAAGAGATAACACTGCATTT |
| -2,23 | 4,3E-16 | BC015836 | BC015836 | GGCTGGATTGTGGGCTGCATCATAATGATTTATGTTGTCTTCTCTTAGAAAGGCAAGAAG |
| -2,23 | 8,1E-17 | NM_001007271 | DUSP13 | GATTTCCCTGACCCAATTCAGAGATTCTTTATGCAAAAGTGAGTTCAGTCCATCTCTATA |
| -2,24 | 1,2E-19 | NM_006732 | FOSB | TTTTTACAATCTGTATCTTTGACAATTCTGGGTGCGAGTGTGAGAGTGTGAGCAGGGCTT |
| -2,25 | 5,6E-14 | NM_000517 | HBA2 | CACAGACTCAGAGAGAACCCACCATGGTGCTGTCTCCTGCCGACAAGACCAACGTCAAGG |
| -2,26 | 2,7E-14 | NM_006783 | GJB6 | AGAAGACCGTGTTTACCATTTTTATGATTTCTGCGTCTGTGATTTGCATGCTGCTTAACG |
| -2,27 | 1,3E-16 | NM_021116 | ADCY1 | AGAGTGATGTGGGCAAGGGTGTCAATTTTCTCGCACAATACAAACTCACTGAGGATGCTT |
| -2,28 | 1,5E-13 | NM_080874 | ASB5 | CAGATATTAAAGCTGCTTTTGGGTAAAACAGGTTTCCACCAGTATTTTCCCTGAGCTAGA |
| -2,30 | 1,7E-14 | ENST00000284767 | ENST00000284767 | TTAACTTAAACATAGGGATAATGGCAAGCCACTCATAATGTTGTCATTTGAAGATCACAC |
| -2,31 | 5,8E-15 | NM_024875 | SYNP02L | ACAGCATCTTGTGAAAGTTATGGAGCATGAAAAGACTGAAGGGCCAGGACAGTTTGCATG |
| -2,32 | 3,0E-19 | NM_004816 | C9orf61 | GAGGATTATTGTTGATTCCATTTACTCATGCTTGCAAAACTAGAGACCCCTAAGGCAGAA |
| -2,32 | 1,3E-12 | NM_004004 | GJB2 | GTCATAGCACCTAACAACATTGTAGCCTCAATCGAGTGAGACAGACTAGAAGTTCCTAGT |
| -2,33 | 3,7E-16 | NM_004660 | DDX3Y | CACTGATAGGAAGGTCCACATCCACAAAGTTTCTCTTGAGTTTTGTTATGTGTTTTGCTG |
| -2,34 | 5,7E-14 | NM_001007176 | C8orf22 | AAAGACTATCGCTGGAAGTGGCACTTGCTACCTGGTGCATCTTTGAAAAGTGTGCCTTTA |
| -2,35 | 2,8E-15 | NM_006790 | MYOT | TAACCCAGAAGGAGAATTTCAGCGTTTGGCAGCTCAATCTGGACTCTATGAAAGTGAAGA |
| -2,38 | 1,8E-15 | NM_032588 | TRlM63 | ACGATGTGCTGTATTTCAGTGTCTATCCCAGACATACGGGGTGGTAACTGAGTTTGTGTT |
| -2,40 | 4,5E-15 | NM_004533 | MYBPC2 | CCAAGACAATTGGTGGTGGAGTCCTGACCCCAATCCCCAACCTCCCAGGACTGTGTTCTT |
| -2,40 | 2,4E-13 | NM_021245 | MYOZ1 | GCAATGCCCTATGGTGGATATGAGAAGGCCTCCAAACGCATGACCTTCCAGATGCCCAAG |
| -2,41 | 3,4E-10 | NM_003063 | SLN | CAATATCTGCTATGCTGTAGTGCTAGGATTGATTATGTGTTCTCCAAAGATGCTGCTCCC |
| -2,44 | 7,9E-16 | NM_001976 | EN03 | CTCGGAGCGTCTGGCCAAATACAACCAACTCATGAGGATCGAGGAGGCTCTTGGGGACAA |
| -2,52 | 4,9E-09 | NM_001008 | RPS4Y1 | CAACTTTATCAAATTTGATACAGGCAATTTGTGTATGGTGATTGGTGGAGCCAACCTCGG |
| -2,52 | 3,8E-10 | NM_001039567 | RPS4Y2 | TGTTGGTGTGATCACAAACAGGGAAAGACATCCTGGTTCTTGCGATGTGGTACATGTGAA |
| -2,56 | 1,4E-17 | NM_033118 | MYLK2 | CAGGAGAATTAGGAAGGCCATGGGGCAGCCTCCAGTCTGCTCTCAGCTTGTGCCTTGTAA |
| -2,56 | 3,6E-20 | NM_006789 | APOBEC2 | ACAGCCTCAGACCCGAGGTTTAGATTTCTGAAATATGCATTTTATGTTAAGTTGGGTATT |
| -2,57 | 2,7E-09 | NM_004543 | NEB | TCTGCAGGACTTACAGATCCTGCAGTCAATGTTTCGGTTTAGACTCTCCACTGTTACCTA |
| -2,67 | 1,4E-10 | BC107798 | TNNT1 | TCCGAGCGTAAGAAGCCTCTGGACATTGACTACATGGGGGAGGAACAGCTCCGGGAGAAA |
| -2,69 | 7,0E-21 | NM_144994 | ANKRD23 | ACACACCTTTTTATACCAATCAGTATCCTCTGTTCATTAAAACTGGCTATCCATTAAAAA |
| -2,71 | 2,8E-13 | NM_000366 | TPM1 | GTGCTTTCTATTGTACAGAAGCTCTTCGTTTCAGTGTCAAATAAACACTGTGTAAGCTAA |
| -2,75 | 3,7E-12 | NM_006757 | TNNT3 | GACAAGGCCAAGGAGCTCTGGGAGACCCTGCACCAGCTGGAGATTGACAAGTTCGAGTTT |
| -2,77 | 7,1E-13 | NM_003280 | TNNC1 | CAGCGGCACGGTGGACTTTGATGAGTTCCTGGTCATGATGGTTCGGTGCATGAAGGACGA |
| -2,98 | 3,1E-11 | NM_003279 | TNNC2 | GACTTCGACGAGTTCCTGAAGATGATGGAGGACGTGCAGTAAGGAGTGGACAGTCGCCTC |
| -3,14 | 2,9E-16 | NM_005181 | CA3 | AGACAGCAAGAATTGAGCTAATAATATGTTTTAACTCTTAACACCAGCAAGAAGTCAGTC |
| -3,20 | 4,1E-14 | NM_003673 | TCAP | AGGCAAGGATGGTGGGGCCCCCAGCACCCTCTGTTAGTGCCGCAATAAATGCTCAATCAT |
| -3,24 | 1,4E-20 | NM_014332 | SMPX | GTGTGGTTTTGAGGAGGGATATGATTTTATGGAGAATGATATGGCAATGTGCCTAACGAT |
| -3,27 | 2,3E-13 | NM_133378 | TTN | CTGACAACCCTGATCATCATGGACGTACAGAAACAAGATGGTGGACTTTATACCCTGAGT |
| -3,29 | 5,8E-15 | NM_019885 | CYP26B1 | TTTGTTTTGATATGAAACTGGTACCGTGTGAGTGTTTTTGCTGTCGTGGTTTTAATCTGT |
| -3,31 | 1,8E-14 | NM_000432 | MYL2 | AAGGAGGCTCCGGGTCCAATTAACTTTACTGTGTTCCTCACAATGTTTGGGGAGAAACTT |
| -3,52 | 5,2E-18 | NM_006063 | KBTBD10 | GCTGGGATGTTGAAGGAAATACGTTATGCTTCAGGAGCTAGTTGCCTAGCAACACGTTTA |
| -3,52 | 6,5E-17 | NM_206819 | MYBPC1 | AGGATGCCACCATGACTAAAGAGAGTGCAGTGATCGCCAGGGATGGTAAAATCTACAAAA |
| -3,64 | 6,1E-20 | BX647448 | CMYA3 | TTTGAGGAACTTGATGTAAACATGGTGTTCAGAAATCTCGTGTCTATCTCAATGGGATAT |
| -3,69 | 7,6E-16 | NM_013292 | MYLPF | GAGGAGTTGGATGCCATGATGAAGGAAGCCAGCGGTCCCATCAACTTCACCGTCTTCCTG |
| -3,81 | 2,8E-14 | NM_198060 | NRAP | GGCCAGAGAGGAAGTTTGTTCACCAGAGACAGGCTTCAGATGGCTTTGATTTCGGCAAGC |
| -3,96 | 8,9E-17 | NM_003282 | TNNI2 | GGAGCGGGACCTGCGAGACGTGGGTGACTGGAGGAAGAACATCGAGGAGAAGTCTGGCAT |
| -4,25 | 1,4E-20 | NM_001103 | ACTN2 | TCCCATCAGAATGCAATAAAAGCGGAAGTCACAGTTTGTTTCCTGGAAACTTTGACAAGC |
| -4,33 | 5,5E-18 | NM_003476 | CSRP3 | TATGTGTTTCTCCTCAGAAGTGATCAGGTCTTTACTGAATGTTAGAAGAGGCCTTTGGAA |
| -4,69 | 4,6E-14 | NM_005963 | MYH1 | TTATCTAACTGCTGAAAGGTGACCAAAGAAATGCACAAAATGTGAAAATCTTTGTCACTC |
| -5,01 | 1,6E-16 | NM_079422 | MYL1 | AGAAAATTGAGTTTGAACAATTTCTGCCTATGATGCAAGCCATTTCCAACAACAAGGACC |
| -5,43 | 1,7E-17 | NM_203377 | MB | GGAGTTCATCTCGGAATGCATCATCCAGGTTCTGCAGAGCAAGCATCCCGGGGACTTTGG |
| -8,36 | 8,1E-16 | NM_000257 | MYH7 | CCGTGACATTGGCACGAAGGGCTTGAATGAGGAGTAGCTTTGCCACATCTTGATCTGCTC |
| -12,08 | 2,6E-18 | NM_001824 | CKM | ACACTCGGAGCTTGTGCTTTGTCTCCACGCAAAGCGATAAATAAAAGCATTGGTGGCCTT |
| -12,49 | 1,1E-17 | NM_017534 | MYH2 | CGGGAGGTTCACACAAAAGTCATAAGTGAAGAGTGATCATGTCCTGATGCCATGGAATGA |
| -15,31 | 2,5E-18 | NM_001100 | ACTA1 | CCGCAGTCACTTTCTTTGTAACAACTTCCGTTGCTGCCATCGTAAACTGACACAGTGTTT |

**Table 5: Ingenuity Pathways Analysis of significantly differential expressed genes**

| **Top Canonical Pathways overrepresented in RA** | | |
|---|---|---|
| Pathway | p-value | Ratio |
| Cytotoxic T Lymphocyte-mediated Apoptosis of Target Cells | 7.19E-06 | 7/31 (0.226) |
| Communication between Innate and Adaptive Immune Cells | 8.85E-05 | 10/90 (0.111) |
| Hepatic Fibrosis / Hepatic Stellate Cell Activation | 1.26E-04 | 14/135 (0.104) |
| Death Receptor Signaling | 1.85E-04 | 9/64 (0.141) |
| Type I Diabetes Mellitus Signaling | 1.93E-04 | 12/114 (0.105) |

| **Top Canonical Pathways overrepresented in SpA** | | |
|---|---|---|
| Pathway | p-value | Ratio |
| Calcium Signaling | 5.72E-12 | 21/206 (0.102) |
| Actin Cytoskeleton Signaling | 1.77E-09 | 20/237 (0.084) |
| ILK Signaling | 1.92E-08 | 17/186 (0.091) |
| Cellular Effects of Sildenafil (Viagra) | 1.25E-07 | 14/151 (0.093) |
| Hepatic Fibrosis / Hepatic Stellate Cell Activation | 3.4E-06 | 12/135 (0.089) |

### References

1. Dougados M, van der Linden S, Juhlin R, Huitfeldt B, Amor B, et al. The European Spondyloarthropathy Study Group preliminary criteria for the classification of spondyloarthropathy. Arthritis Rheum. 1991; 34: 1218-27.
2. Arnett FC, Edworthy SM, Bloch DA, McShane DJ, Fries JF, et al. The American Rheumatism Association 1987 revised criteria for the classification of rheumatoid arthritis. Arthritis Rheum. 1988; 31: 315-24.
3. Baeten D, Van den Bosch F, Elewaut D, Stuer A, Veys EM, De Keyser F. Needle arthroscopy of the knee with synovial biopsy sampling: technical experience in 150 patients. Clin Rheumatil 1999; 18: 434-41.
4. Smyth GK. Limma: linear models for microarray data. In: Bioinformatics and Computational Biology Solutions using R and Bioconductor, R. Gentleman, V. Carey, S. Dudoit, R. Irizarry, W. Huber (eds.), 2005, Springer, New York: 397―420.
5. Cleveland W, Devlin S. Locally weighted regression: An approach to regression analysis by local fitting. J Am Stat Assoc 1983; 83: 596-410.
6. Yang YH, Dudoit S, Luu P, Lin DM, Peng V, Ngai J, Speed TP. Normalization for cDNA microarray data: a robust composite method addressing single and multiple slide systematic variation. Nucleic Acids Res 2002; 30:e15.
7. Smyth GK, Speed T. Normalization of cDNA microarray data. Methods 2003; 31: 265-73.
8. Smyth GK. Linear models and empirical Bayes methods for assessing differential expression in microarray experiments. Statistical Applications in Genetics and Molecular Biology 2004;3: Article 3.
9. Efron B, Tibshirani R. Empirical Bayes methods and false discovery rates for microarrays. Genet Epidemiol 2002; 23: 70-86.
10. Baeten D, Demetter P, Cuvelier C, Van Den Bosch F, Kruithof E, et al. Comparative study of the synovial histology in rheumatoid arthritis, spondyloarthropathy, and osteoarthritis: influence of disease duration and activity. Ann Rheum Dis 2000; 59: 945-53.
11. Baeten D, Demetter P, Cuvelier CA, Kruithof E, Van Damme N, et al. Macrophages expressing the scavenger receptor CD163: a link between immune alterations of the gut and synovial inflammation in spondyloarthropathy. J Pathol 2002; 196: 343-50.
12. Baeten D, Steenbakkers PG, Rijnders AM, Boots AM, Veys EM, De Keyser F. Detection of major histocompatibility complex/human cartilage gp-39 complexes in rheumatoid synovitis as a specific and independent histologic marker. Arthritis Rheum 2004; 50: 444-51.
13. Baeten D, Moller HJ, Delanghe J, Veys EM, Moestrup SK, De Keyser F. Association of CD163+ macrophages and local production of soluble CD163 with decreased lymphocyte activation in spondylarthropathy synovitis. Arthritis Rheum 2004; 50: 1611-23.
14. Baeten D, Kruithof E, De Rycke L, Vandooren B, Wyns B, et al. Diagnostic classification of spondylarthropathy and rheumatoid arthritis by synovial histopathology: a prospective study in 154 consecutive patients. Arthritis Rheum 2004; 50: 2931-41.
15. Kruithof E, Baeten D, De Rycke L, Vandooren B, Foell D, et al. Synovial histopathology of psoriatic arthritis, both oligo- and polyarticular, resembles spondyloarthropathy more than it does rheumatoid arthritis. Arthritis Rs Ther 2005; 7: R569-80.
16. De Rycke L, Baeten D, Foell D, Kruithof E, Veys EM, Roth J, De Keyser F. Differential expression and response to anti-TNFalpha treatment of infiltrating versus resident tissue macrophage subsets in autoimmune arthritis. J Pathol 2005; 206: 17-27.

## Claims

1. A method for typing a sample of an individual suffering from or suspected of suffering from rheumatoid arthritis or spondyloarthritis, the method comprising:
- determining levels of expression products of a set of genes, said expression products being obtained from, and/or present in, a sample of said individual;
- typing said sample on the basis of the expression product levels determined for said set of genes;
wherein said set of genes comprises at least 5 genes listed in table 3.

2. A method according to claim 1 wherein said set of genes comprises at least 5 genes listed in table 4.

3. A method according to claim 1 or 2 wherein said set of genes comprises at least 10 genes listed in table 3 or 4, more preferably at least 20 genes listed in table 3 or 4, most preferably at least 50 genes listed in table 3 or 4.

4. A method according to any one of claims 1-3 wherein said expression product is RNA.

5. A method according to any one of claims 1-4 wherein said sample is, a blood sample, a peripheral blood mononuclear cell sample or a synovial tissue sample.

6. A method according to any one of claims 1-5 wherein said synovial tissue sample is obtained from a knee joint or an ankle-joint.

7. A method according to any one of claims 1-6 wherein said set of genes comprises tumour necrosis factor receptor superfamily member 17 (TNFRSF17) and/or immunoglobulin J polypeptide, linker protein for immunoglobulin alpha and mu polypeptides (IGJ) and/or POU class 2 associating factor 1 (POU2AF1), and/or Der1-like domain family, member 3 (DERL3), and/or Fc receptor-like 5 (FCRL5), and/or plasma cell-induced ER protein 1 (PACAP), and/or prepronociceptin (PNOC), and/or secreted phosphoprotein 1 (SPP1), and/or interferon regulatory factor 4 (IRF4), and/or lymphocyte transmembrane adaptor 1 (LAX1), and/or ELL associated factor 2 (EAF2), and/or pim-2 oncogene (PIM2) and/or actin, alpha 1, skeletal muscle (ACTA1) and/or Myosin, heavy chain 3, skeletal muscle, embryonic (MYH2) and/or myosin, heavy chain 1, skeletal muscle, adult (MYH1), and/or cysteine and glycine-rich protein 3 (CSRP3), and/or actinin, alpha 2 (ACTN2), and/or troponin I type 2 (TNNI2), and/or cytochrome P450, family 26, subfamily B, polypeptide 1 (CYP26B1), and/or titin-cap (TCAP).

8. A method according to any one of claims 1-7 wherein RNA levels are determined by microarray analysis.

9. A nucleotide microarray, comprising at least 5 nucleic acid sequences being able to hybridize to at least 5 genes listed in table 3 or 4, and wherein at least 80% of the nucleic acid molecules of said microarray are able to hybridize to any gene listed in table 3 or 4.

10. A nucleotide microarray according to claim 9 comprising at least 10 nucleic acid sequences being able to hybridize to at least 10 genes listed in table 3 or 4, more preferably comprising at least 20 nucleic acid sequences being able to hybridize to at least 20 genes listed in table 3 or 4, more preferably comprising at least 50 nucleic acid sequences being able to hybridize to at least 50 genes listed in table 3 or 4.

11. Use of a nucleotide microarray according to claim 9 or 10 for typing a sample of an individual suffering from or suspected of suffering from rheumatoid arthritis or spondyloarthritis.
